# EUROPEAN PATENT APPLICATION

(11) **EP 1 767 643 A1**
(43) Date of publication of application: **28.03.2007**
(21) Application number: 05021039.2
(22) Date of filing: 27.09.2005
(51) Int. Cl.: C12N 15/85, C07K 14/805, A61K 48/00

(54) **Host cells and methods for the cytoprotection from oxidative and nitrosative stress**

(71) Applicant: Alexander Daniel Frey, 8057 Zürich (CH); Pauli T. Kallio, 8964 Rudolfstetten (CH)
(72) Inventor: Alexander Daniel Frey, 8057 Zürich (CH); Pauli T. Kallio, 8964 Rudolfstetten (CH)
(74) Representative: Grünecker, Kinkeldey, Stockmair & Schwanhäusser Anwaltssozietät

(57) **Abstract**

The present invention relates to nucleic acids coding for bacterial hemoglobin or a bacterial flavohemoglobin having oxidative and/or nitrosative stress protective activity, mammalian host cells containing the aforementioned nucleic acids, and pharmaceutical compositions based on these nucleic acids and host cells which may be used inter alia for the treatment of inflammatory and autoinflammatory diseases including diabetes type I disease.

SEQUENCE LISTING

<110>
Frey, Ph.D., Alexander
Pauli, Ph.D., T. Kallio

<120> Host cells and methods for the cytoprotection from oxidative and
nitrosative stress

<130> EP36303

<140> not yet assigned
<141> not yet assigned

<160> 44

<170> PatentIn version 3.3

<210> 1
<211> 146
<212> PRT
<213> Vitreoscilla sp.

<220>
<223> Amino acid sequence of Vitreoscilla sp. hemoglobin

<400> 1

Met Leu Asp Gln Gln Thr Ile Asn Ile Ile Lys Ala Thr Val Pro Val
1               5                   10                  15

Leu Lys Glu His Gly Val Thr Ile Thr Thr Thr Phe Tyr Lys Asn Leu
            20                  25                  30

Phe Ala Lys His Pro Glu Val Arg Pro Leu Phe Asp Met Gly Arg Gln
        35                  40                  45

Glu Ser Leu Glu Gln Pro Lys Ala Leu Ala Met Thr Val Leu Ala Ala
    50                  55                  60

Ala Gln Asn Ile Glu Asn Leu Pro Ala Ile Leu Pro Ala Val Lys Lys
65                  70                  75                  80

Ile Ala Val Lys His Cys Gln Ala Gly Val Ala Ala Ala His Tyr Pro
                85                  90                  95

Ile Val Gly Gln Glu Leu Leu Gly Ala Ile Lys Glu Val Leu Gly Asp
            100                 105                 110

Ala Ala Thr Asp Asp Ile Leu Asp Ala Trp Gly Lys Ala Tyr Gly Val
        115                 120                 125

Ile Ala Asp Val Phe Ile Gln Val Glu Ala Asp Leu Tyr Ala Gln Ala
    130                 135                 140

Val Glu
145


<210> 2
<211> 441
<212> DNA
<213> Vitreoscilla sp.


<220>
<223> Nucleic acid coding sequence of Vitreoscilla sp. hemoglobin

<400> 2
atgttagacc agcaaaccat taacatcatc aaagccactg ttcctgtatt gaaggagcat 60

ggcgttacca ttaccacgac tttttataaa aacttgtttg ccaaacaccc tgaagtacgt 120

cctttgtttg atatgggtcg ccaagaatct ttggagcagc ctaaggcttt ggcgatgacg 180

gtattggcgg cagcgcaaaa cattgaaaat ttgccagcta ttttgcctgc ggtcaaaaaa 240

attgcagtca aacattgtca agcaggcgtg gcagcagcgc attatccgat tgtcggtcaa 300

gaattgttgg gtgcgattaa agaagtattg ggcgatgccg caaccgatga cattttggac 360

gcgtggggca aggcttatgg cgtgattgca gatgtgttta ttcaagtgga agcagatttg 420

tacgctcaag cggttgaata a 441


<210> 3
<211> 403
<212> PRT
<213> Ralstonia eutropha


<220>
<223> Amino acid sequence of Ralstonia eutropha flavohemoglobin

<400> 3

<210>  4
<211>  1212
<212>  DNA
<213>  Ralstonia eutropha

<220>
<223>  Nucleic acid coding sequence of Ralstonia eutropha
       flavohemoglobin

<400>  4
atgctgaccc agaaaacaaa agacatcgta aaggccaccg caccggtgct ggccgaacac      60

ggctacgaca tcatcaagtg cttctatcag cgcatgttcg aagcgcatcc cgaactgaag     120

aatgtgttca acatggccca ccaggaacag ggccagcagc agcaggcgct ggcacgggcg     180

gtctatgcct atgcggagaa tatcgaggac ccaaacagtc tgatggccgt cctgaagaac     240

atcgccaaca agcatgcaag tctcggcgtc aaaccggaac agtaccccat tgttggagaa     300

cacctactgg ccgccatcaa ggaagtgttg ggcaatgccg caaccgacga catcatttcc     360

gcttgggcgc aagcctacgg gaatctggcc gatgtcctga tgggcatgga gagcgagttg     420

tatgagcgct ctgcggaaca acccggtggc tggaaggggt ggcgcacctt tgtgattcgt     480

gagaagcgcc ccgagagcga tgtgatcacg tcattcatcc ttgagcctgc cgatggtggt     540

cccgtggtga acttcgaacc cggacaatac accagcgtcg caatcgatgt gcccgcgctc     600

gggttgcagc agattcgcca atacagcctg tcggatatgc cgaacgggcg cacgtatcgc     660

atctcggtga agcgcgaggg cggcgggccg cagcctccgg gctatgtttc gaacctgttg     720

catgatcacg tgaatgttgg ggatcaggtc aagctggctg cgccctatgg aagcttccat     780

atcgacgttg acgccaagac acccattgtc ctgatcagcg gtggcgttgg gctgacaccg     840

atggtcagca tgctcaaagt ggcattgcag gccccaccga ggcaagttgt attcgtgcat     900

ggcgcacgta acagcgcagt acacgcgatg cgcgacaggc tgcgcgaagc ggcgaagacc 960

tatgaaaacc ttgacctctt tgttttctat gaccaaccgc taccggaaga tgtccaggga 1020

cgggactatg attatccggg cttagtcgat gtgaagcaga tcgagaaatc gatcctgctg 1080

ccagacgccg actactacat ctgcggtccg attccgttca tgcgaatgca gcatgatgcc 1140

ctgaagaatc tcggcattca tgaggcacgc attcattacg aagtgtttgg tcccgacctg 1200

tttgctgagt ag 1212

<210> 5
<211> 24
<212> DNA
<213> Artificial

<220>
<223> VHb forward primer for Vitreoscilla sp. hemoglobin amplification

<400> 5
cgggatccat gttagaccag caaa 24

<210> 6
<211> 35
<212> DNA
<213> Artificial

<220>
<223> VHb reverse primer for Vitreoscilla sp. hemoglobin amplification

<400> 6
atagtttagc ggccgcttat tcaaccgctt gagcg 35

<210> 7
<211> 28
<212> DNA
<213> Artificial

<220>
<223> FHP forward primer for Ralstonia eutropha flavohemoglobin
amplification

<400> 7
cgggatccat gctgacccag aaaacaaa 28

<210> 8
<211> 35
<212> DNA
<213> Artificial

<220>
<223> FHP reverse primer for Ralstonia eutropha flavohemoglobin
amplification

<400> 8
atagtttagc ggccgcttac tactcagcaa acagg 35

<210> 9
<211> 7000
<212> DNA
<213> Artificial

<220>
<223> pEF6-FHP expression vector for expression of Ralstonia eutropha
      flavohemoglobin

<400> 9
aatattattg aagcatttat cagggttatt gtctcatgag cggatacata tttgaatgta 60

tttagaaaaa taaacaaata ggggttccgc gcacatttcc ccgaaaagtg ccacctgacg 120

tcgacggatc gggagatctc ccgatcccct atggtcgact ctcagtacaa tctgctctga 180

tgccgcatag ttaagccagt atctgctccc tgcttgtgtg ttggaggtcg ctgagtagtg 240

cgcgagcaaa atttaagcta caacaaggca aggcttgacc gacaattgca tgaagaatct 300

gcttagggtt aggcgttttg cgctgcttcg cgatgtacgg gccagatata cgcgttgaca 360

ttgattattg actaggcttt tgcaaaaagc tttgcaaaga tggataaagt tttaaacaga 420

gaggaatctt tgcagctaat ggaccttcta ggtcttgaaa ggagtgcctc gtgaggctcc 480

ggtgcccgtc agtgggcaga gcgcacatcg cccacagtcc ccgagaagtt ggggggaggg 540

gtcggcaatt gaaccggtgc ctagagaagg tggcgcgggg taaactggga aagtgatgtc 600

gtgtactggc tccgcctttt tcccgagggt gggggagaac cgtatataag tgcagtagtc 660

gccgtgaacg ttctttttcg caacgggttt gccgccagaa cacaggtaag tgccgtgtgt 720

ggttcccgcg ggcctggcct ctttacgggt tatggccctt gcgtgccttg aattacttcc 780

acctggctgc agtacgtgat tcttgatccc gagcttcggg ttggaagtgg gtgggagagt 840

tcgaggcctt gcgcttaagg agccccttcg cctcgtgctt gagttgaggc ctggcctggg 900

cgctggggcc gccgcgtgcg aatctggtgg caccttcgcg cctgtctcgc tgctttcgat 960

aagtctctag ccatttaaaa tttttgatga cctgctgcga cgcttttttt ctggcaagat 1020

agtcttgtaa atgcgggcca agatctgcac actggtattt cggtttttgg ggccgcgggc 1080

ggcgacgggg cccgtgcgtc ccagcgcaca tgttcggcga ggcggggcct gcgagcgcgg 1140

ccaccgagaa tcggacgggg gtagtctcaa gctggccggc ctgctctggt gcctggcctc 1200

gcgccgccgt gtatcgcccc gccctgggcg gcaaggctgg cccggtcggc accagttgcg 1260

tgagcggaaa gatggccgct tcccggccct gctgcaggga gctcaaaatg gaggacgcgg 1320

cgctcgggag agcgggcggg tgagtcaccc acacaaagga aaagggcctt tccgtcctca 1380

gccgtcgctt catgtgactc cacggagtac cgggcgccgt ccaggcacct cgattagttc 1440

tcgagctttt ggagtacgtc gtctttaggt tggggggagg ggttttatgc gatggagttt 1500

ccccacactg agtgggtgga gactgaagtt aggccagctt ggcacttgat gtaattctcc 1560

ttggaatttg ccctttttga gtttggatct tggttcattc tcaagcctca gacagtggtt 1620

caaagttttt ttcttccatt tcaggtgtcg tgaggaatta gcttggtact aatacgactc 1680

actataggga gacccaagct ggctaggtaa gcttggtacc gagctcggat ccaccatgct 1740

gacccagaaa acaaaagaca tcgtaaaggc caccgcaccg gtgctggccg aacacggcta 1800

cgacatcatc aagtgcttct atcagcgcat gttcgaagcg catcccgaac tgaagaatgt 1860

gttcaacatg gcccaccagg aacagggcca gcagcagcag gcgctggcac gggcggtcta 1920

tgcctatgcg gagaatatcg aggacccaaa cagtctgatg gccgtcctga agaacatcgc 1980

caacaagcat gcaagtctcg gcgtcaaacc ggaacagtac cccattgttg gagaacacct 2040

actggccgcc atcaaggaag tgttgggcaa tgccgcaacc gacgacatca tttccgcttg 2100

ggcgcaagcc tacgggaatc tggccgatgt cctgatgggc atggagagcg agttgtatga 2160

gcgctctgcg gaacaacccg gtggctggaa ggggtggcgc acctttgtga ttcgtgagaa 2220

gcgccccgag agcgatgtga tcacgtcatt catccttgag cctgccgatg gtggtcccgt 2280

ggtgaacttc gaacccggac aatacaccag cgtcgcaatc gatgtgcccg cgctcgggtt 2340

gcagcagatt cgccaataca gcctgtcgga tatgccgaac gggcgcagct atcgcatctc 2400

ggtgaagcgc gagggcggcg ggccgcagcc tccgggctat gtttcgaacc tgttgcatga 2460

tcacgtgaat gttggggatc aggtcaagct ggctgcgccc tatggaagct tccatatcga 2520

cgttgacgcc aagacaccca ttgtcctgat cagcggtggc gttgggctga caccgatggt 2580

cagcatgctc aaagtggcat tgcaggcccc accgaggcaa gttgtattcg tgcatggcgc 2640

acgtaacagc gcagtacacg cgatgcgcga caggctgcgc gaagcggcga agacctatga 2700

aaaccttgac ctctttgttt tctatgacca accgctaccg gaagatgtcc agggacggga 2760

ctatgattat ccgggcttag tcgatgtgaa gcagatcgag aaatcgatcc tgctgccaga 2820

cgccgactac tacatctgcg gtccgattcc gttcatgcga atgcagcatg atgccctgaa 2880

gaatctcggc attcatgagg cacgcattca ttacgaagtg tttggtcccg acctgtttgc 2940

tgagtagtaa gcggccgctc gagtctagag ggcccgcggt tcgaaggtaa gcctatccct 3000

aaccctctcc tcggtctcga ttctacgcgt accggtcatc atcaccatca ccattgagtt 3060

taaacccgct gatcagcctc gactgtgcct tctagttgcc agccatctgt tgtttgcccc 3120

tcccccgtgc cttccttgac cctggaaggt gccactccca ctgtcctttc ctaataaaat 3180

gaggaaattg catcgcattg tctgagtagg tgtcattcta ttctgggggg tggggtgggg 3240

caggacagca agggggagga ttgggaagac aatagcaggc atgctgggga tgcggtgggc 3300

tctatggctt ctgaggcgga aagaaccagc tggggctcta gggggtatcc ccacgcgccc 3360

tgtagcggcg cattaagcgc ggcgggtgtg gtggttacgc gcagcgtgac cgctacactt 3420

gccagcgccc tagcgcccgc tcctttcgct ttcttccctt cctttctcgc cacgttcgcc 3480

ggctttcccc gtcaagctct aaatcggggc atccctttag ggttccgatt tagtgcttta 3540
cggcacctcg accccaaaaa acttgattag ggtgatggtt cacgtagtgg gccatcgccc 3600
tgatagacgg tttttcgccc tttgacgttg gagtccacgt tctttaatag tggactcttg 3660
ttccaaactg gaacaacact caaccctatc tcggtctatt cttttgattt ataagggatt 3720
ttggggattt cggcctattg gttaaaaaat gagctgattt aacaaaaatt taacgcgaat 3780
taattctgtg gaatgtgtgt cagttagggt gtggaaagtc cccaggctcc ccaggcaggc 3840
agaagtatgc aaagcatgca tctcaattag tcagcaacca ggtgtggaaa gtccccaggc 3900
tccccagcag gcagaagtat gcaaagcatg catctcaatt agtcagcaac catagtcccg 3960
cccctaactc cgcccatccc gcccctaact ccgcccagtt ccgcccattc tccgccccat 4020
ggctgactaa ttttttttat ttatgcagag gccgaggccg cctctgcctc tgagctattc 4080
cagaagtagt gaggaggctt ttttggaggc ctaggctttt gcaaaaagct cccgggagct 4140
tgtatatcca ttttcggatc tgatcagcac gtgttgacaa ttaatcatcg gcatagtata 4200
tcggcatagt ataatacgac aaggtgagga actaaaccat ggccaagcct ttgtctcaag 4260
aagaatccac cctcattgaa agagcaacgg ctacaatcaa cagcatcccc atctctgaag 4320
actacagcgt cgccagcgca gctctctcta gcgacggccg catcttcact ggtgtcaatg 4380
tatatcattt tactggggga ccttgtgcag aactcgtggt gctgggcact gctgctgctg 4440
cggcagctgg caacctgact tgtatcgtcg cgatcggaaa tgagaacagg ggcatcttga 4500
gcccctgcgg acggtgtcga caggtgcttc tcgatctgca tcctgggatc aaagcgatag 4560
tgaaggacag tgatggacag ccgacggcag ttgggattcg tgaattgctg ccctctggtt 4620
atgtgtggga gggctaagca cttcgtggcc gaggagcagg actgacacgt gctacgagat 4680
ttcgattcca ccgccgcctt ctatgaaagg ttgggcttcg gaatcgtttt ccgggacgcc 4740
ggctggatga tcctccagcg cggggatctc atgctggagt tcttcgccca ccccaacttg 4800
tttattgcag cttataatgg ttacaaataa agcaatagca tcacaaattt cacaaataaa 4860
gcattttttt cactgcattc tagttgtggt ttgtccaaac tcatcaatgt atcttatcat 4920
gtctgtatac cgtcgacctc tagctagagc ttggcgtaat catggtcata gctgtttcct 4980
gtgtgaaatt gttatccgct cacaattcca cacaacatac gagccggaag cataaagtgt 5040
aaagcctggg gtgcctaatg agtgagctaa ctcacattaa ttgcgttgcg ctcactgccc 5100
gctttccagt cgggaaacct gtcgtgccag ctgcattaat gaatcggcca acgcgcgggg 5160
agaggcggtt tgcgtattgg gcgctcttcc gcttcctcgc tcactgactc gctgcgctcg 5220
gtcgttcggc tgcggcgagc ggtatcagct cactcaaagg cggtaatacg gttatccaca 5280
gaatcagggg ataacgcagg aaagaacatg tgagcaaaag gccagcaaaa ggccaggaac 5340
cgtaaaaagg ccgcgttgct ggcgtttttc cataggctcc gcccccctga cgagcatcac 5400

aaaaatcgac gctcaagtca gaggtggcga aacccgacag gactataaag ataccaggcg 5460

tttccccctg gaagctccct cgtgcgctct cctgttccga ccctgccgct taccggatac 5520

ctgtccgcct ttctcccttc gggaagcgtg gcgctttctc aatgctcacg ctgtaggtat 5580

ctcagttcgg tgtaggtcgt tcgctccaag ctgggctgtg tgcacgaacc ccccgttcag 5640

cccgaccgct gcgccttatc cggtaactat cgtcttgagt ccaacccggt aagacacgac 5700

ttatcgccac tggcagcagc cactggtaac aggattagca gagcgaggta tgtaggcggt 5760

gctacagagt tcttgaagtg gtggcctaac tacggctaca ctagaaggac agtatttggt 5820

atctgcgctc tgctgaagcc agttaccttc ggaaaaagag ttggtagctc ttgatccggc 5880

aaacaaacca ccgctggtag cggtggtttt tttgtttgca agcagcagat tacgcgcaga 5940

aaaaaaggat ctcaagaaga tcctttgatc ttttctacgg ggtctgacgc tcagtggaac 6000

gaaaactcac gttaagggat tttggtcatg agattatcaa aaaggatctt cacctagatc 6060

cttttaaatt aaaaatgaag ttttaaatca atctaaagta tatatgagta aacttggtct 6120

gacagttacc aatgcttaat cagtgaggca cctatctcag cgatctgtct atttcgttca 6180

tccatagttg cctgactccc cgtcgtgtag ataactacga tacgggaggg cttaccatct 6240

ggccccagtg ctgcaatgat accgcgagac ccacgctcac cggctccaga tttatcagca 6300

ataaaccagc cagccggaag ggccgagcgc agaagtggtc ctgcaacttt atccgcctcc 6360

atccagtcta ttaattgttg ccgggaagct agagtaagta gttcgccagt taatagtttg 6420

cgcaacgttg ttgccattgc tacaggcatc gtggtgtcac gctcgtcgtt tggtatggct 6480

tcattcagct ccggttccca acgatcaagg cgagttacat gatcccccat gttgtgcaaa 6540

aaagcggtta gctccttcgg tcctccgatc gttgtcagaa gtaagttggc cgcagtgtta 6600

tcactcatgg ttatggcagc actgcataat tctcttactg tcatgccatc cgtaagatgc 6660

ttttctgtga ctggtgagta ctcaaccaag tcattctgag aatagtgtat gcggcgaccg 6720

agttgctctt gcccggcgtc aatacgggat aataccgcgc cacatagcag aactttaaaa 6780

gtgctcatca ttggaaaacg ttcttcgggg cgaaaactct caaggatctt accgctgttg 6840

agatccagtt cgatgtaacc cactcgtgca cccaactgat cttcagcatc ttttactttc 6900

accagcgttt ctgggtgagc aaaaacagga aggcaaaatg ccgcaaaaaa gggaataagg 6960

gcgacacgga aatgttgaat actcatactc ttcctttttc 7000

<210> 10
<211> 6226
<212> DNA
<213> Artificial

<220>
<223> pEF6-VHb expression vector for expression of Vitreoscilla sp.

hemoglobin

<400> 10
aatattattg aagcatttat cagggttatt gtctcatgag cggatacata tttgaatgta 60

tttagaaaaa taaacaaata ggggttccgc gcacatttcc ccgaaaagtg ccacctgacg 120

tcgacggatc gggagatctc ccgatcccct atggtcgact ctcagtacaa tctgctctga 180

tgccgcatag ttaagccagt atctgctccc tgcttgtgtg ttggaggtcg ctgagtagtg 240

cgcgagcaaa atttaagcta caacaaggca aggcttgacc gacaattgca tgaagaatct 300

gcttagggtt aggcgttttg cgctgcttcg cgatgtacgg gccagatata cgcgttgaca 360

ttgattattg actaggcttt tgcaaaaagc tttgcaaaga tggataaagt tttaaacaga 420

gaggaatctt tgcagctaat ggaccttcta ggtcttgaaa ggagtgcctc gtgaggctcc 480

ggtgcccgtc agtgggcaga gcgcacatcg cccacagtcc ccgagaagtt ggggggaggg 540

gtcggcaatt gaaccggtgc ctagagaagg tggcgcgggg taaactggga aagtgatgtc 600

gtgtactggc tccgcctttt tcccgagggt gggggagaac cgtatataag tgcagtagtc 660

gccgtgaacg ttctttttcg caacgggttt gccgccagaa cacaggtaag tgccgtgtgt 720

ggttcccgcg ggcctggcct ctttacgggt tatggccctt gcgtgccttg aattacttcc 780

acctggctgc agtacgtgat tcttgatccc gagcttcggg ttggaagtgg gtgggagagt 840

tcgaggcctt gcgcttaagg agccccttcg cctcgtgctt gagttgaggc ctggcctggg 900

cgctggggcc gccgcgtgcg aatctggtgg caccttcgcg cctgtctcgc tgctttcgat 960

aagtctctag ccatttaaaa tttttgatga cctgctgcga cgcttttttt ctggcaagat 1020

agtcttgtaa atgcgggcca agatctgcac actggtattt cggtttttgg ggccgcgggc 1080

ggcgacgggg cccgtgcgtc ccagcgcaca tgttcggcga ggcggggcct gcgagcgcgg 1140

ccaccgagaa tcggacgggg gtagtctcaa gctggccggc ctgctctggt gcctggcctc 1200

gcgccgccgt gtatcgcccc gccctgggcg gcaaggctgg cccggtcggc accagttgcg 1260

tgagcggaaa gatggccgct tcccggccct gctgcaggga gctcaaaatg gaggacgcgg 1320

cgctcgggag agcgggcggg tgagtcaccc acacaaagga aaagggcctt tccgtcctca 1380

gccgtcgctt catgtgactc cacggagtac cgggcgccgt ccaggcacct cgattagttc 1440

tcgagctttt ggagtacgtc gtctttaggt tggggggagg ggttttatgc gatggagttt 1500

ccccacactg agtgggtgga gactgaagtt aggccagctt ggcacttgat gtaattctcc 1560

ttggaatttg ccctttttga gtttggatct tggttcattc tcaagcctca gacagtggtt 1620

caaagttttt ttcttccatt tcaggtgtcg tgaggaatta gcttggtact aatacgactc 1680

actataggga gacccaagct ggctaggtaa gcttggtacc gagctcggat ccaccatgtt 1740

agaccagcaa accattaaca tcatcaaagc cactgttcct gtattgaagg agcatggcgt 1800

taccattacc acgacttttt ataaaaactt gtttgccaaa caccctgaag tacgtccttt 1860

gtttgatatg ggtcgccaag aatctttgga gcagcctaag gctttggcga tgacggtatt 1920

ggcggcagcg caaaacattg aaaatttgcc agctattttg cctgcggtca aaaaaattgc 1980

agtcaaacat tgtcaagcag gcgtggcagc agcgcattat ccgattgtcg gtcaagaatt 2040

gttgggtgcg attaaagaag tattgggcga tgccgcaacc gatgacattt tggacgcgtg 2100

gggcaaggct tatggcgtga ttgcagatgt gtttattcaa gtggaagcag atttgtacgc 2160

tcaagcggtt gaataagcgg ccgctcgagt ctagagggcc cgcggttcga aggtaagcct 2220

atccctaacc ctctcctcgg tctcgattct acgcgtaccg gtcatcatca ccatcaccat 2280

tgagtttaaa cccgctgatc agcctcgact gtgccttcta gttgccagcc atctgttgtt 2340

tgcccctccc ccgtgccttc cttgaccctg gaaggtgcca ctcccactgt cctttcctaa 2400

taaaatgagg aaattgcatc gcattgtctg agtaggtgtc attctattct ggggggtggg 2460

gtggggcagg acagcaaggg ggaggattgg gaagacaata gcaggcatgc tggggatgcg 2520

gtgggctcta tggcttctga ggcggaaaga accagctggg gctctagggg gtatccccac 2580

gcgccctgta gcggcgcatt aagcgcggcg ggtgtggtgg ttacgcgcag cgtgaccgct 2640

acacttgcca gcgccctagc gcccgctcct ttcgctttct tcccttcctt tctcgccacg 2700

ttcgccggct ttccccgtca agctctaaat cggggcatcc ctttagggtt ccgatttagt 2760

gctttacggc acctcgaccc caaaaaactt gattagggtg atggttcacg tagtgggcca 2820

tcgccctgat agacggtttt tcgccctttg acgttggagt ccacgttctt taatagtgga 2880

ctcttgttcc aaactggaac aacactcaac cctatctcgg tctattcttt tgatttataa 2940

gggattttgg ggatttcggc ctattggtta aaaaatgagc tgatttaaca aaaatttaac 3000

gcgaattaat tctgtggaat gtgtgtcagt tagggtgtgg aaagtcccca ggctccccag 3060

gcaggcagaa gtatgcaaag catgcatctc aattagtcag caaccaggtg tggaaagtcc 3120

ccaggctccc cagcaggcag aagtatgcaa agcatgcatc tcaattagtc agcaaccata 3180

gtcccgcccc taactccgcc catcccgccc ctaactccgc ccagttccgc ccattctccg 3240

ccccatggct gactaatttt ttttatttat gcagaggccg aggccgcctc tgcctctgag 3300

ctattccaga agtagtgagg aggctttttt ggaggcctag gcttttgcaa aaagctcccg 3360

ggagcttgta tatccatttt cggatctgat cagcacgtgt tgacaattaa tcatcggcat 3420

agtatatcgg catagtataa tacgacaagg tgaggaacta aaccatggcc aagcctttgt 3480

ctcaagaaga atccaccctc attgaaagag caacggctac aatcaacagc atccccatct 3540

ctgaagacta cagcgtcgcc agcgcagctc tctctagcga cggccgcatc ttcactggtg 3600

tcaatgtata tcattttact gggggacctt gtgcagaact cgtggtgctg ggcactgctg 3660

ctgctgcggc agctggcaac ctgacttgta tcgtcgcgat cggaaatgag aacaggggca 3720

tcttgagccc ctgcggacgg tgtcgacagg tgcttctcga tctgcatcct gggatcaaag 3780
cgatagtgaa ggacagtgat ggacagccga cggcagttgg gattcgtgaa ttgctgccct 3840
ctggttatgt gtgggagggc taagcacttc gtggccgagg agcaggactg acacgtgcta 3900
cgagatttcg attccaccgc cgccttctat gaaaggttgg gcttcggaat cgttttccgg 3960
gacgccggct ggatgatcct ccagcgcggg gatctcatgc tggagttctt cgcccacccc 4020
aacttgttta ttgcagctta taatggttac aaataaagca atagcatcac aaatttcaca 4080
aataaagcat ttttttcact gcattctagt tgtggtttgt ccaaactcat caatgtatct 4140
tatcatgtct gtataccgtc gacctctagc tagagcttgg cgtaatcatg gtcatagctg 4200
tttcctgtgt gaaattgtta tccgctcaca attccacaca acatacgagc cggaagcata 4260
aagtgtaaag cctggggtgc ctaatgagtg agctaactca cattaattgc gttgcgctca 4320
ctgcccgctt tccagtcggg aaacctgtcg tgccagctgc attaatgaat cggccaacgc 4380
gcggggagag gcggtttgcg tattgggcgc tcttccgctt cctcgctcac tgactcgctg 4440
cgctcggtcg ttcggctgcg gcgagcggta tcagctcact caaaggcggt aatacggtta 4500
tccacagaat caggggataa cgcaggaaag aacatgtgag caaaaggcca gcaaaaggcc 4560
aggaaccgta aaaaggccgc gttgctggcg tttttccata ggctccgccc ccctgacgag 4620
catcacaaaa atcgacgctc aagtcagagg tggcgaaacc cgacaggact ataaagatac 4680
caggcgtttc cccctggaag ctccctcgtg cgctctcctg ttccgaccct gccgcttacc 4740
ggatacctgt ccgcctttct cccttcggga agcgtggcgc tttctcaatg ctcacgctgt 4800
aggtatctca gttcggtgta ggtcgttcgc tccaagctgg gctgtgtgca cgaacccccc 4860
gttcagcccg accgctgcgc cttatccggt aactatcgtc ttgagtccaa cccggtaaga 4920
cacgacttat cgccactggc agcagccact ggtaacagga ttagcagagc gaggtatgta 4980
ggcggtgcta cagagttctt gaagtggtgg cctaactacg gctacactag aaggacagta 5040
tttggtatct gcgctctgct gaagccagtt accttcggaa aaagagttgg tagctcttga 5100
tccggcaaac aaaccaccgc tggtagcggt ggtttttttg tttgcaagca gcagattacg 5160
cgcagaaaaa aaggatctca agaagatcct ttgatctttt ctacggggtc tgacgctcag 5220
tggaacgaaa actcacgtta agggattttg gtcatgagat tatcaaaaag gatcttcacc 5280
tagatccttt taaattaaaa atgaagtttt aaatcaatct aaagtatata tgagtaaact 5340
tggtctgaca gttaccaatg cttaatcagt gaggcaccta tctcagcgat ctgtctattt 5400
cgttcatcca tagttgcctg actccccgtc gtgtagataa ctacgatacg ggagggctta 5460
ccatctggcc ccagtgctgc aatgataccg cgagacccac gctcaccggc tccagattta 5520
tcagcaataa accagccagc cggaagggcc gagcgcagaa gtggtcctgc aactttatcc 5580

gcctccatcc agtctattaa ttgttgccgg gaagctagag taagtagttc gccagttaat 5640

agtttgcgca acgttgttgc cattgctaca ggcatcgtgg tgtcacgctc gtcgtttggt 5700

atggcttcat tcagctccgg ttcccaacga tcaaggcgag ttacatgatc ccccatgttg 5760

tgcaaaaaag cggttagctc cttcggtcct ccgatcgttg tcagaagtaa gttggccgca 5820

gtgttatcac tcatggttat ggcagcactg cataattctc ttactgtcat gccatccgta 5880

agatgctttt ctgtgactgg tgagtactca accaagtcat tctgagaata gtgtatgcgg 5940

cgaccgagtt gctcttgccc ggcgtcaata cgggataata ccgcgccaca tagcagaact 6000

ttaaaagtgc tcatcattgg aaaacgttct tcggggcgaa aactctcaag gatcttaccg 6060

ctgttgagat ccagttcgat gtaacccact cgtgcaccca actgatcttc agcatctttt 6120

actttcacca gcgtttctgg gtgagcaaaa acaggaaggc aaaatgccgc aaaaaaggga 6180

ataagggcga cacggaaatg ttgaatactc atactcttcc tttttc 6226

<210> 11
<211> 144
<212> PRT
<213> Clostridium perfringens

<220>
<223> Amino acid sequence of Clostridium perfringens hemoglobin (Cp-Hb)

<400> 11

Met Leu Asp Gln Lys Thr Ile Asp Ile Ile Lys Ser Thr Val Pro Val
1               5                   10                  15

Leu Lys Ser Asn Gly Leu Glu Ile Thr Lys Thr Phe Tyr Lys Asn Met
            20                  25                  30

Phe Glu Gln Asn Pro Glu Val Lys Pro Leu Phe Asn Met Asn Lys Gln
        35                  40                  45

Glu Ser Glu Glu Gln Pro Lys Ala Leu Ala Met Ala Ile Leu Ala Val
    50                  55                  60

Ala Gln Asn Ile Asp Asn Leu Glu Ala Ile Lys Pro Val Val Asn Arg
65                  70                  75                  80

Ile Gly Val Ile His Cys Asn Ala Lys Val Gln Pro Glu His Tyr Pro
                85                  90                  95

Ile Val Gly Lys His Leu Leu Gly Ala Ile Lys Glu Val Leu Gly Asp
            100                 105                 110

Gly Ala Thr Glu Asp Ile Ile Asn Ala Trp Ala Lys Thr Tyr Gly Val

115                 120                 125

Ile Ala Glu Val Phe Ile Asn Asn Glu Lys Glu Met Tyr Ala Ser Arg
    130                 135                 140

<210>  12
<211>  435
<212>  DNA
<213>  Clostridium perfringens

<220>
<223>  Nucleic acid coding sequence for Clostridium perfringens
       hemoglobin (hbCp)

<400>  12
atgttagatc aaaagacaat agatataata aaaagtacag taccagtttt aaaatccaat      60

ggattagaaa taacaaaaac tttctataaa aatatgtttg aacaaaatcc tgaagtaaaa     120

cctttgttca atatgaataa acaagaatct gaagagcaac caaaagcttt agcaatggca     180

atattagcag ttgctcaaaa tatagataac ttagaagcta taaaacctgt agttaataga     240

ataggagtta tacactgtaa tgcaaaagtt caaccagaac attatccaat agtaggtaaa     300

cacttattag gagcaataaa agaagtttta ggtgatggtg caactgaaga tattatcaat     360

gcgtgggcta aaacatatgg agttatagct gaagtattta taaataatga aaaagagatg     420

tatgcttcaa gatag                                                      435

<210>  13
<211>  409
<212>  PRT
<213>  Bacillus halodurans

<220>
<223>  Amino acid sequence of Bacillus halodurans flavohemoglobin
       (HmpBh)

<400>  13

Met Ser Thr Ala Thr Leu Ser Gln Glu Thr Lys Gln Ile Val Lys Ala
1               5                   10                  15

Thr Val Pro Ile Leu Glu Ala His Gly Glu Ala Ile Thr Lys His Phe
            20                  25                  30

Tyr Lys Arg Met Phe Ser His His Pro Glu Leu Leu Asn Ile Phe Asn
        35                  40                  45

Gln Thr His Gln Lys Gln Gly Arg Gln Pro Gln Ala Leu Ala Asn Ser
    50                  55                  60

Ile Tyr Ala Ala Ala Glu His Ile Asp Asn Leu Glu Ala Ile Leu Pro
65                  70                  75                  80

Val Val Ser Arg Ile Ala His Lys His Arg Ser Leu Asn Ile Lys Pro
                85                  90                  95

Glu Gln Tyr Pro Ile Val Gly Glu Asn Leu Leu Ala Ala Met Arg Glu
            100                 105                 110

Val Leu Gly Asp Ala Ala Ser Asp Asp Val Leu Glu Ala Trp Arg Glu
        115                 120                 125

Ala Tyr Glu Leu Ile Ala Asp Val Phe Ile Gln Val Glu Lys Lys Met
    130                 135                 140

Tyr Glu Glu Ala Ser Gln Ala Pro Gly Gly Trp Arg Glu Phe Arg Ser
145                 150                 155                 160

Phe Val Val Glu Lys Lys Gln Arg Glu Ser Ala Thr Ile Thr Ser Phe
                165                 170                 175

Tyr Leu Lys Pro Glu Asp Gly Lys Ala Leu Ala Ser Tyr Lys Pro Gly
            180                 185                 190

Gln Tyr Ile Thr Lys Val Thr Ile Pro Gly His Glu His Thr His Met
        195                 200                 205

Arg Gln Tyr Ser Leu Ser Asp Ala Pro Glu Lys Gly Tyr Tyr Arg Ile
    210                 215                 220

Thr Val Lys Arg Glu Glu Gly Asp Gly Asp Leu Pro Pro Gly Ile Val
225                 230                 235                 240

Ser Asn Tyr Leu His Gln His Ile His Glu Gly Asp Val Leu Glu Ile
                245                 250                 255

Thr Ala Pro Ala Gly Asp Phe Thr Leu Gln Glu Glu Gly Glu Arg Pro
            260                 265                 270

Ile Val Phe Ile Ser Gly Gly Val Gly Ile Thr Pro Leu Met Ser Met
        275                 280                 285

Phe Asn Thr Leu Met Gln Arg Gly Val Lys Arg Glu Val Ile Phe Ile
    290                 295                 300

His Ala Ala Ile Asn Gly Phe Tyr His Ala Met His Asp His Leu Ala
305                 310                 315                 320

Gly Thr Ala Ser Gln Gln Glu Asn Val His Tyr Ala Val Cys Glu Arg
                325                 330                 335

Pro Thr Pro Gly Asp Arg Met Asn Pro Phe Met Lys Lys Glu Gly Phe
            340                 345                 350

Ile Asp Glu Ser Phe Leu Arg Ser Ile Leu His Asp Arg Glu Ala Asp
        355                 360                 365

Phe Tyr Phe Cys Gly Pro Val Pro Phe Met Lys Thr Ile Ala Gln Ile
    370                 375                 380

Leu Lys Asp Trp Asp Val Pro Glu Gln Gln Val His Tyr Glu Phe Phe
385                 390                 395                 400

Gly Pro Ala Gly Thr Leu Ala Ser Ser
                405

<210>  14
<211>  1236
<212>  DNA
<213>  Bacillus halodurans

<220>
<223>  Nucleic acid coding sequence for Bacillus halodurans
       flavohemoglobin (hmpBh)

<400>  14
atgagtactg cgacgttaag ccaagaaacg aaacaaatcg ttaaagccac cgtaccgatt      60

ttagccgaac acggggaagc gataacgaag cacttttaca aacgaatgtt tagtcatcat     120

ccggaattgt taaacatctt taatcagacg catcaaaaac aaggaagaca accgcaagca     180

ctagcaaatt ccatctatgc ggccgctgag catatcgaca atttggaagc aattttacct     240

gttgtcagtc gtatcgccca caagcatcgc agcttaaaca ttaaaccaga acagtatccg     300

atcgtcgggg agaatttgct tgctgcgatg agagaagtac taggagatgc tgcatctgat     360

gacgtattgg aggcgtggcg ggaagcgtat gaactgatcg cagacgtgtt cattcaagtt     420

gagaaaaaaa tgtacgaaga ggctagtcag gctccgggtg gttggcgcga gttccgttcc     480

tttgttgttg aaaaaaagca aagggaaagt gcgacaatta catcatttta tttaaagcct     540

gaagacggga aagcacttgc ctcctacaaa ccagggcaat atattaccgt aaaagtaacg     600

attccaggac atgagcatac tcatatgcgg caatatagtt tatcagatgc cccagaaaaa     660

ggctattacc ggatcactgt taagcgtgag gagggggatg gcgatttgcc cccagggatc     720

gtctcgaatt atttgcatca acatatccat gagggggatg tgctagagat cacagcacca     780

gcaggagatt ttacgttaca ggaggaaggg gagcgtccaa ttgtgttcat aagcggtggc     840

gtagggataa cgcctcttat gagtatgttt aacactttaa tgcaaagagg agtaaaacgt     900

gaagtgatat ttattcatgc agcaatcaat ggtttttatc atgccatgca tgaccattta     960

gctcaaaccg ctagccagca agaaaatgtc cactatgctg tatgctacga acggccaaca    1020

ccaggggatc ggatgaatcc ttttatgaaa aaggaaggat ttatcgatga atccttcctt    1080

cgttccatcc tccacgatcg agaggcagac ttttattttt gcgggccagt cccatttatg    1140

aaaacgatcg ctcagatttt aaaagactgg gacgttcccg aacagcaggt tcattatgag    1200

ttttttggac ctgcaggaac gctagcgtct tcttag                              1236

<210>  15
<211>  399
<212>  PRT
<213>  Bacillus subtilis

<220>
<223>  Amino acid sequence of Bacillus subtilis flavohemoglobin (HmpBs)

<400>  15

Met Leu Asp Asn Lys Thr Ile Glu Ile Ile Lys Ser Thr Val Pro Val
1               5                   10                  15

Leu Gln Gln His Gly Glu Thr Ile Thr Gly Arg Phe Tyr Asp Arg Met
            20                  25                  30

Phe Gln Asp His Pro Glu Leu Leu Asn Ile Phe Asn Gln Thr Asn Gln
        35                  40                  45

Lys Lys Lys Thr Gln Arg Thr Ala Leu Ala Asn Ala Val Ile Ala Ala
    50                  55                  60

Ala Ala Asn Ile Asp Gln Leu Gly Asn Ile Ile Pro Val Val Lys Gln
65                  70                  75                  80

Ile Gly His Lys His Arg Ser Ile Gly Ile Lys Pro Glu His Tyr Pro
                85                  90                  95

Ile Val Gly Lys Tyr Leu Leu Ile Ala Ile Lys Asp Val Leu Gly Asp
            100                 105                 110

Ala Ala Thr Pro Asp Ile Met Gln Ala Trp Glu Lys Ala Tyr Gly Val
        115                 120                 125

Ile Ala Asp Ala Phe Ile Gly Ile Glu Lys Asp Met Tyr Glu Gln Ala
    130                 135                 140

Glu Glu Gln Ala Gly Gly Trp Lys Glu Tyr Lys Pro Phe Val Ile Ala
145                 150                 155                 160

Lys Lys Glu Arg Glu Ser Lys Glu Ile Thr Ser Phe Tyr Leu Lys Pro
                165                 170                 175

Glu Asp Ser Lys Pro Leu Pro Glu Phe Gln Ala Gly Gln Tyr Ile Ser
            180                 185                 190

Ile Lys Val Arg Ile Pro Asp Ser Glu Tyr Thr His Ile Arg Gln Tyr
        195                 200                 205

Ser Leu Ser Asp Met Pro Gly Lys Asp Tyr Tyr Arg Ile Ser Val Lys
    210                 215                 220

Lys Asp Gly Val Val Ser Ser Tyr Leu His Asp Gly Leu Gln Glu Gly
225                 230                 235                 240

Asp Ser Ile Glu Ile Ser Ala Pro Ala Gly Asp Phe Val Leu Asp His
                245                 250                 255

Ala Ser Gln Lys Asp Leu Val Leu Ile Ser Ala Gly Val Gly Ile Thr
            260                 265                 270

Pro Met Ile Ser Met Leu Lys Thr Ser Val Ser Lys Gln Pro Glu Arg
        275                 280                 285

Gln Ile Leu Phe Ile His Ala Ala Lys Asn Ser Glu Tyr His Ala Leu
    290                 295                 300

Arg His Glu Val Glu Glu Ala Ala Lys His Ser Ala Val Lys Thr Ala
305                 310                 315                 320

Phe Val Tyr Arg Glu Pro Thr Glu Glu Asp Arg Ala Gly Asp Leu His
                325                 330                 335

Phe His Glu Gly Gln Ile Asp Gln Gln Phe Leu Lys Glu Leu Ile Ala
            340                 345                 350

Asn Thr Asp Ala Asp Tyr Tyr Ile Cys Gly Ser Pro Ser Phe Ile Thr
        355                 360                 365

Ala Met His Lys Leu Val Ser Glu Leu Gly Ser Ala Pro Glu Ser Ile
    370                 375                 380

His Tyr Glu Leu Phe Gly Pro Gln Leu Ser Leu Ala Gln Ser Val
385                 390                 395

<210> 16
<211> 1200
<212> DNA
<213> Bacillus subtilis

<220>
<223> Nucleic acid coding sequence for Bacillus subtilis
flavohemoglobin (hmpBs)

<400> 16
atgttagata acaaaacaat cgaaattatt aaaagcactg tacccgtatt gcaacagcat 60

ggagaaacaa tcactggccg tttttacgat cggatgtttc aagaccatcc agagcttttg 120

aacattttta atcaaacaaa ccaaaagaaa aaaacacagc gaactgcact ggcaaatgcc 180

gttattgcag ctgcggcaaa cattgaccaa ttaggaaaca tcattccggt tgtcaaacaa 240

atcggccaca agcaccgcag tatcggaatt aagcctgagc actacccaat cgtcggtaaa 300

tatttgctga ttgcgattaa agatgtattg ggagacgctg caactcctga cattatgcag 360

gcatgggaaa aagcgtacgg agtcattgct gacgcgttca tcggaattga aaaagacatg 420

tatgaacagg ctgaagagca agccggcggc tggaaggagt ataagccatt cgttatcgca 480

aaaaaagagc gggaaagcaa agaaattaca tccttctatt tgaaaccgga agacagcaag 540

ccgttacctg aattccaagc agggcaatat atcagcatca aagtgcggat tcctgattct 600

gaatatacgc atatccgcca gtacagcctg tctgatatgc cgggaaaaga ctattatcga 660

atttcagtga agaaagatgg tgtcgtgtct tcctacttac acgatgggct gcaggaggga 720

gattcaatag agatcagcgc accggcaggg gactttgttt tagatcatgc atcacaaaaa 780

gatctcgttt tgatcagtgc cggagttggc attacaccaa tgatcagcat gctgaaaaca 840

tctgtttcca aacagcctga acggcaaatt ctgttcattc atgcggcgaa aaacagtgaa 900

tatcatgcat tgcgccatga agtagaagaa gcagcaaaac attctgctgt taaaacagca 960

tttgtgtatc gcgagccgac agaggaagac cgtgcaggtg atctgcattt tcatgaagga 1020

cagattgatc agcaattcct caaagaactg attgccaaca cagatgcgga ttactatatt 1080

tgcggttcac cgtcgtttat tacggcaatg cataagcttg tatcagagct cggatcagcg 1140

ccggagtcta tccattacga gctattcggc ccgcaattaa gtttggcgca gtccgtttga 1200

<210> 17
<211> 395
<212> PRT
<213> Erwinia chrysanthemi

<220>
<223> Amino acid sequence of Erwinia chrysanthemi flavohemoglobin
(HmpX)

<400> 17

Met Leu Asp Gln Gln Thr Ile Ala Thr Ile Lys Ser Thr Ile Pro Leu
1               5                   10                  15

Leu Ala Glu Thr Gly Pro Ala Leu Thr Ala His Phe Tyr Gln Arg Met
            20                  25                  30

Phe His His Asn Pro Glu Leu Lys Asp Ile Phe Asn Met Ser Asn Gln
        35                  40                  45

Arg Asn Gly Asp Gln Arg Glu Ala Leu Phe Asn Ala Ile Cys Ala Tyr
    50                  55                  60

Ala Thr His Ile Glu Asn Leu Pro Ala Leu Leu Pro Ala Val Glu Arg
65                  70                  75                  80

Ile Ala Gln Lys His Ala Ser Phe Asn Ile Gln Pro Glu Gln Tyr Gln
                85                  90                  95

Ile Val Gly Thr His Leu Leu Ala Thr Leu Glu Glu Met Phe Gln Pro
            100                 105                 110

Gly Gln Ala Val Leu Asp Ala Trp Gly Lys Arg Tyr Gly Val Leu Ala
        115                 120                 125

Asn Val Phe Ile Gln Arg Glu Ser Asp Ile Tyr Gln Gln Ser Ala Gly
    130                 135                 140

Gln Asn Gly Gly Trp His Gly Ile Arg Pro Phe Arg Ile Val Ala Lys
145                 150                 155                 160

Gln Pro Gln Ser Ser Leu Ile Thr Ser Phe Thr Leu Glu Pro Val Asp
                165                 170                 175

Gly Gly Pro Ile Ala Ala Phe Arg Pro Gly Gln Tyr Leu Ala Val Tyr
            180                 185                 190

Ile Arg Asp Lys Arg Phe Glu Tyr Gln Glu Ile Arg Gln Tyr Ser Leu
        195                 200                 205

Thr Asn Glu Pro Asn Gly Arg Tyr Tyr Arg Ile Ala Val Lys Arg Glu
    210                 215                 220

Thr Met Gly Ser Val Ser Gly Tyr Leu His Asp Val Ala Arg Glu Gly
225                 230                 235                 240

Asp Val Ile Glu Leu Ala Ala Pro His Gly Asp Phe Tyr Leu Glu Val

                245                 250                 255

Thr Pro Glu Thr Pro Val Ala Leu Ile Ser Ala Gly Val Gly Gln Thr
            260                 265                 270

Pro Met Leu Ser Met Leu His Ser Leu Lys Asn Gln Gln His Gln Ala
        275                 280                 285

Asp Ile Phe Trp Leu His Ala Ala Glu Asn Thr Glu Val His Ala Phe
    290                 295                 300

Ala Asp Glu Ile Ala Asp Val Ala Ala Thr Leu Pro Gln Leu Gln Ser
305                 310                 315                 320

Tyr Val Trp Tyr Arg Glu Ala Ser Ser Glu Ala Ala Arg Ser Ala His
                325                 330                 335

Ala Phe His Gly Leu Met Ala Leu Lys Asp Leu Pro Thr Pro Leu Pro
            340                 345                 350

Met Thr Asn Leu His Cys Tyr Leu Cys Gly Pro Val Ala Phe Met Gln
        355                 360                 365

Phe Ala Ala Arg Gln Leu Leu Glu Leu Gly Ile Thr Glu Ser Gln Ile
    370                 375                 380

His Tyr Glu Cys Phe Gly Pro His Lys Val Ile
385                 390                 395

<210> 18
<211> 1188
<212> DNA
<213> Erwinia chrysanthemi

<220>
<223> Nucleic acid coding sequence for Erwinia chrysanthemi
flavohemoglobin (hmpX)

<400> 18
atgctggatc aacaaaccat cgccaccatc aaatccacca ttcctctgct ggccgaaacc 60

ggtccggcgt tgaccgctca cttttatcag cggatgttcc accataaccc ggaactgaaa 120

gatatcttca atatgagtaa tcagcgtaat ggcgaccagc gcgaagcgct gttcaatgcc 180

atttgcgcct acgcgacgca tatcgaaaac ctgccggcgt tgctgcccgc ggtagaacgt 240

atcgcgcaaa agcatgccag cttcaatatt cagccggaac agtatcagat agtgggtact 300

cacctgctgg caacgctgga agaaatgttc cagcccggtc aggcggtgct ggatgcctgg 360

ggcaaacgct acggcgtgct ggcgaatgtg tttatccagc gggaaagcga catttatcag 420

caaagcgccg gacaaaacgg cggctggcat ggcatccggc cgtttcgcat tgtggcgaaa 480

cagccccaga gcagcctgat caccagcttc acgctggaac cggtggatgg cggcccgatc 540

gccgcattcc gtcccggcca gtatctggcg gtctacatcc gcgataagcg ctttgaatat 600

caggagattc gtcagtactc actaacgaac gaacccaacg ggcgctatta ccgtatcgcg 660

gtcaaacggg aaacgatggg cagcgtatcg ggctatttgc acgatgtcgc ccgcgaaggc 720

gatgtgattg aactggccgc cccgcacggc gatttctacc ttgaggtcac gccagagacg 780

ccggtagcgc tgatttccgc cggtgttggt caaacaccga tgctcagcat gctgcacagc 840

ctgaaaaatc agcagcatca ggcggatatc ttctggttac acgcggctga aaacacagaa 900

gtacatgcct tcgccgacga aatcgccgat gttgccgcta cgctgccgca gttgcaatcc 960

tatgtctggt atcgggaagc ctcatcggaa gcggcacgtt cggcgcatgc ttttcacgga 1020

ttgatggcgt tgaaggacct cccgacgccg ctgccgatga caaacctgca ctgttattta 1080

tgcggcccgg tcgcctttat gcaattcgcc gcccgacaat tgttggagtt ggggattacc 1140

gaatcgcaga ttcattacga atgctttggt ccgcacaagg ttatataa 1188

<210> 19
<211> 396
<212> PRT
<213> Escherichia coli

<220>
<223> Amino acid sequence of Escherichia coli flavohemoglobin (Hmp)

<400> 19

<210>  20
<211>  1191
<212>  DNA
<213>  Escherichia coli

<220>
<223>  Nucleic acid coding sequence for Escherichia coli flavohemoglobin
       (hmp)

<400>  20
atgcttgacg ctcaaaccat cgctacagta aaagccacca tccctttact ggtggaaacg      60

gggccaaagt taaccgccca tttctacgac cgtatgttta ctcataaccc agaactcaaa     120

gaaattttta acatgagtaa ccagcgtaat ggcgatcaac gtgaagccct gtttaacgct     180

attgccgcct acgccagtaa tattgaaaac ctgcctgcgc tgctgccagc ggtagaaaaa     240

atcgcgcaga agcacaccag cttccagatc aaaccggaac agtacaacat cgtcggtgaa     300

cacctgttgg caacgctgga cgaaatgttc agcccggggc aggaagtgct ggacgcgtgg     360

ggtaaagcct atggtgtact ggctaatgta tttatcaatc gcgaggcgga aatctataac     420

gaaaacgcca gcaaagccgg tggttgggaa ggtactcgcg atttccgcat tgtggctaaa     480

acaccgcgca gcgcgcttat caccagcttc gaactggagc cggtcgacgg tggcgcagtg     540

gcagaatacc gtccggggca atatctcggc gtctggctga agccggaagg tttcccacat     600

caggaaattc gtcagtactc tttgactcgc aaaccggatg gcaaaggcta tcgtattgcg     660

gtgaaacgcg aagagggtgg gcaggtatcc aactggttgc acaatcacgc caatgttggc     720

gatgtcgtga aactggtcgc tccggcaggt gatttcttta tggctgtcgc agatgacaca     780

ccagtgacgt taatctctgc cggtgttggt caaacgccaa tgctggcaat gctcgacacg     840

ctggcaaaag caggccacac agcacaagtg aactggttcc atgcggcaga aaatggcgat     900

gttcacgcct ttgccgatga agttaaggaa ctggggcagt cactgccgcg ctttaccgcg     960

cacacctggt atcgtcagcc gagcgaagcc gatcgcgcta aaggtcagtt tgatagcgaa    1020

ggtctgatgg atttgagcaa actggaaggt gcgttcagcg atccgacaat gcagttctat    1080

ctctgcggcc cggttggctt catgcagttt accgcgaaac agttagtgga tctgggcgtg    1140

aagcaggaaa acattcatta cgaatgcttt ggcccgcata aggtgctgta a             1191

<210>  21
<211>  393
<212>  PRT
<213>  Pseudomonas aeruginosa

<220>
<223>  Amino acid sequence of Pseudomonas aeruginosa flavohemoglobin
       (HmpPa)

<400>  21

Met Leu Ser Asn Ala Gln Arg Ala Leu Ile Lys Ala Thr Val Pro Leu
1               5                   10                  15

Leu Glu Thr Gly Gly Glu Ala Leu Ile Thr His Phe Tyr Arg Thr Met
            20                  25                  30

Leu Gly Glu Tyr Pro Glu Val Arg Pro Leu Phe Asn Gln Ala His Gln
        35                  40                  45

Ala Ser Gly Asp Gln Pro Arg Ala Leu Ala Asn Gly Val Leu Met Tyr
    50                  55                  60

Ala Arg His Ile Asp Gln Leu Gln Glu Leu Gly Pro Leu Val Ala Lys
65                  70                  75                  80

Val Val Asn Lys His Val Ser Leu Gln Val Leu Pro Glu His Tyr Pro
                85                  90                  95

Ile Val Gly Thr Cys Leu Leu Arg Ala Ile Arg Glu Val Leu Gly Glu
            100                 105                 110

Gln Ile Ala Thr Asp Glu Val Leu Glu Ala Trp Gly Ala Ala Tyr Gln
        115                 120                 125

Gln Leu Ala Asp Leu Leu Ile Glu Ala Glu Glu Ser Val Tyr Ala Ala
    130                 135                 140

Ser Ala Gln Ala Asp Gly Gly Trp Arg Gly Val Arg Arg Phe Arg Val
145                 150                 155                 160

Ala Arg Lys Gln Ala Glu Ser Glu Glu Ile Thr Ser Phe Tyr Leu Glu
                165                 170                 175

Pro Val Asp Gly Gln Pro Leu Leu Ala Phe Gln Pro Gly Gln Tyr Ile
            180                 185                 190

Gly Leu Arg Leu Asp Ile Asp Gly Glu Glu Val Arg Arg Asn Tyr Ser

195                 200                 205

Leu Ser Ala Ala Ser Asn Gly Arg Glu Tyr Arg Ile Ser Val Lys Arg
    210                 215                 220

Glu Ala Gly Gly Arg Val Ser Asn Tyr Leu His Asp Arg Val Ala Glu
225                 230                 235                 240

Gly Asp Glu Leu Asp Leu Phe Pro Pro Ala Gly Asp Phe Val Leu Arg
                245                 250                 255

Asp Ser Asp Lys Pro Leu Val Leu Ile Thr Ala Gly Val Gly Ile Thr
            260                 265                 270

Pro Ala Leu Ala Met Leu Gln Glu Ala Leu Pro Gln Ala Arg Pro Ile
        275                 280                 285

Arg Phe Ile His Cys Ala Arg His Gly Gly Val His Ala Phe Arg Asp
    290                 295                 300

Trp Ile Glu Asp Val Ser Ala Gln His Glu Gln Val Glu His Phe Phe
305                 310                 315                 320

Cys Tyr Ser Glu Pro Arg Ala Gly Asp Ser Ala Asp Ala Glu Gly Leu
                325                 330                 335

Leu Ser Arg Glu Lys Leu Ala Asp Trp Leu Pro Gln Glu Arg Asp Leu
            340                 345                 350

Asp Ala Tyr Phe Leu Gly Pro Arg Pro Phe Met Ala Gln Val Lys Arg
        355                 360                 365

His Leu Ala Asp Leu Gly Val Pro Ser Gln Gln Cys His Tyr Glu Phe
    370                 375                 380

Phe Gly Pro Ala Ala Ala Leu Asp Ala
385                 390

<210>  22
<211>  1182
<212>  DNA
<213>  Pseudomonas aeruginosa

<220>
<223>  Nucleic acid coding sequence for Pseudomonas
       aeruginosaflavohemoglobin (hmpPa)

<400>  22
atgttgtcca atgcccaacg tgccctcatc aaagccaccg taccgctgct ggagaccggc      60

ggtgaagcgc tgatcaccca tttctaccgc accatgctcg gcgagtaccc cgaggttcgc 120

ccgctgttca accaggccca ccaggccagc ggcgaccagc cgcgggcgct ggccaatggc 180

gtgctgatgt acgccaggca catcgaccag ttgcaggaac tcggcccgct ggtggccaag 240

gtggtgaaca agcatgtctc cctgcaggtg ctgcccgaac actatccgat cgtcggcact 300

tgcctgttgc gggcgatccg cgaggtcctc ggcgaacaga tcgccaccga cgaagtcctc 360

gaagcctggg gcgcggccta ccagcaactg gccgacctgc tgatcgaggc cgaggagtcg 420

gtgtatgccg ccagcgccca ggccgatggc ggctggcgcg gcgtgcggcg cttccgcgtg 480

gcacgcaagc aggcggagag cgaggaaatc acctcgttct acctggagcc ggtggacggc 540

cagccgctgc tggcgttcca gccgggacag tacatcggcc tgcggctgga catcgacggc 600

gaggaagtgc gccgcaacta ttcgctttcc gctgccagca acggccgcga ataccgcatc 660

agcgtcaagc gcgaggcggg tggacgggtc tccaattacc tgcatgaccg ggtcgccgag 720

ggcgacgagc tggatctgtt tccgccggct ggcgatttcg tcctgcgtga cagcgacaag 780

ccgctggtgc tgatcaccgc cggcgtcggc atcaccccgg ccctggccat gttgcaggaa 840

gcgctgccgc aggcgcggcc gatccgcttc atccattgcg cccggcatgg cggcgtgcat 900

gccttccgcg actggatcga agacgtgtcg gcgcagcacg agcaggtcga gcacttcttc 960

tgctacagcg aaccgcgcgc cggcgacagt gccgacgccg agggattgct gagccgcgag 1020

aaactggccg actggttgcc gcaggagcgc gacctggacg cctacttcct cggaccccgg 1080

cctttcatgg cgcaggtcaa gcgccacctg gccgacctcg gcgtaccctc gcagcaatgc 1140

cactacgagt tcttcggtcc ggccgccgcg ctggacgcct ga 1182

<210> 23
<211> 403
<212> PRT
<213> Deinococcus radiodurans

<220>
<223> Amino acid sequence of Deinococcus radiodurans flavohemoglobin
(HmpDr)

<400> 23

Met Leu Thr Pro Glu Gln Lys Ala Ile Val Lys Ala Thr Val Pro Ala
1               5                   10                  15

Leu Glu Ala His Gly Glu Thr Ile Thr Arg Thr Phe Tyr Ala Ser Met
            20                  25                  30

Phe Ala Ala His Pro Glu Leu Leu Asn Ile Phe Asn Pro Ala Asn Gln
        35                  40                  45

Gln Thr Gly Lys Gln Ala Arg Ser Leu Ala Ala Ser Val Leu Ala Tyr
    50                  55                  60

Ala Ala His Ile Asp His Pro Glu Ala Leu Gly Gly Met Val Gly Arg
65                  70                  75                  80

Ile Ala His Lys His Val Ser Leu Glu Val Leu Pro Glu His Tyr Pro
                85                  90                  95

Ile Val Gly Gln Tyr Leu Leu Gly Ala Ile Ala Gly Val Leu Gly Asp
            100                 105                 110

Ala Ala Lys Pro Glu Ile Leu Asp Ala Trp Ala Ala Ala Tyr Gly Glu
        115                 120                 125

Leu Ala Asp Leu Met Ile Gly Ile Glu Lys Gly Met Tyr Asp Ala Gly
    130                 135                 140

Ala Gly Gln Pro Gly Gly Trp Arg Asp Phe Arg Pro Phe Arg Val Ala
145                 150                 155                 160

Arg Lys Val Ala Glu Ser Arg Val Ile Thr Ser Phe Val Leu Glu Pro
                165                 170                 175

Val Gly Gly Gly Ala Leu Pro Ala Tyr Gln Pro Gly Gln Tyr Leu Ser
            180                 185                 190

Leu Lys Val Lys Val Pro Gly Gln Glu Arg Trp Gln Ile Arg Gln Tyr
        195                 200                 205

Ser Leu Ser Asp Ala Pro Ser Pro Asp His Tyr Arg Ile Ser Val Lys
    210                 215                 220

Arg Glu Gly Gly Gly Leu Val Ser Glu Tyr Leu His Gly Ala Val Gln
225                 230                 235                 240

Glu Gly Asp Glu Leu Leu Val His Val Pro Ala Gly Asp Phe Val Leu
                245                 250                 255

Gln Gln Ser Glu Arg Pro Val Val Leu Ile Ser Ala Gly Val Gly Ile
            260                 265                 270

Thr Pro Met Leu Ala Met Val Gln Thr Leu Ala Gln Ala Gly Ser Gln
        275                 280                 285

Arg Pro Val Thr Phe Ile His Ala Ala Gln Asn Gly Ser Val His Ala
    290                 295                 300

Phe Arg Asp Asp Val Ala Arg Leu Thr His Glu Tyr Pro His Phe Arg
305                 310                 315                 320

Lys Val Val Phe Tyr Asp Glu Ala Gly Pro Asp Asp Gln Leu Gly Thr
                325                 330                 335

His His Asp Val Ala Gly Arg Leu Ser Leu Asp Ala Val Arg Gly Ala
            340                 345                 350

Leu Pro Ala Gly Glu Ala Glu Phe Tyr Tyr Cys Gly Pro Ala Gly Phe
        355                 360                 365

Ala Gly Ala Val Glu Ala Ile Leu Asp Asp Leu Gln Val Pro Ala Glu
    370                 375                 380

Arg Arg Phe Thr Glu Thr Phe Gly Pro Ser Gln Ser Phe Ala Pro Val
385                 390                 395                 400

Ile Leu Gly

<210>  24
<211>  1212
<212>  DNA
<213>  Deinococcus radiodurans

<220>
<223>  Nucleic acid coding sequence for Deinococcus radiodurans
       flavohemoglobin (hmpDr)

<400>  24
atgctgaccc ccgaacagaa agccatcgtc aaggccaccg ttcccgcgct ggaagcgcac      60

ggcgagacga tcacccgcac cttctacgcc tcgatgttcg ccgcgcaccc cgaactgctc     120

aacatcttca accccgccaa ccagcagacc ggcaagcagg cccgcagcct ggcggcgtcg     180

gtgctggcct acgcggcaca tatcgaccat ccggaggcgc tgggcggcat ggtggggcgg     240

attgcccaca agcacgtcag cctggaagtc ctgcccgagc attaccccat cgtgggccag     300

tacctgctcg gcgcgattgc cggggtgctg ggcgacgccg ccaagccgga gattctggac     360

gcctgggccg ccgcctacgg ggagctggcc gacctgatga tcgggatcga gaagggcatg     420

tacgacgccg gagccgggca gcccggcggc tggcgcgact tccggccctt ccgggtcgcc     480

cgcaaggtgg ccgagagccg cgtgatcacc tccttcgtgc tcgagcccgt gggcggcggg     540

gcgttgcccg cctatcagcc ggggcagtac cttagcctga aggtgaaggt gccggggcag     600

gagcggtggc aaatccggca gtacagcctc tcggacgcgc cgagcccgga ccactaccgc     660

atctcggtca agcgcgaggg cggcgggctg gtgtcggagt acctgcacgg cgcggtgcag 720

gagggcgacg aactgctcgt tcacgttccg gcgggcgact tcgtgctgca acagagtgag 780

cgcccggtgg tcctgatcag cgcaggcgtg ggcatcaccc cgatgctggc gatggtgcag 840

acgctggccc aggcggggtc gcagcggccc gtcaccttca tccacgcggc gcagaacggc 900

tccgttcacg ccttccgcga cgacgtggcg cggctgacgc acgagtaccc gcactttcgc 960

aaggtggtct tctacgacga ggccggcccc gacgaccagc tcggcaccca ccacgacgtg 1020

gccgggcgcc tgagcctgga cgccgtgcgg ggcgcgctcc cggcgggtga ggccgagttc 1080

tactactgcg gtccggcggg cttcgccggg gcggtggaag ccatactcga cgacttgcag 1140

gtgcccgccg agcgacgctt taccgaaacc ttcgggccga gccagagctt cgcgccggtg 1200

attctgggct ga 1212

<210> 25
<211> 396
<212> PRT
<213> Salmonella enterica

<220>
<223> Amino acid sequence of Salmonella enterica serovar Typhi CT18
      flavohemoglobin (HmpSt)

<400> 25

Met Leu Asp Ala Gln Thr Ile Ala Thr Val Lys Ala Thr Ile Pro Leu
1               5                   10                  15

Leu Val Glu Thr Gly Pro Lys Leu Thr Ala His Phe Tyr Asp Arg Met
            20                  25                  30

Phe Thr His Asn Pro Glu Leu Lys Glu Ile Phe Asn Met Ser Asn Gln
        35                  40                  45

Arg Asn Gly Asp Gln Arg Glu Ala Leu Phe Asn Ala Ile Ala Ala Tyr
    50                  55                  60

Ala Ser Asn Ile Glu Asn Leu Pro Ala Leu Leu Pro Ala Val Glu Lys
65                  70                  75                  80

Ile Ala Gln Lys His Thr Ser Phe Gln Ile Lys Pro Glu Gln Tyr Asn
                85                  90                  95

Ile Val Gly Thr His Leu Leu Ala Thr Leu Asp Glu Met Phe Asn Pro
            100                 105                 110

Gly Gln Glu Val Leu Asp Ala Trp Gly Lys Ala Tyr Gly Val Leu Ala
        115                 120                 125

Asn Val Phe Ile His Arg Glu Ala Glu Ile Tyr His Glu Asn Ala Ser
    130                 135                 140

Lys Asp Gly Gly Trp Glu Gly Thr Arg Pro Phe Arg Ile Val Ala Lys
145                 150                 155                 160

Thr Pro Arg Ser Ala Leu Ile Thr Ser Phe Glu Phe Glu Pro Val Asp
                165                 170                 175

Gly Gly Thr Val Ala Glu Tyr Arg Pro Gly Gln Tyr Leu Gly Val Trp
            180                 185                 190

Leu Lys Pro Glu Gly Phe Ala His Gln Glu Ile Arg Gln Tyr Ser Leu
        195                 200                 205

Thr Arg Lys Pro Asp Gly Lys Gly Tyr Arg Ile Ala Val Lys Arg Glu
    210                 215                 220

Asp Gly Gly Gln Val Ser Asn Trp Leu His His His Ala Asn Val Gly
225                 230                 235                 240

Asp Val Val His Leu Ala Ala Pro Ala Gly Asp Phe Phe Met Asn Val
                245                 250                 255

Ala Ala Asp Thr Pro Val Ser Leu Ile Ser Ala Gly Val Gly Gln Thr
            260                 265                 270

Pro Met Leu Ala Met Leu Asp Thr Leu Ala Lys Glu Gln His Thr Ala
        275                 280                 285

Gln Val Asn Trp Phe His Ala Ala Glu Asn Gly Asp Val His Ala Phe
    290                 295                 300

Ala Asp Glu Val Ser Glu Leu Gly Arg Thr Leu Pro Arg Phe Thr Ala
305                 310                 315                 320

His Thr Trp Tyr Arg Glu Pro Thr Glu Ala Asp Arg Ala Gln Arg Val
                325                 330                 335

Phe Asp Ser Glu Gly Leu Met Asp Leu Ser Lys Leu Glu Ala Ala Ile
            340                 345                 350

Ser Asp Pro Ala Met Gln Phe Tyr Leu Cys Gly Pro Val Gly Phe Met
        355                 360                 365

Gln Phe Ala Ala Lys Gln Leu Val Ser Leu Gly Val Asn Asn Glu Asn

370                 375                 380

Ile His Tyr Glu Cys Phe Gly Pro His Lys Val Leu
385                 390                 395

<210>  26
<211>  1191
<212>  DNA
<213>  Salmonella enterica

<220>
<223>  Nucleic acid coding sequence for Salmonella enterica serovar
       Typhi CT18 flavohemoglobin (hmpSt)

<400>  26
atgcttgacg cacaaaccat cgctacagta aaggccacca ttcccctgct ggttgaaaca      60

ggaccgaaac tgaccgccca cttctacgac cgtatgttta cgcataaccc ggagctcaaa     120

gaaatcttca atatgagcaa ccagcgtaac ggtgatcagc gcgaagccct gtttaatgct     180

atcgcggcct acgccagcaa tatcgaaaat ttaccggcgt tgctgccggc ggtagaaaaa     240

atcgcgcaga agcacaccag cttccagatt aagccggagc agtacaacat cgtcgggacg     300

catctgctgg cgacgctgga tgaaatgttc aacccgggcc aggaagtgct ggacgcatgg     360

ggtaaagcct atggcgtact ggctaacgtc tttattcatc gggaagccga gatttatcac     420

gagaacgcca gcaaagacgg cggctgggaa ggcacgcgcc ccttccgcat cgttgcgaaa     480

accccgcgta gcgcactgat caccagcttt gagtttgaac cggtcgacgg cggtacggtg     540

gcggaatacc gtcccgggca gtatctgggc gtctggctga agccggaagg ttttgcgcat     600

caggagatcc gccaatattc actgacccgt aaacccgatg gcaaagggta ccgtattgcc     660

gtgaagcgtg aagacggcgg ccaggtatca aactggttac accatcacgc caacgtaggc     720

gatgtggtgc atctggctgc gccggcaggt gacttcttta tgaatgtcgc cgctgatacc     780

cccgtttcgc tgatttccgc tggcgtcggc cagacgccga tgttggcgat gctcgatacg     840

ctggcgaaag aacagcatac cgcgcaggtg aactggttcc acgcggcgga gaacggcgac     900

gtccatgcgt ttgccgacga agtgagcgag ctgggccgta cactgccgcg tttcactgcc     960

catacctggt accgcgagcc aaccgaggcc gatcgcgccc aacgcgtctt tgacagcgaa    1020

gggctgatgg atttaagcaa actggaagcc gcgatcagcg atccggcaat gcagttctat    1080

ctttgcggcc cggtaggctt tatgcagttt gccgcaaaac aactggtttc acttggcgtg    1140

aataacgaaa acattcatta cgaatgcttc ggcccgcata aagtgctgta a             1191

<210>  27
<211>  396
<212>  PRT
<213>  Salmonella typhimurium

<220>
<223>  Amino acid sequence of Salmonella typhimurium flavohemoglobin
      (HmpStm)

<400>  27

Met Leu Asp Ala Gln Thr Ile Ala Thr Val Lys Ala Thr Ile Pro Leu
1               5                   10                  15

Leu Val Glu Thr Gly Pro Lys Leu Thr Ala His Phe Tyr Asp Arg Met
            20                  25                  30

Phe Thr His Asn Pro Glu Leu Lys Glu Ile Phe Asn Met Ser Asn Gln
        35                  40                  45

Arg Asn Gly Asp Gln Arg Glu Ala Leu Phe Asn Ala Ile Ala Ala Tyr
    50                  55                  60

Ala Ser Asn Ile Glu Asn Leu Pro Ala Leu Leu Pro Ala Val Glu Lys
65                  70                  75                  80

Ile Ala Gln Lys His Thr Ser Phe Gln Ile Lys Pro Glu Gln Tyr Asn
                85                  90                  95

Ile Val Gly Thr His Leu Leu Ala Thr Leu Asp Glu Met Phe Asn Pro
            100                 105                 110

Gly Gln Glu Val Leu Asp Ala Trp Gly Lys Ala Tyr Gly Val Leu Ala
        115                 120                 125

Asn Val Phe Ile His Arg Glu Ala Glu Ile Tyr His Glu Asn Ala Ser
    130                 135                 140

Lys Asp Gly Gly Trp Glu Gly Thr Arg Pro Phe Arg Ile Val Ala Lys
145                 150                 155                 160

Thr Pro Arg Ser Ala Leu Ile Thr Ser Phe Glu Phe Glu Pro Val Asp
                165                 170                 175

Gly Gly Thr Val Ala Glu Tyr Arg Pro Gly Gln Tyr Leu Gly Val Trp
            180                 185                 190

Leu Lys Pro Glu Gly Phe Ala His Gln Glu Ile Arg Gln Tyr Ser Leu
        195                 200                 205

Thr Arg Lys Pro Asp Gly Lys Gly Tyr Arg Ile Ala Val Lys Arg Glu
    210                 215                 220

Asp Gly Gly Gln Val Ser Asn Trp Leu His His His Ala Ser Val Gly
225                 230                 235                 240

Asp Gly Val His Leu Ala Ala Pro Ala Gly Asp Phe Phe Met Asn Val
                245                 250                 255

Ala Ala Asp Thr Pro Val Ser Leu Ile Ser Ala Gly Val Gly Gln Thr
            260                 265                 270

Pro Met Leu Ala Met Leu Asp Thr Leu Ala Lys Glu Gln His Thr Ala
        275                 280                 285

Gln Val Asn Trp Phe His Ala Ala Glu Asn Gly Asp Val His Ala Phe
    290                 295                 300

Ala Asp Glu Val Ser Glu Leu Gly Arg Thr Leu Pro Arg Phe Thr Ala
305                 310                 315                 320

His Thr Trp Tyr Arg Glu Pro Thr Glu Ser Asp Arg Ala Gln Arg Leu
                325                 330                 335

Phe Asp Ser Glu Gly Leu Met Asp Leu Ser Lys Leu Glu Ala Ala Ile
            340                 345                 350

Ser Asp Pro Ala Met Gln Phe Tyr Leu Cys Gly Pro Val Gly Phe Met
        355                 360                 365

Gln Phe Ala Ala Lys Gln Leu Val Ser Leu Gly Val Asn Asn Glu Asn
    370                 375                 380

Ile His Tyr Glu Cys Phe Gly Pro His Lys Val Leu
385                 390                 395

<210>  28
<211>  1191
<212>  DNA
<213>  Salmonella typhimurium

<220>
<223>  Nucleic acid coding sequence for Salmonella typhimurium

<400>  28
atgcttgacg cacaaaccat cgctacagta aaggccacca ttcccctgct ggttgaaaca      60

ggaccgaaac tgaccgccca cttctacgac cgtatgttta cgcataaccc ggagctcaaa     120

gaaatcttca atatgagcaa ccagcgtaac ggcgatcagc gtgaagccct gtttaatgct     180

atcgcggcct acgccagcaa tatcgaaaat ttaccggcgt tgctgccggc ggtagaaaaa     240

atcgcgcaga agcacaccag cttccagatt aagccggagc agtacaacat cgtcggacca 300

catctgctgg cgacgctgga cgaaatgttc aacccgggcc aggaagtgct ggacgcgtgg 360

ggcaaagcct atggcgtact ggctaacgtc tttattcatc gggaagccga gatttatcac 420

gagaacgcca gcaaagacgg cggctgggaa ggcacgcgcc ccttccgcat cgttgcgaaa 480

accccgcgta gcgcactgat caccagcttt gaatttgaac cagtcgacgg cggtacggtg 540

gcggaatacc gtcccgggca gtatctgggc gtctggctga agccggaagg ttttgcgcat 600

caggtgttcc gccaatattc actgacccgt aaacccgatg gcaaagggta ccgtattgcc 660

gtgaagcgtg aagacggcgg ccaggtatca aactggttac accatcacgc cagcgtaggc 720

gatgtggtgc atctggctgc gccggcaggt gacttcttta tgaatgtcgc cgctgatacc 780

cccgtttcgc tgatttccgc tggcgtcggc cagacgccga tgttagcgat gctcgatacg 840

ctggcgaaag aacagcatac cgcgcaggtg aactggttcc acgcggcgga gaacggcgac 900

gtccatgcgt ttgccgacga agtgagcgag ctgggccgta cactgccgcg tttcactgcc 960

catacctggt accgcgagcc gactgaggcc gatcgcgccc aacgcctctt cgacagcgaa 1020

gggctgatgg atttaagcaa actggaagcc gcgatcagcg atccagcaat gcagttctat 1080

ctttgcggcc cggtaggctt tatgcagttt gccgcaaaac aactggtttc acttggcgtg 1140

aataacgaaa acattcatta cgaatgcttc ggcccgcata aagtgctgta a 1191

<210> 29
<211> 399
<212> PRT
<213> Saccharomyces cerevisiae

<220>
<223> Amino acid sequence of Saccharomyces cerevisiae flavohemoglobin
(Yhb)

<400> 29

Met Leu Ala Glu Lys Thr Arg Ser Ile Ile Lys Ala Thr Val Pro Val
1               5                   10                  15

Leu Glu Gln Gln Gly Thr Val Ile Thr Arg Thr Phe Tyr Lys Asn Met
            20                  25                  30

Leu Thr Glu His Thr Glu Leu Leu Asn Ile Phe Asn Arg Thr Asn Gln
        35                  40                  45

Lys Val Gly Ala Gln Pro Asn Ala Leu Ala Thr Thr Val Leu Ala Ala
    50                  55                  60

Ala Lys Asn Ile Asp Asp Leu Ser Val Leu Met Asp His Val Lys Gln
65                  70                  75                  80

Ile Gly His Lys His Arg Ala Leu Gln Ile Lys Pro Glu His Tyr Pro
                85                  90                  95

Ile Val Gly Glu Tyr Leu Leu Lys Ala Ile Lys Glu Val Leu Gly Asp
            100                 105                 110

Ala Ala Thr Pro Glu Ile Ile Asn Ala Trp Gly Glu Ala Tyr Gln Ala
        115                 120                 125

Ile Ala Asp Ile Phe Ile Thr Val Glu Lys Lys Met Tyr Glu Glu Ala
    130                 135                 140

Leu Trp Pro Gly Trp Lys Pro Phe Glu Ile Thr Ala Lys Glu Tyr Val
145                 150                 155                 160

Ala Ser Asp Ile Val Glu Phe Thr Val Lys Pro Lys Phe Gly Ser Gly
                165                 170                 175

Ile Glu Leu Glu Ser Leu Pro Ile Thr Pro Gly Gln Tyr Ile Thr Val
            180                 185                 190

Asn Thr His Pro Ile Arg Gln Glu Asn Gln Tyr Asp Ala Leu Arg His
        195                 200                 205

Tyr Ser Leu Cys Ser Ala Ser Thr Lys Asn Gly Leu Arg Phe Ala Val
    210                 215                 220

Lys Met Glu Ala Ala Arg Glu Asn Phe Pro Ala Gly Leu Val Ser Glu
225                 230                 235                 240

Tyr Leu His Lys Asp Ala Lys Val Gly Asp Glu Ile Lys Leu Ser Ala
                245                 250                 255

Pro Ala Gly Asp Phe Ala Ile Asn Lys Glu Leu Ile His Gln Asn Glu
            260                 265                 270

Val Pro Leu Val Leu Leu Ser Ser Gly Val Gly Val Thr Pro Leu Leu
        275                 280                 285

Ala Met Leu Glu Glu Gln Val Lys Cys Asn Pro Asn Arg Pro Ile Tyr
    290                 295                 300

Trp Ile Gln Ser Ser Tyr Asp Glu Lys Thr Gln Ala Phe Lys Lys His
305                 310                 315                 320

Val Asp Glu Leu Leu Ala Glu Cys Ala Asn Val Asp Lys Ile Ile Val

325                 330                 335

His Thr Asp Thr Glu Pro Leu Ile Asp Ala Ala Phe Leu Lys Glu Lys
            340                 345                 350

Ser Pro Ala His Ala Asp Val Tyr Thr Cys Gly Ser Val Ala Phe Met
        355                 360                 365

Gln Ala Met Ile Gly His Leu Lys Glu Leu Glu His Arg Asp Asp Met
    370                 375                 380

Ile His Tyr Glu Pro Phe Gly Pro Lys Met Ser Thr Val Gln Val
385                 390                 395

<210>  30
<211>  1200
<212>  DNA
<213>  Saccharomyces cerevisiae

<220>
<223>  Nucleic acid coding sequence for Saccharomyces cerevisiae
       flavohemoglobin (YHB1)

<400>  30
atgctagccg aaaaaacccg ttccataatc aaagcaaccg ttcctgtttt ggaacagcag      60

ggcactgtca tcacgcgtac attctacaaa aatatgctta ctgaacacac cgaattactt     120

aatattttta acagaactaa tcaaaaggtt ggcgcacaac caaatgcctt ggccactact     180

gttttggcag cggctaagaa cattgacgat ttgtctgtgc ttatggacca tgtcaagcaa     240

attggccaca agcatcgtgc cttgcaaatt aaacctgagc attatccaat cgttggtgag     300

tacttgttaa aagcaatcaa ggaagtttta ggcgatgctg cgactccaga gataattaat     360

gcatggggcg aggcttacca agcaattgct gacatcttca tcactgttga aaaaaaaatg     420

tacgaagaag ctttatggcc aggttggaaa ccatttgaca ttactgccaa agagtacgtt     480

gcttcagata ttgttgaatt tactgttaag ccaaagttcg gttctggtat tgaattggaa     540

agcttgccaa tcactccagg tcagtatatt acggtaaaca ctcacccaat tagacaggaa     600

aaccaatacg acgctttaag acactactct ctttgttctg cttcaactaa aaatggttta     660

cgatttgcag taaagatgga agctgccaga gaaaattttc ctgcaggatt agtctctgaa     720

tatttacaca aggacgctaa agttggtgat gaaattaagc ttagtgctcc ggctggtgat     780

ttcgcaataa ataaagagtt gatacaccaa aacgaagttc cattggtgtt actatcgtct     840

ggtgttggtg ttacaccact tttagctatg ttagaagaac aagtcaaatg caacccaaat     900

aggccaattt actggattca atcttcctat gatgaaaaga ctcaagcatt caaaaaacat     960

gtagatgaac tattagctga atgtgcgaat gtcgacaaaa taattgttca tactgatact    1020

gaaccactga ttaatgctgc attcttgaag gaaaagtcac ctgcgcatgc cgatgtctac 1080

acttgcggct ctttagcttt tatgcaagct atgattggtc atctaaaaga actagagcat 1140

cgtgatgata tgattcacta tgaaccattt ggtccaaaga tgtcaaccgt gcaagtttag 1200

<210> 31
<211> 151
<212> PRT
<213> Homo sapiens

<220>
<223> Amino acid sequence of Homo sapiens neuroglobin (NGB)

<400> 31

Met Glu Arg Pro Glu Pro Glu Leu Ile Arg Gln Ser Trp Arg Ala Val
1               5                   10                  15

Ser Arg Ser Pro Leu Glu His Gly Thr Val Leu Phe Ala Arg Leu Phe
            20                  25                  30

Ala Leu Glu Pro Asp Leu Leu Pro Leu Phe Gln Tyr Asn Cys Arg Gln
        35                  40                  45

Phe Ser Ser Pro Glu Asp Cys Leu Ser Ser Pro Glu Phe Leu Asp His
    50                  55                  60

Ile Arg Lys Val Met Leu Val Ile Asp Ala Ala Val Thr Asn Val Glu
65                  70                  75                  80

Asp Leu Ser Ser Leu Glu Glu Tyr Leu Ala Ser Leu Gly Arg Lys His
                85                  90                  95

Arg Ala Val Gly Val Lys Leu Ser Ser Phe Ser Thr Val Gly Glu Ser
            100                 105                 110

Leu Leu Tyr Met Leu Glu Lys Cys Leu Gly Pro Ala Phe Thr Pro Ala
        115                 120                 125

Thr Arg Ala Ala Trp Ser Gln Leu Tyr Gly Ala Val Val Gln Ala Met
    130                 135                 140

Ser Arg Gly Trp Asp Gly Glu
145                 150

<210> 32
<211> 456
<212> DNA
<213> Homo sapiens

<220>
<223>  mRNA sequence for Homo sapiens neuroglobin (NGB)

<400>  32
atggagcgcc cggagcccga gctgatccgg cagagctggc gggcagtgag ccgcagcccg      60

ctggagcacg gcaccgtcct gtttgccagg ctgtttgccc tggagcctga cctgctgccc     120

ctcttccagt acaactgccg ccagttctcc agcccagagg actgtctctc ctcgcctgag     180

ttcctggacc acatcaggaa ggtgatgctc gtgattgatg ctgcagtgac caatgtggaa     240

gacctgtcct cactggagga gtaccttgcc agcctgggca ggaagcaccg ggcagtgggt     300

gtgaagctca gctccttctc gacagtgggt gagtctctgc tctacatgct ggagaagtgt     360

ctgggccctg ccttcacacc agccacacgg gctgcctgga gccaactcta cggggccgta     420

gtgcaggcca tgagtcgagg ctgggatggc gagtaa                               456

<210>  33
<211>  190
<212>  PRT
<213>  Homo sapiens

<220>
<223>  Amino acid sequence of Homo sapiens cytoglobin (Cgb)

<400>  33

Met Glu Lys Val Pro Gly Glu Met Glu Ile Glu Arg Arg Glu Arg Ser
1               5                   10                  15

Glu Glu Leu Ser Glu Ala Glu Arg Lys Ala Val Gln Ala Met Trp Ala
            20                  25                  30

Arg Leu Tyr Ala Asn Cys Glu Asp Val Gly Val Ala Ile Leu Val Arg
        35                  40                  45

Phe Phe Val Asn Phe Pro Ser Ala Lys Gln Tyr Phe Ser Gln Phe Lys
    50                  55                  60

His Met Glu Asp Pro Leu Glu Met Glu Arg Ser Pro Gln Leu Arg Lys
65                  70                  75                  80

His Ala Cys Arg Val Met Gly Ala Leu Asn Thr Val Val Glu Asn Leu
                85                  90                  95

His Asp Pro Asp Lys Val Ser Ser Val Leu Ala Leu Val Gly Lys Ala
            100                 105                 110

His Ala Leu Lys His Lys Val Glu Pro Val Tyr Phe Lys Ile Leu Ser

        115                 120                 125

Gly Val Ile Leu Glu Val Val Ala Glu Glu Phe Ala Ser Asp Phe Pro
    130                 135                 140

Pro Glu Thr Gln Arg Ala Trp Ala Lys Leu Arg Gly Leu Ile Tyr Ser
145                 150                 155                 160

His Val Thr Ala Ala Tyr Lys Glu Val Gly Trp Val Gln Gln Val Pro
                165                 170                 175

Asn Ala Thr Thr Pro Pro Ala Thr Leu Pro Ser Ser Gly Pro
            180                 185                 190

<210> 34
<211> 573
<212> DNA
<213> Homo sapiens

<220>
<223> mRNA sequence for Homo sapiens cytoglobin (CYGB)

<400> 34
atggagaaag tgccaggcga gatggagatc gagcgcaggg agcggagcga ggagctgtcc 60

gaggcggaga ggaaggcggt gcaggctatg tgggcccggc tctatgccaa ctgcgaggac 120

gtgggggtgg ccatcctggt gaggttcttt gtgaacttcc cctcggccaa gcagtacttc 180

agccagttca agcacatgga ggatcccctg gagatggagc ggagccccca gctgcggaag 240

cacgcctgcc gagtcatggg ggccctcaac actgtcgtgg agaacctgca tgaccccgac 300

aaggtgtcct ctgtgctcgc ccttgtgggg aaagcccacg ccctcaagca caaggtggaa 360

ccggtgtact tcaagatcct ctctggggtc attctggagg tggtcgccga ggaatttgcc 420

agtgacttcc cacctgagac gcagagagcc tgggccaagc tgcgtggcct catctacagc 480

cacgtgaccg ctgcctacaa ggaagtgggc tgggtgcagc aggtccccaa cgccaccacc 540

ccaccggcca cactgccctc ttcggggccg tag 573

<210> 35
<211> 154
<212> PRT
<213> Homo sapiens

<220>
<223> Amino acid sequence of Homo sapiens myoglobin (Mgb)

<400> 35

Met Gly Leu Ser Asp Gly Glu Trp Gln Leu Val Leu Asn Val Trp Gly
1               5                   10                  15

Lys Val Glu Ala Asp Ile Pro Gly His Gly Gln Glu Val Leu Ile Arg
            20                  25                  30

Leu Phe Lys Gly His Pro Glu Thr Leu Glu Lys Phe Asp Lys Phe Lys
        35                  40                  45

His Leu Lys Ser Glu Asp Glu Met Lys Ala Ser Glu Asp Leu Lys Lys
    50                  55                  60

His Gly Ala Thr Val Leu Thr Ala Leu Gly Gly Ile Leu Lys Lys Lys
65                  70                  75                  80

Gly His His Glu Ala Glu Ile Lys Pro Leu Ala Gln Ser His Ala Thr
                85                  90                  95

Lys His Lys Ile Pro Val Lys Tyr Leu Glu Phe Ile Ser Glu Cys Ile
            100                 105                 110

Ile Gln Val Leu Gln Ser Lys His Pro Gly Asp Phe Gly Ala Asp Ala
        115                 120                 125

Gln Gly Ala Met Asn Lys Ala Leu Glu Leu Phe Arg Lys Asp Met Ala
    130                 135                 140

Ser Asn Tyr Lys Glu Leu Gly Phe Gln Gly
145                 150

<210> 36
<211> 465
<212> DNA
<213> Homo sapiens

<220>
<223> Nucleic acid coding sequence for Homo sapiens myoglobin

<400> 36
atggggctca gcgacgggga atggcagttg gtgctgaacg tctgggggaa ggtggaggct 60

gacatcccag gccatgggca ggaagtcctc atcaggctct ttaagggtca cccagagact 120

ctggagaagt ttgacaagtt caagcacctg aagtcagagg acgagatgaa ggcgtctgag 180

gacttaaaga agcatggtgc caccgtgctc accgccctgg gtggcatcct taagaagaag 240

gggcatcatg aggcagagat taagcccctg gcacagtcgc atgccaccaa gcacaagatc 300

cccgtgaagt acctggagtt catctcggaa tgcatcatcc aggttctgca gagcaagcat 360

cccggggact ttggtgctga tgcccagggg gccatgaaca aggccctgga gctgttccgg 420

aaggacatgg cctccaacta caaggagctg ggcttccagg gctag 465

<210>  37
<211>  258
<212>  PRT
<213>  Ralstonia eutropha

<220>
<223>  Amino acid sequence of FHPg-FAD: FHPg-FAD is lacking the NADPH
       domain of FHP (Ralstonia eutropha flavohemoglobin)

<400>  37

Met Leu Thr Gln Lys Thr Lys Asp Ile Val Lys Ala Thr Ala Pro Val
1               5                   10                  15

Leu Ala Glu His Gly Tyr Asp Ile Ile Lys Cys Phe Tyr Gln Arg Met
            20                  25                  30

Phe Glu Ala His Pro Glu Leu Lys Asn Val Phe Asn Met Ala His Gln
        35                  40                  45

Glu Gln Gly Gln Gln Gln Gln Ala Leu Ala Arg Ala Val Tyr Ala Tyr
    50                  55                  60

Ala Glu Asn Ile Glu Asp Pro Asn Ser Leu Met Ala Val Leu Lys Asn
65                  70                  75                  80

Ile Ala Asn Lys His Ala Ser Leu Gly Val Lys Pro Glu Gln Tyr Pro
                85                  90                  95

Ile Val Gly Glu His Leu Leu Ala Ala Ile Lys Glu Val Leu Gly Asn
            100                 105                 110

Ala Ala Thr Asp Asp Ile Ile Ser Ala Trp Ala Gln Ala Tyr Gly Asn
        115                 120                 125

Leu Ala Asp Val Leu Met Gly Met Glu Ser Glu Leu Tyr Glu Arg Ser
    130                 135                 140

Ala Glu Gln Pro Gly Gly Trp Lys Gly Trp Arg Thr Phe Val Ile Arg
145                 150                 155                 160

Glu Lys Arg Pro Glu Ser Asp Val Ile Thr Ser Phe Ile Leu Glu Pro
                165                 170                 175

Ala Asp Gly Gly Pro Val Val Asn Phe Glu Pro Gly Gln Tyr Thr Ser
            180                 185                 190

Val Ala Ile Asp Val Pro Ala Leu Gly Leu Gln Gln Ile Arg Gln Tyr

195                 200                 205

Ser Leu Ser Asp Met Pro Asn Gly Arg Thr Tyr Arg Ile Ser Val Lys
    210                 215                 220

Arg Glu Gly Gly Gly Pro Gln Pro Pro Gly Tyr Val Ser Asn Leu Leu
225                 230                 235                 240

His Asp His Val Asn Val Gly Asp Gln Val Lys Leu Ala Ala Pro Tyr
                245                 250                 255

Gly Ser

<210>  38
<211>  777
<212>  DNA
<213>  Ralstonia eutropha

<220>
<223>  Nucleic acid coding sequence for FHPg-FAD: FHPg-FAD is lacking
       the NADPH domain of FHP (Ralstonia eutropha flavohemoglobin)

<400>  38
atgctgaccc agaaaacaaa agacatcgta aaggccaccg caccggtgct ggccgaacac      60

ggctacgaca tcatcaagtg cttctatcag cgcatgttcg aagcgcatcc cgaactgaag     120

aatgtgttca acatggccca ccaggaacag ggccagcagc agcaggcgct ggcacgggcg     180

gtctatgcct atgcggagaa tatcgaggac ccaaacagtc tgatggccgt cctgaagaac     240

atcgccaaca agcatgcaag tctcggcgtc aaaccggaac agtaccccat tgttggagaa     300

cacctactgg ccgccatcaa ggaagtgttg ggcaatgccg caaccgacga catcatttcc     360

gcttgggcgc aagcctacgg gaatctggcc gatgtcctga tgggcatgga gagcgagttg     420

tatgagcgct ctgcggaaca acccggtggc tggaaggggt ggcgcacctt tgtgattcgt     480

gagaagcgcc ccgagagcga tgtgatcacg tcattcatcc ttgagcctgc cgatggtggt     540

cccgtggtga acttcgaacc cggacaatac accagcgtcg caatcgatgt gcccgcgctc     600

gggttgcagc agattcgcca atacagcctg tcggatatgc cgaacgggcg cacgtatcgc     660

atctcggtga agcgcgaggg cggcgggccg cagcctccgg gctatgtttc gaacctgttg     720

catgatcacg tgaatgttgg ggatcaggtc aagctggctg cgccctatgg aagctaa        777

<210>  39
<211>  402
<212>  PRT
<213>  Artificial

<220>

<223>  Amino acid sequence of VHb-Red: a fusion of VHb (Vitreoscilla sp.
      hemoglobin) with the reductase domain of FHP (Ralstonia eutropha
      flavohemoglobin)

<400>  39

Met Leu Asp Gln Gln Thr Ile Asn Ile Ile Lys Ala Thr Val Pro Val
1               5                   10                  15

Leu Lys Glu His Gly Val Thr Ile Thr Thr Thr Phe Tyr Lys Asn Leu
            20                  25                  30

Phe Ala Lys His Pro Glu Val Arg Pro Leu Phe Asp Met Gly Arg Gln
        35                  40                  45

Glu Ser Leu Glu Gln Pro Lys Ala Leu Ala Met Thr Val Leu Ala Ala
    50                  55                  60

Ala Gln Asn Ile Glu Asn Leu Pro Ala Ile Leu Pro Ala Val Lys Lys
65                  70                  75                  80

Ile Ala Val Lys His Cys Gln Ala Gly Val Ala Ala Ala His Tyr Pro
                85                  90                  95

Ile Val Gly Gln Glu Leu Leu Gly Ala Ile Lys Glu Val Leu Gly Asp
            100                 105                 110

Ala Ala Thr Asp Asp Ile Leu Asp Ala Trp Gly Lys Ala Tyr Gly Val
        115                 120                 125

Ile Ala Asp Val Phe Ile Gln Val Glu Ala Asp Leu Tyr Ala Gln Ala
    130                 135                 140

Val Glu Pro Gly Thr Trp Lys Gly Trp Arg Thr Phe Val Ile Arg Glu
145                 150                 155                 160

Lys Arg Pro Glu Ser Asp Val Ile Thr Ser Phe Ile Leu Glu Pro Ala
                165                 170                 175

Asp Gly Gly Pro Val Val Asn Phe Glu Pro Gly Gln Tyr Thr Ser Val
            180                 185                 190

Ala Ile Asp Val Pro Ala Leu Gly Leu Gln Gln Ile Arg Gln Tyr Ser
        195                 200                 205

Leu Ser Asp Met Pro Asn Gly Arg Thr Tyr Arg Ile Ser Val Lys Arg
    210                 215                 220

Glu Gly Gly Gly Pro Gln Pro Pro Gly Tyr Val Ser Asn Leu Leu His

225                 230                 235                 240

Asp His Val Asn Val Gly Asp Gln Val Lys Leu Ala Ala Pro Tyr Gly
                245                 250                 255

Ser Phe His Ile Asp Val Asp Ala Lys Thr Pro Ile Val Leu Ile Ser
            260                 265                 270

Gly Gly Val Gly Leu Thr Pro Met Val Ser Met Leu Lys Val Ala Leu
        275                 280                 285

Gln Ala Pro Pro Arg Gln Val Val Phe Val His Gly Ala Arg Asn Ser
    290                 295                 300

Ala Val His Ala Met Arg Asp Arg Leu Arg Glu Ala Ala Lys Thr Tyr
305                 310                 315                 320

Glu Asn Leu Asp Leu Phe Val Phe Tyr Asp Gln Pro Leu Pro Glu Asp
                325                 330                 335

Val Gln Gly Arg Asp Tyr Asp Tyr Pro Gly Leu Val Asp Val Lys Gln
            340                 345                 350

Ile Glu Lys Ser Ile Leu Leu Pro Asp Ala Asp Tyr Tyr Ile Cys Gly
        355                 360                 365

Pro Ile Pro Phe Met Arg Met Gln His Asp Ala Leu Lys Asn Leu Gly
    370                 375                 380

Ile His Glu Ala Arg Ile His Tyr Glu Val Phe Gly Pro Asp Leu Phe
385                 390                 395                 400

Ala Glu

<210>  40
<211>  1209
<212>  DNA
<213>  Artificial

<220>
<223>  Nucleic acid coding sequence for VHb-Red: a fusion of VHb
       (Vitreoscilla sp. hemoglobin)  with the reductase domain of FHP
       (Ralstonia eutropha flavohemoglobin)

<400>  40
atgttagacc agcaaaccat taacatcatc aaagccactg ttcctgtatt gaaggagcat      60

ggcgttacca ttaccacgac tttttataaa aacttgtttg ccaaacaccc tgaagtacgt     120

cctttgtttg atatgggtcg ccaagaatct ttggagcagc ctaaggcttt ggcgatgacg     180

gtattggcgg cagcgcaaaa cattgaaaat ttgccagcta ttttgcctgc ggtcaaaaaa 240

attgcagtca aacattgtca agcaggcgtg gcagcagcgc attatccgat tgtcggtcaa 300

gaattgttgg gtgcgattaa agaagtattg ggcgatgccg caaccgatga cattttggac 360

gcgtggggca aggcttatgg cgtgattgca gatgtgttta ttcaagtgga agcagatttg 420

tacgctcaag cggttgaacc cggtacctgg aaggggtggc gcacctttgt gattcgtgag 480

aagcgccccg agagcgatgt gatcacgtca ttcatccttg agcctgccga tggtggtccc 540

gtggtgaact tcgaacccgg acaatacacc agcgtcgcaa tcgatgtgcc cgcgctcggg 600

ttgcagcaga ttcgccaata cagcctgtcg gatatgccga acgggcgcac gtatcgcatc 660

tcggtgaagc gcgagggcgg cgggccgcag cctccgggct atgtttcgaa cctgttgcat 720

gatcacgtga atgttgggga tcaggtcaag ctggctgcgc cctatggaag cttccatatc 780

gacgttgacg ccaagacacc cattgtcctg atcagcggtg gcgttgggct gacaccgatg 840

gtcagcatgc tcaaagtggc attgcaggcc ccaccgaggc aagttgtatt cgtgcatggc 900

gcacgtaaca gcgcagtaca cgcgatgcgc gacaggctgc gcgaagcggc gaagacctat 960

gaaaaccttg acctctttgt tttctatgac caaccgctac cggaagatgt ccagggacgg 1020

gactatgatt atccgggctt agtcgatgtg aagcagatcg agaaatcgat cctgctgcca 1080

gacgccgact actacatctg cggtccgatt ccgttcatgc gaatgcagca tgatgccctg 1140

aagaatctcg gcattcatga ggcacgcatt cattacgaag tgtttggtcc cgacctgttt 1200

gctgagtag 1209

<210> 41
<211> 257
<212> PRT
<213> Artificial

<220>
<223> Amino acid sequence of VHB-FAD: a fusion of VHb (Vitreoscilla sp.
hemoglobin) with the FAD-binding domain of FHP (Ralstonia
eutropha flavohemoglobin)

<400> 41

Met Leu Asp Gln Gln Thr Ile Asn Ile Ile Lys Ala Thr Val Pro Val
1               5                   10                  15

Leu Lys Glu His Gly Val Thr Ile Thr Thr Thr Phe Tyr Lys Asn Leu
            20                  25                  30

Phe Ala Lys His Pro Glu Val Arg Pro Leu Phe Asp Met Gly Arg Gln
        35                  40                  45

Glu Ser Leu Glu Gln Pro Lys Ala Leu Ala Met Thr Val Leu Ala Ala

50                  55                  60

Ala Gln Asn Ile Glu Asn Leu Pro Ala Ile Leu Pro Ala Val Lys Lys
65                  70                  75                  80

Ile Ala Val Lys His Cys Gln Ala Gly Val Ala Ala Ala His Tyr Pro
                85                  90                  95

Ile Val Gly Gln Glu Leu Leu Gly Ala Ile Lys Glu Val Leu Gly Asp
            100                 105                 110

Ala Ala Thr Asp Asp Ile Leu Asp Ala Trp Gly Lys Ala Tyr Gly Val
        115                 120                 125

Ile Ala Asp Val Phe Ile Gln Val Glu Ala Asp Leu Tyr Ala Gln Ala
    130                 135                 140

Val Glu Pro Gly Thr Trp Lys Gly Trp Arg Thr Phe Val Ile Arg Glu
145                 150                 155                 160

Lys Arg Pro Glu Ser Asp Val Ile Thr Ser Phe Ile Leu Glu Pro Ala
                165                 170                 175

Asp Gly Gly Pro Val Val Asn Phe Glu Pro Gly Gln Tyr Thr Ser Val
            180                 185                 190

Ala Ile Asp Val Pro Ala Leu Gly Leu Gln Gln Ile Arg Gln Tyr Ser
        195                 200                 205

Leu Ser Asp Met Pro Asn Gly Arg Thr Tyr Arg Ile Ser Val Lys Arg
    210                 215                 220

Glu Gly Gly Gly Pro Gln Pro Pro Gly Tyr Val Ser Asn Leu Leu His
225                 230                 235                 240

Asp His Val Asn Val Gly Asp Gln Val Lys Leu Ala Ala Pro Tyr Gly
                245                 250                 255

Ser

<210>  42
<211>  774
<212>  DNA
<213>  Artificial

<220>
<223>  Nucleic acid coding sequence for VHB-FAD: a fusion of VHb
       (Vitreoscilla sp. hemoglobin) with the FAD-binding domain of FHP

(Ralstonia eutropha flavohemoglobin)

<400> 42
atgttagacc agcaaaccat taacatcatc aaagccactg ttcctgtatt gaaggagcat 60

ggcgttacca ttaccacgac tttttataaa aacttgtttg ccaaacaccc tgaagtacgt 120

cctttgtttg atatgggtcg ccaagaatct ttggagcagc ctaaggcttt ggcgatgacg 180

gtattggcgg cagcgcaaaa cattgaaaat ttgccagcta ttttgcctgc ggtcaaaaaa 240

attgcagtca aacattgtca agcaggcgtg gcagcagcgc attatccgat tgtcggtcaa 300

gaattgttgg gtgcgattaa agaagtattg ggcgatgccg caaccgatga cattttggac 360

gcgtggggca aggcttatgg cgtgattgca gatgtgttta ttcaagtgga agcagatttg 420

tacgctcaag cggttgaacc cggtacctgg aaggggtggc gcacctttgt gattcgtgag 480

aagcgccccg agagcgatgt gatcacgtca ttcatccttg agcctgccga tggtggtccc 540

gtggtgaact tcgaacccgg acaatacacc agcgtcgcaa tcgatgtgcc cgcgctcggg 600

ttgcagcaga ttcgccaata cagcctgtcg gatatgccga acgggcgcac gtatcgcatc 660

tcggtgaagc gcgagggcgg cgggccgcag cctccgggct atgtttcgaa cctgttgcat 720

gatcacgtga atgttgggga tcaggtcaag ctggctgcgc cctatggaag ctaa 774

<210> 43
<211> 147
<212> PRT
<213> Ralstonia eutropha

<220>
<223> Amino acid sequence of FHPg: a truncated version of FHP
(Ralstonia eutropha flavohemoglobin) lacking the complete
reductase domain

<400> 43

Met Leu Thr Gln Lys Thr Lys Asp Ile Val Lys Ala Thr Ala Pro Val
1               5                   10                  15

Leu Ala Glu His Gly Tyr Asp Ile Ile Lys Cys Phe Tyr Gln Arg Met
            20                  25                  30

Phe Glu Ala His Pro Glu Leu Lys Asn Val Phe Asn Met Ala His Gln
        35                  40                  45

Glu Gln Gly Gln Gln Gln Gln Ala Leu Ala Arg Ala Val Tyr Ala Tyr
    50                  55                  60

Ala Glu Asn Ile Glu Asp Pro Asn Ser Leu Met Ala Val Leu Lys Asn
65                  70                  75                  80

Ile Ala Asn Lys His Ala Ser Leu Gly Val Lys Pro Glu Gln Tyr Pro
                85                  90                  95

Ile Val Gly Glu His Leu Leu Ala Ala Ile Lys Glu Val Leu Gly Asn
            100                 105                 110

Ala Ala Thr Asp Asp Ile Ile Ser Ala Trp Ala Gln Ala Tyr Gly Asn
        115                 120                 125

Leu Ala Asp Val Leu Met Gly Met Glu Ser Glu Leu Tyr Glu Arg Ser
    130                 135                 140

Ala Glu Gln
145

<210>  44
<211>  444
<212>  DNA
<213>  Ralstonia eutropha

<220>
<223>  Nucleic acid coding sequence for FHPg: a truncated version of FHP
       (Ralstonia eutropha flavohemoglobin) lacking the complete
       reductase domain

<400>  44
atgctgaccc agaaaacaaa agacatcgta aaggccaccg caccggtgct ggccgaacac      60

ggctacgaca tcatcaagtg cttctatcag cgcatgttcg aagcgcatcc cgaactgaag     120

aatgtgttca acatggccca ccaggaacag ggccagcagc agcaggcgct ggcacgggcg     180

gtctatgcct atgcggagaa tatcgaggac ccaaacagtc tgatggccgt cctgaagaac     240

atcgccaaca agcatgcaag tctcggcgtc aaaccggaac agtaccccat tgttggagaa     300

cacctactgg ccgccatcaa ggaagtgttg ggcaatgccg caaccgacga catcatttcc     360

gcttgggcgc aagcctacgg gaatctggcc gatgtcctga tgggcatgga gagcgagttg     420

tatgagcgct ctgcggaaga ataa                                            444

## Description

Despite numerous defenses all organisms are vulnerable to oxidative and nitrosative stress which may ultimately lead to cell death. Reactive oxygen species (ROS) such as superoxide and reactive nitrogen species (NOS) like nitric oxide radical NO may be formed as undesired and noxious metabolic side products or alternatively, such stress may arise from exogenous sources. ROS, for example, are not only produced during respiration when molecular oxygen is reduced to water but also as a consequence of initiation of events during hypoxia, ischemia and inflammatory responses. RNS in turn such as NO, are molecules mediating nitrosative stress. In the case of NO, its formation is both constitutive and inductive, for example when cells are exposed to a hypoxic state or in response to autoimmune or inflammatory processes.

One area of therapy where oxidative and nitrosative stress often severely affects the life of patients is ischemia in particular in the context of neural and brain damages caused by stroke. The brain has potent defenses against superoxide including dietary free-radical scavengers, the endogenous tripeptide glutathione, and enzymatic antioxidants. Enzymatic antioxidants regulate superoxide concentration by dismutation of superoxide to hydrogen peroxide (superoxide dismutase or SOD), which is then converted to water (peroxidases such as glutathione peroxidase and peroxiredoxin) or dismuted to water and oxygen (catalase). Although increased expression of these enzymes can occur in response to ischemia, endogenous antioxidant capacity can be overwhelmed, leading to increased superoxide and hydrogen peroxide concentrations. Superoxide can cause oxidative damage of iron/sulfur clusters of aconitase, an important enzyme in the tricarboxylic acid cycle. The major oxidative stress produced by superoxide, however, is derived from its participation in peroxynitrite formation and its involvement in the iron-catalyzed Haber-W eiss reaction (superoxide-driven Fenton chemistry), causing hydrogen peroxide to be converted to hydroxyl radical. Hydroxyl radical, peroxynitrite and peroxynitrite-derived products (hydroxyl radical, carbonate radical and nitrogen dioxide) all have the potential to react with and damage most cellular targets including lipids, proteins and DNA.

Ischemia-induced nitric oxide overproduction in the brain is in part caused by glutamatergic-mediated increases in intracellular calcium concentration, resulting in a calmodulin-dependent upregulation of nitric oxide synthase (NOS). Under pathophysiological conditions, excessive nitric oxide production can elicit nitrosative damage via independent nitrosylation of protein heme sites (e.g. cytochrome c) or through its reaction products with oxygen or other nitrogen oxides.

Other areas, where oxidative and nitrosative stress is considered to be involved or underlie the disease are indications including neurodegenerative disorders, disorders of gastrointestinal motility and inflammation. These diseases and disorders include neurodegenerative disorders, disorders of gastrointestinal motility, hypotension, septic shock, toxic shock syndrome, hemodialysis, cancer, cachexia, diseases requiring immunosuppression, diseases requiring transplant therapy, autoimmune diseases, inflammatory diseases, including sunburn, eczema or psoriasis and, a respiratory conditions such as bronchitis, asthma, oxidant-induced lung injury and acute respiratory distress (ARDS), glomerulonephritis, viral infections, myocarditis, heart failure, atherosclerosis, arthritis, rheumatoid arthritis, chronic bowel disease, inflammatory bowel disease, ulcerative colitis, Crohn's disease, systemic lupus erythematosis (SLE), ocular conditions such as ocular hypertension, retinitis and uveitis, insulin-dependent type 1 diabetes mellitus, diabetes type 2, cystic fibrosis, hypoxia, hyperbaric oxygen convulsions, a toxicity indication, dementia, Sydenham's chorea, Parkinson's disease, Huntington's disease, amyotrophic lateral sclerosis, multiple sclerosis, Korsakoff's disease, imbecility related to cerebral vessel disorder, NO mediated cerebral trauma, ischemic brain edema, stroke, sleeping disorders, schizophrenia, depression, pre-menstrual syndrome (PMS), anxiety, drug addiction, pain, migraine, immune complex disease, allograft rejection, infections caused by invasive microorganisms which produce NO, and radio contrast induced renal failure.

In these diseases an improved treatment is needed. Success of current treatment is often limited because the body's immune system attacks cells of the body and/or transplanted of cells, tissue or organs. Thus there is a need for protecting cells, tissues and organs against attacks of the immune system.

Immunosuppressive treatment of patients, for example, with cyclosporin A or FK506, also has been effected, but with only limited success. Immunosuppressive drugs have toxic side effects, including the potential for infection as the result of suppression of the immune system. It would therefore be advantageous if a treatment could be provided which compared to current treatments only requires administration of smaller doses of immunosuppressive agents or which even obviates the administration of these agents in toto.

Worldwide 15 million people are diagnosed with type 1 diabetes, also designated as insulin-dependent diabetes mellitus (NDDM). Type 1 diabetes is characterized, inter alia, by a loss of insulin-producing beta-cells and decompensation of metabolism following an autoimmune and inflammatory process (reviewed in Mathis et al., 2001). Treatments of such patients have included primarily parenteral administration of bovine or porcine insulin or recombinant human insulin. This treatment, however, delays but does not avoid the pathological progression of this disease and, in addition, requires multiple daily injections of insulin and/or the use of an indwelling catheter and an insulin pump.

Currently practiced therapies also include whole pancreas transplantation. In this case, a clear discrepancy between the number of available organs and the number of type 1 diabetes patients exists which leads to a strong shortage of transplantable organs. In addition, adult human pancreatic islets have been transplanted into patients in order to achieve independence from insulin injections. Despite these advances, the limited number of organ donors, the inadequate islet masses obtainable from mature pancreases, and graft rejection problems have limited the general usefulness of this approach.

An alternate source of pancreatic islets for transplantation is the fetal pancreas. This tissue is rich in undifferentiated beta-cells that can, at least in theory, grow and mature after transplantation. While the immature immune system of the fetus reduces the likelihood of fetal islet rejection by the recipient, problems relating to the limited availability of suitable fetal pancreases and to the immaturity of the insulin-producing cells in such tissues continue to hinder success in this approach (for a review, see Andersson, 1992) creating a need for a therapy that is not restricted due to a dependency on limited resources.

Transplantation of beta-islet cells according to current techniques results in a 25% survival rate of the transplanted cells or tissue only; 50% of the transplantable cells die prior to transplantation and another 25% die due to the recipient's immune response. As a result, there also exists a need for therapies that increase the survival rate of insulin-producing cells, tissues and organs used for transplantation.

In analogy to apoptosis processes taking place in the cells, tissues and organs suffering from one of the diseases mentioned above, in the case of diabetes type 1 disease beta-islet cell apoptosis is induced by a variety of immune mediators central to the pathogenesis of autoimmune type 1 diabetes and the failure of islet cell transplantation. Loss of normal beta islet cell function has been implicated in type 2 diabetes as well. Nitric oxide is induced by inflammatory cytokines, interleukine 1-beta (IL-1 beta), tumor necrosis factor alpha (TNF-alpha) and interferon-gamma (IFN-gamma) is a possible mediator of beta-cell destruction. Besides the generation of NO, cytokines such as TNF-alpha, IFN-gamma and IL-1 beta also induce the generation of oxygen radicals. Cytokines stimulate cells resident- or intraislet macrophage-resident inducible nitric oxide synthase (iNOS) expression with subsequent production of NO. Notably, the distinct stimuli that cause apoptosis converge on a common cell death machinery, which is driven and controlled by caspases. The oxidative and nitrosative stress-dependent autoimmune response poses a severe problem to implantation and transplantation of cells, tissues and organs and in particular to type 1 diabetes patients receiving implants or transplants of pancreatic beta-cells, pancreatic islets, or islet grafts up to entire pancreas organs. Beta-cells are particularly known to suffer from hypoxia encountered after transplantation of islets before the revascularization of the transplanted tissue.

NO is synthesized by the enzyme NO synthase (NOS) through the five-electron oxidation of one of the chemically equivalent guanidine nitrogens associated with L-arginine. Of the three NOS isoforms, two are constitutively expressed, endothelial NOS (eNOS, NOS3) and neuronal NOS (nNOS, NOS1), and one, inducible NOS (iNOS, NOS2), is regulated at the gene level by a variety of inflammatory mediators. NO production by iNOS is 1,000-fold greater than by any constitutively active NOS isoform. Typically, it is the overproduction of NO by iNOS that contributes most of the excessive NO that leads to disease pathology. In rodents, inhibition of NOS using *N*^{G}-monomethyl-L-arginine (L-NMMA), which belongs to a class of arginine analogues that competitively inhibit all three NOS isoforms, significantly reduced NO production. One drawback to using L-NMMA as a systemic inhibitor of the NO pathway is its apparent inability to discriminate between the different isoforms of NOS. Interference with neuronal and endothelial constitutive NOS (cNOS) could affect cell-cell communication, vascular tone, and neurotransmission. iNOS, which produces the excessive NO in inflammatory diseases, is now known to be constitutively produced in the gut and necessary for intestinal homeostasis. Thus, even highly selective iNOS inhibitors are believed to have undesirable side effects which could complicate any potential iNOS inhibitor therapy. Notably, iNOS inhibitors are cell-type or tissue unspecific and therefore a systemic effect is observed upon administration.

Various gene transfer based therapeutic approaches have been evaluated to protect islet cells against reactive oxygen and nitrogen species (Bottino et al., 2003). Most of the attempts to prevent beta-cell apoptosis were based on the overexpression of enzymatic activities which catalyze metabolization of oxygen species, such as superoxide dismutase or catalase (Bertera et al., 2003; Hohmeier et al., 1998; Lortz et al., 2000; Tiedge et al., 1999). Alternatively, methods designed to specifically target downstream effector molecules such as antiapoptotic genes have been tested (Contreras et al., 2001; Rabinovitch et al., 1999). Unfortunately, none of these approaches provided sufficient protection from attacks by the hosts' immune system. Since NO has been determined as one of the key molecules in the onset of beta-cell apoptosis, specific treatments are necessary to either prevent formation of NO or to abrogate its harmful action within the cells.

Thus, a need exists for therapies that increase the survival rate of cells, tissues and organs used for transplantation, particularly in the areas of immune diseases, autoimmune diseases and especially in diabetes treatment. There is also a need for cells and medicaments for treatment of the mentioned diseases that is not limited by scarce resources and that is readily available. It would also be desirable to provide protection of the body's own cells against attack by the immune system. Finally, it would be desirable to provide a tissue- and cell-specific treatment.

Prokaryotic and eukaryotic cells contain enzymes which consume nitric oxide in catalytic reactions such as hemoglobin. In bacteria an NO degrading function termed nitric oxide dioxygenase (NOD) activity and a cytoprotective role of bacteria globin proteins has been established (Frey et al., 2002). Bacterial globin proteins can be divided into three distinct groups among which flavohemoglobins and hemoglobins are the best-characterized entities (Frey and Kallio, 2003). Both contain a hemoglobin domain which shares a high degree of structural homology to myoglobin and hemoglobin of mammalian origin (Lecomte et al., 2005). Previously it was demonstrated that bacterial globin proteins protect bacteria against nitrosative stress and degrade NO *in vitro* (Frey et al., 2002). *Vitreoscilla sp.* expresses a bacterial hemoglobin referred to as VHb (Koshla and Bailey, 1988) which is a homodimeric hemoglobin. Upon expression VHb conferred resistance to the NO-releasing agent sodium nitroprusside (SNP)-challenged *E. coli.*

In contrast to bacterial hemoglobins, flavohemoglobins additionally carry a carboxy-terminal FAD- and NAD(P)H-binding reductase domain, which enhances the catalytic function of this molecule. Bacterial globins, foremost flavohemoglobins, have been implicated in the degradation of nitric oxide via an oxygen dependent and oxygen independent reaction in the bacterial metabolism (Frey and Kallio, 2003). The flavohemoglobin expressed by *Ralstonia eutropha,* formerly known as *Alcaligenes eutrophus,* is designated FHP. *Escherichia coli* expressing FHP demonstrate significantly increased cell growth and productivity under microaerobic conditions.

In mammals a member of the globin family known as neuroglobin (ngb) has been identified which is a hexacoordinate globin protein of unknown function, and which is expressed in neurons and pancreatic islets. Ngb is induced under hypoxic conditions in neuronal and some endocrine tissues, where it showed a protective function (Hankeln et al., 2005; Sun et al., 2001). Ngb-transduced islets displayed an improved mitochondrial membrane potential after prolonged in vitro culture (Mendoza et al., 2005). However, transduced proteins are prone to proteolysis and therefore their therapeutic effects are limited to a short period of time.

A technical problem underlying the present invention is to provide new cells, tissues and organs which are protected against oxidative and/or nitrosative stress and to provide methods of producing such cells. It is another problem to provide further means for treatment of the mentioned diseases.

A further problem is to provide a new medicament for the treatment of patients in need of protection against oxidative and/or nitrosative stress, especially in patients suffering from diabetes, particularly from diabetes type I.

Moreover, a further problem is the provision of methods of protecting cells from oxidative and/or nitrosative stress which methods avoid the disadvantages known in the art.

At least one of the above problems is solved by the provision of the subject-matter characterized in the claims.

According to one aspect of the invention a mammalian host cell is provided containing a nucleic acid comprising a nucleic acid selected from
a1) a nucleic acid coding for a bacterial hemoglobin or a bacterial flavohemoglobin having oxidative and/or nitrosative stress protective activity, or a complementary strand thereof;
a2) a nucleic acid coding for a polypeptide selected from the group consisting of an amino acid sequence according to SEQ ID NO: 1, 3, 11, 13, 15, 17, 19, 21, 23, 25, 27, 29, 31, 33, 35, 37, 39, 41, and 43; or a complementary strand thereof, and which nucleic acid encodes a polypeptide having oxidative and/or nitrosative stress protective activity;
a3) a nucleic acid coding for a polypeptide having at least 60% sequence identity to one of the polypeptide sequences defined in a1) and a2) and which nucleic acid encodes a polypeptide having oxidative and/or nitrosative stress protective activity;
a4) a nucleic acid selected from the group consisting of a nucleic acid sequence according to SEQ ID NO: 2, 4, 12, 14, 16, 18, 20, 22, 24, 26, 28, 30, 32, 34, 36, 38, 40, 42, and 44;
a5) a nucleic acid which on the basis of the genetic code is degenerate to one of the nucleic acid sequences defined in a1) to a4) and which nucleic acid or complementary strand thereof encodes a polypeptide having oxidative and/or nitrosative stress protective activity;
a6) a nucleic acid which hybridizes with one of the nucleic acids defined in a1) to a5) and which nucleic acid or complementary strand thereof encodes a polypeptide having oxidative and/or nitrosative stress protective activity;
a7) a nucleic acid having at least 60% sequence identity to one of the nucleic acid sequences defined in a1) to a6) and which nucleic acid or complementary strand thereof encodes a polypeptide having oxidative and/or nitrosative stress protective activity;
a8) a variant of one of the nucleic acids defined in a1) to a7), wherein compared to one of the nucleic acids defined in a1) to a7) the variant contains at least one addition, deletion, insertion and/or inversion and wherein the variant or the complementary strand thereof encodes a polypeptide having oxidative and/or nitrosative stress protective activity; and
a9) a fragment of one of the nucleic acids defined in a1) to a8), wherein the fragment or the complementary strand thereof encodes a polypeptide having oxidative and/or nitrosative stress protective activity.

It was surprisingly found that heterologous expression of bacterial hemoglobins and/or flavohemoglobins in the host cells according to the invention actually conveys an effective protection of host cells against nitrosative and/or oxidative stressors, as has been shown in the accompanying examples below. Such host cells according to the invention are well protected against attacks of the immune system and represent an effective cellular system for transplantation into host organisms in need of such treatment. The host cells are easy to produce and readily available as it is evident from the examples described below.

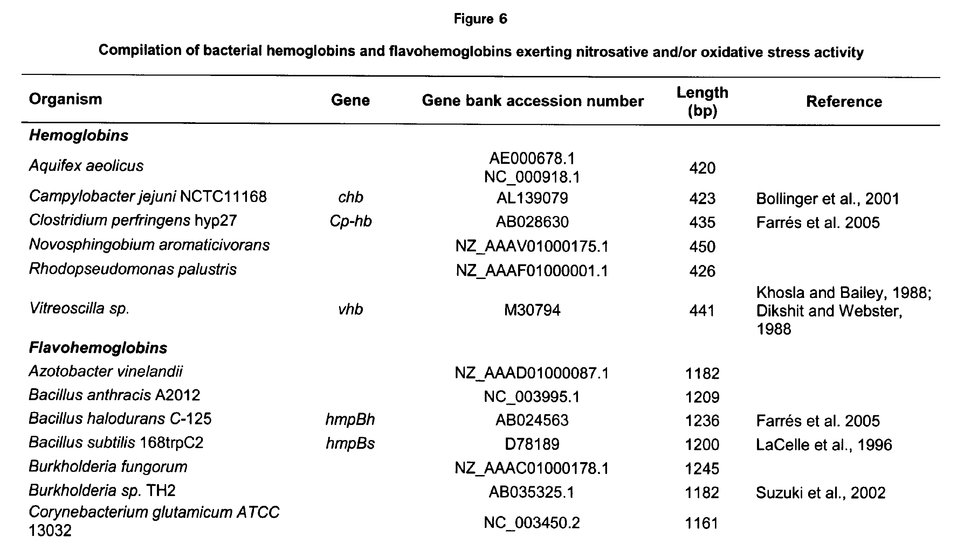

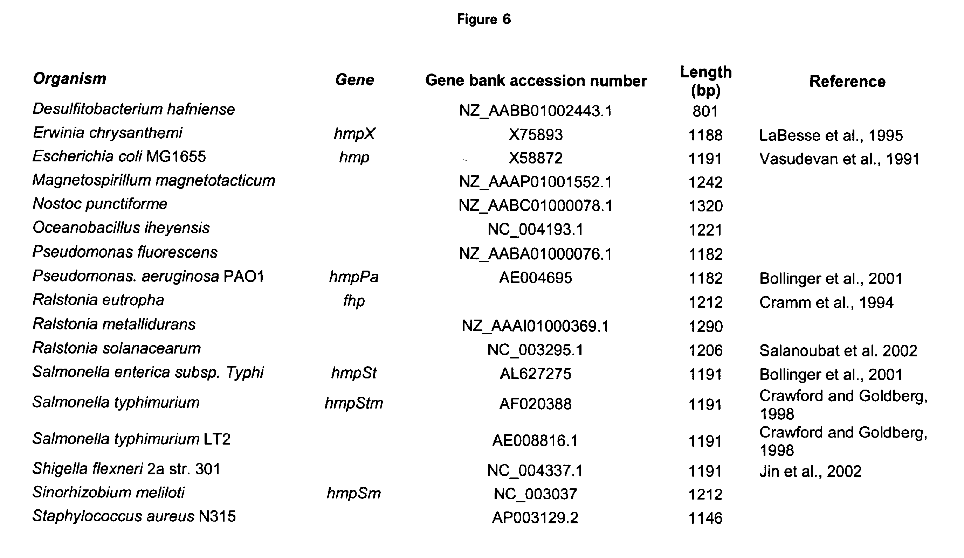

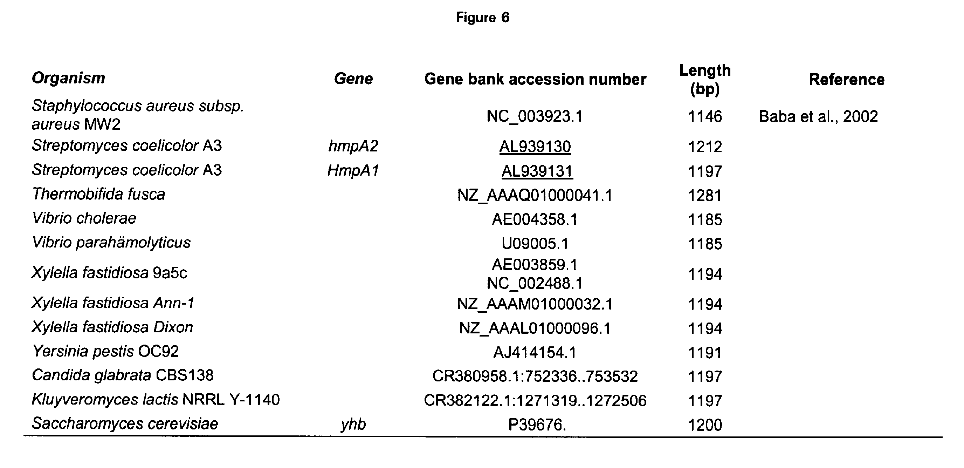

Within the meaning of the invention the term "nucleic acid" is intended to comprise single and double stranded DNA, genomic DNA, cDNA and synthetic DNA, where synthetic DNA is also understood to encompass DNA containing modified internucleoside bonds. The term "nucleic acid" also extends to RNA, in particular to tRNA, mRNA and also to artificial RNA. The nucleic acid may for example be obtained by using a suitable probe specific for a nucleic acid defined in a), preferably a nucleic acid as defined in a1) to a9), or a part thereof, for screening of genomic or cDNA libraries derived from an organism, preferably a bacterial organism especially bacteria from the genus *Ralstonia, Vitreoscilla, Escherichia, Pseudomonas, Streptococcus, Deinococcus, Klebsiella, Saccharomycetes, Salmonella, Campylobacter, Mycobacterium;* even more preferred from the species *Vitreoscilla sp.* or *Ralstonia eutropha,* more preferably a bacteria from the genus or species listed in Table 1 and Figure 6. The selection and synthesis of probes and identification of positive clones is carried out according to standard procedures generally known such as those described in Sambrook and Russell, 2001. Preferably the nucleic acid as defined in a) above is an isolated nucleic acid, i.e. a nucleic acid that has been synthesized, or alternatively a nucleic acid identified, separated and/or recovered from a component of its natural environment.

Preferably, the nucleic acid sequences coding for bacterial hemoglobin and flavohemoglobin, respectively, are derived from the genus *Ralstonia, Vitreoscilla, Escherichia, Pseudomonas, Streptococcus, Deinococcus, Klebsiella, Saccharomycetes, Salmonella, Campylobacter, Mycobacterium;* even more preferred from *Vitreoscilla sp.* or *Ralstonia eutropha,* even more preferably the nucleic acid of the invention is a bacterial hemoglobin or flavohemoglobin listed in Table 1 and Figure 6, most preferably a nucleic acid coding for a bacterial hemoglobin or flavohemoglobin listed in Table 1 and Figure 6.

In a preferred embodiment the hybridization is carried out under stringent hybridization conditions which are generally known in the art. Stringent hybridization conditions are for example incubation at 65 °C overnight in 7% SDS, 1% BSA, 1 mM EDTA, 250 mM sodium phosphate buffer (pH 7.2) and subsequent washing at 65 °C with 2xSSC; 0.1 % SDS.

The phrase "sequence identity" as used herein refers to the percentage of (%) nucleic acid sequence identity of a candidate nucleic acid relative to a reference nucleic acid sequence, i.e. the percentage of nucleotide residues in a candidate sequence that are identical with those of the reference sequence, after aligning the sequences and introducing gaps, if necessary, to achieve the maximum percent sequence identity, and not considering any conservative substitutions as part of the sequence identity. The % identity values used herein may for example be generated by WU-BLAST-2 which was calculated based on Altschul et al., 1996. WU-BLAST-2 uses several search parameters, most of which are set to the default values. The adjustable parameters are set with the following values: overlap span = 1, overlap fraction = 0.125, word threshold (T) = 11. A % nucleic acid sequence identity value is determined by the number of matching identical residues divided by the total number of residues of the "longer" sequence in the aligned region. The "longer" sequence is the one having the most actual residues in the aligned region (gaps introduced by WU-Blast-2 to maximize the alignment score are ignored). The term" positives" in the context of sequence comparison performed as described above includes residues in the sequences compared that are not identical but have similar properties (e.g. as a result of conservative substitutions). The % value of positives is determined by the fraction of residues scoring a positive value in the BLOSUM 62 matrix divided by the total number of residues in the longer sequence, as defined above. The well known Smith-Waterman algorithm may as well be used to determine sequence homologies. Preferred parameters for the nucleic acid sequence comparison include the following: (Algorithm: cf. Needleman and Wunsch, 1970); comparison matrix: matches = + 10, mismatches = 0, gap penalty 50, gap length penalty: 3. The GAP program is also suitable for use with the above parameters. The above parameters are the default parameters for nucleic acid sequence comparisons.

By the same token the phrase "sequence identity" may also refer to the percentage of (%) amino acid sequence identity of a candidate amino acid relative to a reference amino acid sequence, i.e. the percentage of amino acids residues in a candidate sequence that are identical with those of the reference sequence, after aligning the sequences and introducing gaps, if necessary, to achieve the maximum percent sequence identity, and not considering any conservative substitutions as part of the sequence identity. Methods of determining amino acid sequence identity are generally known in the art and include for example the widely known BLASTP algorithm.

In another preferred embodiment the nucleic acid used according to the invention codes for a polypeptide listed in Figure 6 and Table 1 below, even more preferably a nucleic acid coding for a VHb bacterial hemoglobin according to SEQ ID NO: 1, or a nucleic acid coding for a bacterial flavohemoglobin listed in Figure 6 and Table 1 below, even more preferably a FHP bacterial flavohemoglobin according to SEQ ID NO:3. Preferably, the nucleic acid according to the invention codes for the FHP polypeptide according to the SEQ ID NO. 3.

Even more preferably the nucleic acid used according to the invention is a VHb nucleic acid according to SEQ ID NO: 2 or a FHP nucleic acid according to SEQ ID NO: 4.

Preferably the nucleic acid defined in a3) above codes for a polypeptide having at least about 65%, more preferably at least about 70%, even more preferably at least about 80%, most preferably at least about 90%, most preferably at least about 95% of sequence identity to one of the polypeptide sequences defined in a1) and a2) above and which nucleic acid encodes a polypeptide having oxidative and/or nitrosative stress protective activity.

Preferably a variant of the nucleic acid defined in a8) above possesses at least about 40%, preferably at least about 50%, more preferably at least about 60%, more preferably at least about 70%, even more preferably at least about 80%, most preferably at least about 90%, most preferably at least about 95% of the nucleic acid sequence length of the nucleic acid defined in a1) to a4), preferably of the nucleic acid defined in a2) or a4). Even more preferably the variant further displays at least about 40%, preferably at least about 50%, more preferably at least about 60%, more preferably at least about 70%, even more preferably at least about 80%, most preferably at least about 90%, most preferably at least about 95% sequence identity to a nucleic acid defined in a1), preferably of a nucleic acid defined in a2), even more preferably of the VHb nucleic acid according to the SEQ ID NO. 2, most preferably of the FHP nucleic acid according to the SEQ ID NO. 4.

Within the meaning of the invention the term "variant" also encompasses nucleic acids coding for fusion proteins resulting from operably linking at least two polypeptides, each encoded by a nucleic acids defined in a1) to a9) above or by operably linking at least one polypeptide encoded by a nucleic acids defined in a1) to a9) above with a polypeptide encoded by a heterologous nucleic acid sequence, such as a marker molecule. The different parts of the fusion protein may be operably linked by linkage of the respective encoding nucleic acid sequences or alternatively by linkage of the respective polypeptides upon expression. Preferred embodiments of the fusion proteins comprise a sequence according to SEQ ID NOS: 39 and 41 or a nucleic acid coding for the fusion protein selected from a nucleic acid sequence according to SEQ ID NO: 40 and 42.

Preferably the fragment of the nucleic acid defined in a9) above possesses at least about 40%, preferably at least about 50%, more preferably at least about 60%, more preferably at least about 70%, even more preferably at least about 80%, most preferably at least about 90%, most preferably at least about 95% of the nucleic acid sequence length of a nucleic acid defined in a1) to a4), preferably of a nucleic acid defined in a2) or a4), even more preferably of the VHb nucleic acid according to the SEQ ID NO. 2, most preferably of the FHP nucleic acid according to the SEQ ID NO. 4. Preferred fragments and polypeptides encoded by the fragment are provided in Table 1, SEQ ID NO: 43 and 44, respectively.

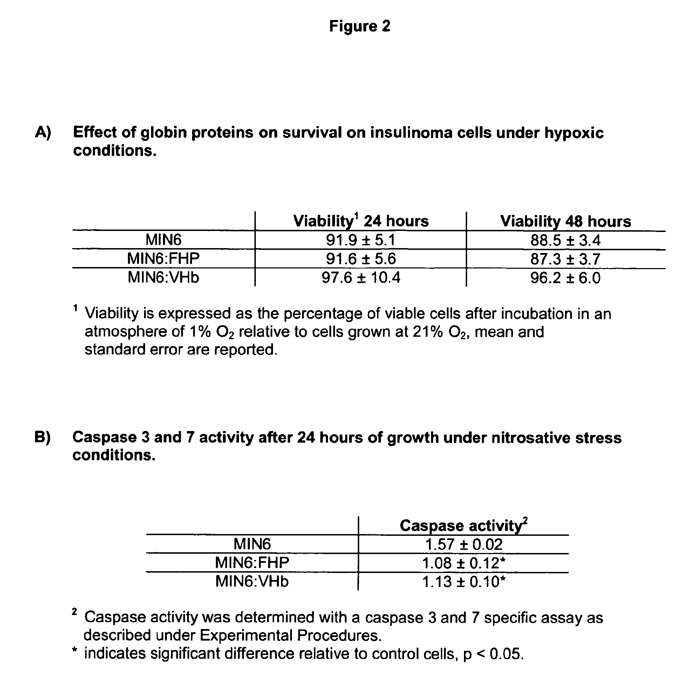

Within the meaning of the present invention the phrase "polypeptide having oxidative stress protective activity" is intended to mean a candidate polypeptide which displays essentially the same oxidative stress protective activity as the *Vitreoscilla sp.* hemoglobin according to the amino acid sequence of SEQ ID NO: 1 or the *Ralstonia eutropha* flavohemoglobin according to the amino acid sequence of SEQ ID NO: 3. Preferably, the candidate polypeptide shows essentially the same oxidative stress protective activity as the *Vitreoscilla sp.* hemoglobin according to the amino acid sequence of SEQ ID NO: 1 as described in the "hypoxia experiments" and Figure 2A, below, and using as quantitative and qualitative criteria for "essentially the same oxidative stress protective activity" the viability of cells expressing the candidate polypeptide in comparison to cells expressing the VHb polypeptide, both cultured under the same hypoxic conditions described below. An assay for measuring such activity is described in Frey et al., 2002 . Alternatively, the assay described in the examples may as well be employed. Preferably, candidate polypeptide is considered to be a polypeptide having oxidative stress protective activity when the cells expressing the candidate polypeptide display a viability of at least about 70%, more preferably of at least about 80%, even more preferably of at least about 90%, even preferably of at least about 100%, even more preferably of more than 100%, of the cells expressing the VHb polypeptide according to the amino acid sequence of SEQ ID NO: 1 or of the cells expressing the *Ralstonia eutropha* flavohemoglobin according to the amino acid sequence of SEQ ID NO: 3, importantly, both groups of cells (candidate vs. control) are subjected to the same experimental conditions described in detail below. It is preferred if the values are compared to the viability of cells expressing the VHb polypeptide according to the amino acid sequence of SEQ ID NO: 1.

Within the meaning of the present invention the phrase "polypeptide having nitrosative stress protective activity" is intended to encompass candidate polypeptides displaying essentially the same nitrosative stress protective activity as the *Vitreoscilla sp.* hemoglobin according to the amino acid sequence of SEQ ID NO: 1 or the *Ralstonia eutropha* flavohemoglobin according to the amino acid sequence of SEQ ID NO: 3. Preferably, the candidate polypeptide shows essentially the same nitrosative stress protective activity as the *Ralstonia eutropha* flavohemoglobin according to the amino acid sequence of SEQ ID NO: 3 as described in the caspase activity experiments and in Figure 2B, below, and using as quantitative and qualitative criteria for "essentially the same nitrosative stress protective activity" the caspase activity of cells expressing the candidate polypeptide in comparison to cells expressing the polypeptide according to the amino acid sequence of SEQ ID NO: 1 or of SEQ ID NO: 3, preferably of SEQ ID NO: 3, both groups of cells (candidate vs. control) cultured under the same nitrosative stress conditions described below, preferably after 24 hours of growth under nitrosative conditions. Preferably, the nitrosative stress is induced applying sodium nitroprusside at a final concentration of 100 µM. An assay for measuring such Caspase activity is described in Leytus et al., 1983. Preferably, a candidate polypeptide is considered to be a polypeptide having nitrosative stress protective activity when the cells expressing the candidate polypeptide display a caspase activity of less than about 140%, more preferably of less than about 130%, even more preferably of less than about 120%, even preferably of less than about 110%, even more preferably of less than about 100%, most preferably of less than about 90%, most preferably of less than about 80%, most preferably of less than about 70%, of the cells expressing the polypeptide according to the amino acid sequence of SEQ ID NO: 1 or SEQ ID NO: 3, preferably of SEQ ID NO: 3.

In another preferred embodiment of the host cell according to the invention the nucleic acid further comprises a promoter suitable for the expression of the nucleic acid and the expression of the nucleic acid encoding a polypeptide having oxidative and/or nitrosative stress protective activity is controlled by the promoter.

The term "promoter suitable for the expression of the nucleic acid" encompasses all kinds of promoter and regulatory elements such as enhancers which may be operably linked to the nucleic acid used according to the invention, particularly to the nucleic acid defined under a), preferably under a1) to a9), and which results in the expression of this nucleic acid when such expression cassette is contained or introduced into the host cell and the cell is cultured under conditions or kept in an environment suitable for the expression of the nucleic acid to be initiated under the control of the promoter. Typically, a promoter is a DNA regulatory region capable of binding RNA polymerase in a cell and initiating transcription of a downstream (3'direction) coding sequence. Usually, a 5'promoter sequence is bounded at its 3'terminus by the transcription initiation site and extends upstream (5'direction) to include the minimum number of bases or elements necessary to initiate transcription at levels detectable above background. Within the promoter sequence transcription initiation site are conveniently definable by mapping with nuclease S1, as well as by protein binding domains, i.e. consensus sequences responsible for the binding of RNA polymerase. Preferably the promoter is a heterologous promoter, though autologous promoters may as well be used.

The choice of the promoter will vary depending on the type of host cell employed and on the purpose of use and is generally known in the field. It is also generally known how to construct an expression cassette containing promoter and nucleic acids the expression of which is to be controlled by this promoter. For some cells and applications use of cell type-specific promoters may be indicated whereas in other cells and applications promoters regulated in many different cell types may be employed such as strong EF-1 alpha promoter, SV40-promoter, LTR-promoter and CMV-promoter. Most preferably the promoter is a promoter employed in the expression vectors used in the examples, i.e. pEF6-FHP (e.g. SEQ ID NO: 9) and pEF6-VHb (e.g. SEQ ID NO: 10) carrying the *R. eutropha fhp* and *Vitreoscilla vhb* gene, respectively, downstream of the strong EF-1 alpha promoter as displayed in Table 1, below.

In case the host cell according to the invention is a beta-cell, a pancreas cell-specific or even beta-cell-specific promoter may be used in host cells according to the invention in order to confine expression of the nucleic acid of the present invention to the desired cells. Examples of such promoters are the islet amyloid polypeptide (IAPP) promoter, the beta-cell-specific glucokinase (GK) promoter, and the insulin promoter, e.g. the human insulin promoter, the rat insulin promoter, the bovine insulin promoter, islet-specific glucose-6-phosphatase related protein (IGRP) promoter, or glucose transporter type 2 gene promoter. All of these promoters and their modes of use are generally known in the field.

The promoters according to the invention are intended to encompass constitutively active promoters as well as inducible promoters such as the tetracycline operator system in combination with a suitable repressor (Gossen et al. 1994).

The term "promoter suitable for the expression of the nucleic acid" is also intended to include enhancer elements which may be coupled to a promoter in order to increase the rate of expression of the nucleic acid contained in the host cell according to the invention or in order to restrict expression, induction or repression thereof to certain kinds of cell types depending on the use intended.

Preferably, the nucleic acid is contained in an expression vector which may be introduced into the host cell for purposes of expression of the nucleic acid of the invention. Suitable expression vectors are not particularly limited and include viral and non-viral expression vectors, such as plasmids, cosmids, bacteriophages, retroviral vectors, vaccinia virus vectors, lentiviral vectors, herpes virus vectors, such as HSV, baculoviral vectors, cytomegalovirus (CMV) vectors, papillomavirus vectors, simian virus (SV40) vectors, sindbis vectors, semliki forest virus vectors, adenoviral vectors, and adeno-associated viral (AAV) vectors. For introduction into the host cell the expression vector may as well be contained or attached to liposomes, cationic lipids, and conjugated to poly-lysine. Most preferably the promoter is a promoter employed in the expression vectors used in the examples, i.e. pEF6-FHP and pEF6-VHb carrying the R. *eutropha fhp* and *Vitreoscilla vhb* gene, respectively, downstream of the strong EF-1 alpha promoter.

The skilled artisan is well aware if and which additional sequences such as poly-A tails, signal peptides included for the purposes of secretion and intracellular targeting of the expressed polypeptide (secretory sequences), enhancers, myc-tags, matrix attachment regions and the like are to be introduced into the expression vector which will depend on the kind of host cell chosen for the purpose of use, the type of expression control intended and the type of nucleic acid to be expressed.

Gutless adenoviral vectors have been shown to achieve high-level transgene expression and improved duration of expression with reduced vector toxicity. Such vectors also have a large coding capacity (up to 38 kb) and can encode multiple transgene expression cassettes including ligand-regulated transcriptional control elements (Kochanek, 1999).

Retroviral vectors and lentiviral viral vectors have a coding capacity of approximately 8 kb, and the viral genome is capable of integration into the host cell chromosome, thus allowing for potentially life-long transgene expression. One non-limiting example of a lentivirus is Bovine-Immunodeficiency-Virus (BIV). (WO 01/44458).

AAV vectors integrate into the host genome and such vectors are capable of long- term transgene expression. The coding capacity of AAV vectors is approximately 5 kb.

The nucleic acids, heterologous nucleic acids and expression vectors used according to the invention may be introduced into the host cell according to the invention by conventional method generally known in the art and are for example described in Sambrook and Russell, 2001. Examples include transfection, infection, transformation, lipofection, gene gun based methods and the like. Preferably, prior to and/or post introduction of the nucleic acid into the host cell the cell is contacted with an anti-apoptotic agent in an amount sufficient to inhibit apoptosis of the cell. The nucleic acids may as well be introduced into the genome of the host cell. To this end a transgenic animal may be generated which carries e.g. beta-cells transgenic for at least one nucleic acids according to the invention which is expressed in these cells. Techniques for generating transgenic animals and cells are generally known in the art. The suitable transgenic host cells may then be isolated from the transgenic animal according to standard techniques.

"Anti-apoptotic agent" is intended to refer to a polypeptide, a polynucleotide, a small molecule inhibitor, or any other compound that may be designed through rational drug design starting from known inhibitors of apoptosis that inhibit apoptosis. Examples of anti-apoptotic agents include the following: an IAP polypeptide, e.g. NAIP, HIAP1, HIAP2, Survivin, Livin, Appollon, TS-IAP/HILP2/TIAP, a BIR domain-containing polypeptide having caspase-inhibiting activity, e.g. a polypeptide containing the BIR3 domain from human XIAP, fusion proteins or chimeric proteins containing all or a fragment of a BIR domain-containing polypeptide having caspase-inhibiting activity, mammalian or viral orthologues or homologues of an IAP polypeptide, or any other IAP polypeptide described herein; caspase inhibitors, e.g. viral caspase inhibitors such as baculovirus p35 or p49 protein or the polypeptide product of the CrmA Cowpox gene, dominant negative caspase constructs, e.g. FLIP (natural caspase-8 dominant negative) or artificial dominant negative polypeptides of caspase-8,-9,-3, or-7.

The term "caspases" within the meaning of the invention refers to cysteine proteases that cleave their substrates after specific aspartic acid residues, i.e. Asp-X, that are produced from inactive zymogens known as procaspases.

The term "host cell" is intended to encompass mammalian cells. Preferably the cells are derived mammals, such as from humans, domestic and farm animals, and zoo, sports, or pet animals, such as dogs, cats, cows, horses, sheep, pigs, etc.. The choice of the cell is evident to those skilled in the art and will depend on the area of application of the host cells according to the invention. Preferably, the mammalian cell is selected from a skin cell, a vascular cell, a neuron, a glia cell, a pancreatic cell, a hepatic cell, a chondrocyte, a stem cell, e.g. adult stem cells or embryonic stem cells, preferably capable of differentiating into insulin-producing cells, and xenogeneic cells from mammals different from human such as cells from pig, sheep, or baboon. The cells may as well be derived from a transgenic animal, e.g. from a transgenic pig expressing the nucleic acids according to the invention in its transgenic cells.

Even more preferred host cells are derived from an HK cell, a CHO cell, a COS cell, a Vero cell, a 293 cell, a HaCaT cell, a PC12 cell, a HepG2 cell, a primary pancreatic beta-cell, a MIN6 cell, a RIN cell, a Ins-1 cell, a NIT-a cell, a HIT-T15 cell, a betaTc-3 cell, an intestinal endocrine K cell, and an intestinal endocrine L cell, all of which are generally available. Particularly in the case of using the host cells in the context of diabetes, a preferred embodiment of the host cell relates to primary pancreatic beta-cells, cell lines derived from insulinomas such as MIN6 cells, RIN cells, Ins-1 cells, NIT-a cells, HIT-T15 cells betaTc-3 cells, beta cell lines such as described in Qin et al. 2002 and intestinal endocrine K and L cells. MIN6 cells represent a potential source of pancreatic beta-cells transplant, due to their ability to secrete insulin in response to glucose indistinguishable from normal pancreatic beta-cells (Miyazaki et al., 1990). Moreover, the host cell may be an autologous or a heterologous cell. The cell may be isolated from or provided by a living donor or a cadaveric donor, preferably the donor is a human donor.

According to another preferred embodiment the host cell comprises a heterologous nucleic acid suitable for expression of the polypeptide encoded by the heterologous nucleic acid and optionally for secretion of the polypeptide. As the host cells are particularly well protected against oxidative and/or nitrosative stress, whenever introduction of a host cell according to the invention into a cell culture, a tissue or an organisms is required, it may be particularly useful if the host cell expresses along with the nucleic acids conveying oxidative and/or nitrosative stress protection at least one more additional heterologous nucleic acid such as a gene which codes for a protein exhibiting the desired function, preferably coding for a therapeutically active polypeptide. If the host cells are used to produce and secrete a protein having therapeutic utility, such as a protein inhibiting neoangiogenesis in tumor tissue setting, the host cells according to the present invention due to their oxidative and/or nitrosative stress protection should not be attacked by the immune system and as a result these cells are also therapeutics suitable for cancer therapy. Those skilled in the art will readily appreciate and know which heterologous nucleic acids coding for proteins having therapeutic utility are suitable to be introduced into a host cell according to the invention in order to construct host cells protected against oxidative and/or nitrosative stress which serve as efficient shuttles to deliver therapeutic nucleic acids and proteins to their appropriate destination in the body of the organism to be treated. It is also generally known in the field which expression vectors and which promoters and secretory signals and the like should be employed in order to effectively express the heterologous nucleic acid of interest in the host cell according to the invention.

Preferably the host cells may be equipped with heterologous sequences generally known in the art which are suitable for the treatment of disease mediated by oxidative and nitrosative stress, such as a neurodegenerative disorder, a disorder of gastrointestinal motility, hypotension, hypertension, septic shock, toxic shock syndrome, hemodialysis, cancer, cachexia, a disease requiring immunosuppression, a disease requiring transplant therapy, an autoimmune disease, an inflammatory disease, including sunburn, eczema or psoriasis and respiratory conditions such as bronchitis, asthma, oxidant-induced lung injury and acute respiratory distress (ARDS), chronic obstructive pulmonary disease, glomerulonephritis, viral infections, myocarditis, viral hepatitis, alcoholic hepatitis, heart failure, atherosclerosis, arthritis, rheumatoid arthritis, chronic bowel disease, inflammatory bowel disease, ulcerative colitis, Crohn's disease, systemic lupus erythematosis (SLE), ocular conditions such as ocular hypertension, retinitis and uveitis, insulin-dependent type 1 diabetes mellitus, diabetes type 2, cystic fibrosis, hypoxia, hyperbaric oxygen convulsions, a toxicity indication, dementia, Sydenham's chorea, Parkinson's disease, Huntington's disease, amyotrophic lateral sclerosis, multiple sclerosis, Korsakoff's disease, imbecility related to cerebral vessel disorder, NO mediated cerebral trauma, ischemic brain edema, stroke, sleeping disorders, schizophrenia, depression, pre-menstrual syndrome (PMS), anxiety, drug addiction, pain, migraine, immune complex disease, allograft rejection, infections caused by invasive microorganisms which produce NO, and radio contrast induced renal failure.

Advantageously the host cell contains a heterologous nucleic acid coding for insulin, preferably human insulin, though insulin coding nucleic acids from other species are also applicable and generally known in the field. A preferred host cell according to the invention is a HK cell, a CHO cell, a COS cell, a Vero cell, a 293 cell, a HaCaT cell, a PC12 cell, a HepG2 cell, a pancreatic cell, preferably a beta-cell, an insulinoma cell line such as RIN cells, lns-1 cells, NIT-a cells, HIT-T15 cells and betaTc-3 cells, an intestinal endocrine K cell, and an intestinal endocrine L cell; even more preferably a MIN6 cell, containing as a heterologous nucleic acid a nucleic acid coding for insulin protein, preferably for human insulin. Such cells are particularly useful for but not limited to diabetes therapy, especially for diabetes type I therapy. Stem cells and beta-islet cell precursors are another source of cells for use in the methods of the invention because they can have an increased proliferative potential. An advantage of the use of stem cells over differentiated islet cells is that stem cells may be more adaptive by virtue of their ability to differentiate in situ. Use of stem cells may result in improved microcirculation and production of the full complement of different islet cell types which, in turn, are more responsive to the physiological needs of the recipient. Another source of cells that may be used in the invention, particularly suited but not limited to diabetes treatment, are neuroendocrine cells, such as those found in the pituitary and adrenal glands. These cells possess the secretory machinery needed for a regulated secretion of polypeptide hormones in response to external stimuli. Due to the expression of pro-hormone convertases 2 and 3 some of these cells can process proinsulin to insulin without any further genetic manipulation and represent a potential source of cells for use in the methods of the invention. Many of the studies using neuroendocrine cells as a target for insulin gene therapy have been performed in AtT20, a neuroendocrine cell line derived from a mouse pituitary corticotroph tumor (Moore et al., 1983). Allogeneic or isogeneic stem cells can be isolated from a preparation of pancreatic tissue, for example islet cells obtained from a biopsy sample of tissue from a diabetic patient. The cells can then be expanded ex vivo and the resulting progeny cells can be transplanted back into the donor. Inside the donor, such cells may differentiate to provide insulin-secreting cells, such as beta islet cells, to cells lost to the autoimmune attack that caused the diabetes. This approach can overcome the problem of immune rejection which might otherwise result from transplantation of beta islet cells from a different individual. Of course this approach applies *mutatis mutandis* to the treatment of other diseases treatable according to the invention.

Even more preferably, the nucleic acid encoding the insulin protein is contained in an expression vector suitable for expression in the selected host cell, preferably the nucleic acid coding for human insulin is under control of a suitable promoter described in detail above. It is particularly advantageous if the expression vector that harbors the insulin sequence is constructed in a way that the control of transcription and/or translation of the insulin mRNA into the insulin polypeptide is controlled by glucose itself or by a signalling cascade that is sensitive to extracellular and/or intracellular glucose concentration levels. Such systems are generally known in the field and permit insulin expression to be regulated as a function of the blood glucose concentration. The advantage of such a host cell is that due to its oxidative and/or nitrosative stress protection the host cell is not recognized by the immune system or at least to a lesser extent than the cells currently used and therefore insulin may be efficiently produced by these host cells, thus providing an efficient therapy of diabetes, preferably of diabetes type I disease.

The host cells according to the invention are well protected from oxidative and/or nitrosative stress. To increase the level of protection it may be advantageous that the host cell contains a nucleic acid coding for bacterial hemoglobin and a nucleic acid coding for a bacterial flavohemoglobin, for example a nucleic acid listed in Figure 6, preferably a nucleic acid coding for a bacterial hemoglobin or flavohemoglobin listed in Figure 6, most preferably the *Vitreoscilla sp.* hemoglobin according to the amino acid sequence of SEQ ID NO: 1 and the *Ralstonia eutropha* flavohemoglobin according to the amino acid sequence of SEQ ID NO: 3. The two nucleic acids may be contained in the same expression vector under control of the same or a different type of promoter or they may be contained in different expression vectors under control of the same or a different type of promoter. According to another preferred embodiment the host cell may still express another heterologous nucleic acid coding for a protein having (i) RNS and/or ROS degrading activity, (ii) an activity that promotes RNS and/or ROS metabolization, (iii) an activity that inhibits production of RNS and/or ROS or precursors thereof, and/or (iv) an activity that inhibits nitric oxide synthetase or cytokines involved in the increase of levels of RNS and/or ROS such as an inhibitory activity against IL-1 beta, TNF alpha, IFN-gamma. Such additional heterologous nucleic acid may increase the level of protection against oxidative and/or nitrosative stress in the host cells of the invention.

In another aspect of the invention a culture of cells is provided containing at least one host cell according to the invention. The culture of cells may be derived from cells obtained from a donor, preferably from a healthy subject or from a patient in need of treatment with the cell culture according to the invention, preferably a patient suffering from a disease treatable based on the present invention such as a disease described in detail above. Such cells may then be cultured and/or expanded, and supplemented with host cells according to the invention resulting in a culture of cells according to the invention. Alternatively, the nucleic acids and/or expression vectors according to the invention, preferably along with the heterologous nucleic acids and/or expression vectors according to the invention, may be introduced into cultured cells in order to produce a culture of cells according to the invention. Such cells are then protected against oxidative and/or nitrosative stress and may be used for research on immunological issues or for transplantation or implantation into a host, preferably a patient in need of treatment with the cell culture according to the invention. Preferably the culture of cell is a culture of pancreas host cells according to the invention such as MIN6 cells, which host cells express insulin, preferably human insulin.

In another aspect of the invention a tissue containing at least one host cell according to the invention is provided. In analogy to the culture of cells according to the invention, the tissue may have been isolated from a donor, preferably from a healthy subject or from a patient in need of treatment with the cell culture according to the invention or alternatively the tissue may be an artificial tissue, for example comprising a biopolymer matrix seeded with cells. Such tissue may then be supplemented with host cells according to the invention and thus a tissue of the invention is obtained. Alternatively, the nucleic acids and/or expression vectors according to the invention, preferably along with the heterologous nucleic acids and/or expression vectors according to the invention, may be introduced into cells of the tissue in order to produce a tissue according to the invention. Such tissue is protected against oxidative and/or nitrosative stress and may be used for transplantation into a host, preferably a patient in need of treatment with the tissue according to the invention. Preferably, the tissue according to the invention is a pancreatic islet, pancreatic islet graft containing at least one host cell according to the invention or a culture of cells as described above. It is also advantageous if the tissue is derived from vascular tissue, neural tissue, muscle, pancreas, liver, lung, stomach, intestine, and tissue from the urogenital system. Even more preferably the tissue is a pancreas tissue containing host cells according to the invention such as MIN6 cells, which host cells preferably express insulin.

According to another aspect of the invention an organ is provided containing at least one host cell, a tissue or a culture of cells according to the invention. Preferably the organ is selected from muscle, pancreas, liver, lung, stomach, intestine, and organs from the tissue from the urogenital system. The organ according to the invention can be obtained, produced and used as described in the section describing the tissue according to the invention.

The term "mammal" for purposes of the present invention refers to any animal classified as a mammal, including humans, domestic and farm animals, and zoo, sports, or pet animals, such as dogs, cats, cows, horses, sheep, pigs, etc. Preferably the mammal is human.

According to yet another aspect of the invention a method for producing a host cell according to the invention is provided, comprising the steps of:
(a) providing a mammalian cell, and
(b) introducing into the cell a nucleic acid comprising a nucleic acid selected from
   b1) a nucleic acid coding for a bacterial hemoglobin or a bacterial flavohemoglobin having oxidative and/or nitrosative stress protective activity, or a complementary strand thereof;
   b2) a nucleic acid coding for a polypeptide selected from the group consisting of an amino acid sequence according to SEQ ID NO: 1, 3, 11, 13, 15, 17, 19, 21, 23, 25, 27, 29, 31, 33, 35, 37, 39, 41, and 43; or a complementary strand thereof, and which nucleic acid encodes a polypeptide having oxidative and/or nitrosative stress protective activity;
   b3) a nucleic acid coding for a polypeptide having at least 60% sequence identity to one of the polypeptide sequences defined in b1) and b2) and which nucleic acid encodes a polypeptide having oxidative and/or nitrosative stress protective activity;
   b4) a nucleic acid selected from the group consisting of a nucleic acid sequence according to SEQ ID NO: 2, 4, 12, 14, 16, 18, 20, 22, 24, 26, 28, 30, 32, 34, 36, 38, 40, 42, and 44;
   b5) a nucleic acid which on the basis of the genetic code is degenerate to one of the nucleic acid sequences defined in b1) to b4) and which nucleic acid or complementary strand thereof encodes a polypeptide having oxidative and/or nitrosative stress protective activity;
   b6) a nucleic acid which hybridizes with one of the nucleic acids defined in b1) to b5) and which nucleic acid or complementary strand thereof encodes a polypeptide having oxidative and/or nitrosative stress protective activity;
   b7) a nucleic acid having at least 60% sequence identity to one of the nucleic acid sequences defined in b1) to b6) and which nucleic acid or complementary strand thereof encodes a polypeptide having oxidative and/or nitrosative stress protective activity;
   b8) a variant of one of the nucleic acids defined in b1) to b7), wherein compared to one of the nucleic acids defined in b1) to b7) the variant contains at least one addition, deletion, insertion and/or inversion and wherein the variant or the complementary strand thereof encodes a polypeptide having oxidative and/or nitrosative stress protective activity; and
   b9) a fragment of one of the nucleic acids defined in b1) to b8), wherein the fragment or the complementary strand thereof encodes a polypeptide having oxidative and/or nitrosative stress protective activity.

The statements made above with reference to the nucleic acids defined in a) and a1) to a9), fragments, fusion proteins, variants, promoters, expression vectors, host cells etc. containing these nucleic acids apply *mutatis mutandis* to the nucleic acids defined in b) and b1) to b9), to promoters, expression vectors, and host cells containing the nucleic acids defined in b) and b1) to b9) and to all other embodiments referring to the nucleic acids defined in b) and b1) to b9 unless stated differently.

The nucleic acids, heterologous nucleic acids and expression vectors used according to the invention may be introduced into the host cell according to the invention by conventional methods generally known in the art and for example described in

Sambrook and Russell, 2001 such as transfection, infection, transformation, lipofection, gene gun based methods and the like. Preferably, prior to and/or post introduction of the nucleic acid into the host cell the cell is contacted with an anti-apoptotic agent in an amount sufficient to inhibit apoptosis of the cell.

Preferably, the method of producing a host cell further comprises step
c) selecting among the cells containing the nucleic acid those cells, which express the nucleic acid defined in b).

This step can also be achieved by conventional methods for example by immunohistochemical methods, by quantitative PCR techniques or by northern blot analysis and the like, all of these methods being well known in the field and readily available (cf. e.g. Sambrook and Russell, 2001). Such preferred method may be useful if it is required to ensure that the host cells produced by the method express a constant or at least comparable amount of the nucleic acid. This step may be adjusted such that those cells are selected among the produced cells that exhibit a certain level of expression of the nucleic acids introduced into the host cells. The level of expression may for example be controlled by the choice of promoter or by introduction of further control elements such as enhancers.

According to a preferred embodiment of the method the nucleic acid further comprises a promoter suitable for the expression of the nucleic acid and wherein the expression of the nucleic acid encoding a polypeptide having oxidative and/or nitrosative stress protective activity is controlled by the promoter. Suitable cells and promoters have been described above.

Even more preferably the nucleic acid is contained in an expression vector, and advantageously, the expression vector is a viral or non-viral vector as defined above and preferably selected from plasmids, cosmids, bacteriophages, retroviral vectors, vaccinia virus vectors, lentiviral vectors, herpes virus vectors, such as HSV, baculoviral vectors, cytomegalovirus (CMV) vectors, papillomavirus vectors, simian virus (SV40) vectors, sindbis vectors, semliki forest virus vectors, adenoviral vectors, and adeno-associated viral (AAV) vectors.

Even more preferred is a method of producing a host cell which further comprises step
d) introducing into the cell a heterologous nucleic acid coding for insulin protein, wherein the heterologous nucleic acid is either contained in an expression cassette which is located on the same nucleic acid molecule as the nucleic acid defined in b) or located on a separate nucleic acid molecule and wherein in the former case the heterologous nucleic acid is introduced into the cell in step b) and in the latter case the heterologous nucleic acid and the nucleic acid defined in b) are introduced simultaneously or sequentially in either order into the cell.

Methods for introducing the heterologous nucleic acid are not particularly limited and have already been described above.

Preferably the heterologous nucleic acid is an insulin sequence. More preferably, the cell produces insulin in an amount sufficient to treat diabetes in the individual. Suitable expression vectors and suitable heterologous sequences have also been outlined before.

Cells suitable for use in the method for producing a cell have previously been described and preferably are mammalian cells, advantageously a skin cell, a vascular cell, a neuron, a glia cell, a pancreatic cell, a hepatic cell, a chondrocyte and a stem cell. Even more preferred host cells are derived from a HK cell, a CHO cell, a COS cell, a Vero cell, a 293 cell, a HaCaT cell, a PC12 cell, a HepG2 cell, a primary pancreatic beta-cell, a MIN6 cell, a RIN cell, a lns-1 cell, a NIT-a cell, a HIT-T15 cell, a betaTc-3 cell, an intestinal endocrine K cell, and an intestinal endocrine L cell, all of which are generally available. Particularly in the case of using the host cells in the context of diabetes, a preferred embodiment of the host cell relates to pancreatic beta-cells, MIN6 cells, RIN cells, Ins-1 cells, NIT-a cells, HIT-T15 cells and betaTc-3 cells, and beta cell lines such as described in Qin et al. 2002. Moreover, the host cell may be an autologous or a heterologous cell.

Another aspect of the invention relates to a method of treating a patient in need of administration of host cells according to the invention, comprising the steps of:
a) administering to the patient host cells according to the invention, preferably host cells which express at least one heterologous sequence, preferably a nucleic acid sequence coding for a polypeptide having therapeutic activity.

Preferably the polypeptide having therapeutic activity is insulin, especially human insulin, and the patient suffers from diabetes, especially diabetes type I disease. More preferably, the patient in need of the treatment suffers from a disease mediated by oxidative and nitrosative stress, such as a neurodegenerative disorder, a disorder of gastrointestinal motility, hypotension, hypertension, septic shock, toxic shock syndrome, hemodialysis, cancer, cachexia, a disease requiring immunosuppression, a disease requiring transplant therapy, an autoimmune disease, an inflammatory disease, including sunburn, eczema or psoriasis and, a respiratory condition such as bronchitis, asthma, oxidant-induced lung injury and acute respiratory distress (ARDS), chronic obstructive pulmonary disease, glomerulonephritis, viral infections, myocarditis, viral hepatitis, alcoholic hepatitis, heart failure, atherosclerosis, arthritis, rheumatoid arthritis, chronic bowel disease, inflammatory bowel disease, ulcerative colitis, Crohn's disease, systemic lupus erythematosis (SLE), ocular conditions such as ocular hypertension, retinitis and uveitis, insulin-dependent type 1 diabetes mellitus, diabetes type 2, cystic fibrosis, hypoxia, hyperbaric oxygen convulsions, a toxicity indication, dementia, Sydenham's chorea, Parkinson's disease, Huntington's disease, amyotrophic lateral sclerosis, multiple sclerosis, Korsakoff's disease, imbecility related to cerebral vessel disorder, NO mediated cerebral trauma, ischemic brain edema, stroke, sleeping disorders, schizophrenia, depression, pre-menstrual syndrome (PMS), anxiety, drug addiction, pain, migraine, immune complex disease, allograft rejection, infections caused by invasive microorganisms which produce NO, and radio contrast induced renal failure. Suitable heterologous sequences and expression vector for use have already been described before.

According to another aspect of the present invention a pharmaceutical composition is provided which contains a host cell according to the invention and a pharmaceutical acceptable carrier.

According to another aspect of the invention a host cell according to the invention is used for the production of a medicament for the treatment of a patient in need of protection against oxidative and/or nitrosative stress and/or for the treatment of a tissue or organ for implantation and/or transplantation into a patient.

Preferably the medicament and/or pharmaceutical composition of the invention is used for the treatment of disease mediated by oxidative and nitrosative stress, a neurodegenerative disorder, a disorder of gastrointestinal motility, hypotension, hypertension, septic shock, toxic shock syndrome, hemodialysis, cancer, cachexia, a disease requiring immunosuppression, a disease requiring transplant therapy, an autoimmune disease, an inflammatory disease, including sunburn, eczema or psoriasis and, a respiratory condition such as bronchitis, asthma, oxidant-induced lung injury and acute respiratory distress (ARDS), chronic obstructive pulmonary disease, glomerulonephritis, viral infections, myocarditis, viral hepatitis, alcoholic hepatitis, heart failure, atherosclerosis, arthritis, rheumatoid arthritis, chronic bowel disease, inflammatory bowel disease, ulcerative colitis, Crohn's disease, systemic lupus erythematosis (SLE), ocular conditions such as ocular hypertension, retinitis and uveitis, insulin-dependent type 1 diabetes mellitus, diabetes type 2, cystic fibrosis, hypoxia, hyperbaric oxygen convulsions, a toxicity indication, dementia, Sydenham's chorea, Parkinson's disease, Huntington's disease, amyotrophic lateral sclerosis, multiple sclerosis, Korsakoff's disease, imbecility related to cerebral vessel disorder, NO mediated cerebral trauma, ischemic brain edema, stroke, sleeping disorders, schizophrenia, depression, pre-menstrual syndrome (PMS), anxiety, drug addiction, pain, migraine, immune complex disease, allograft rejection, infections caused by invasive microorganisms which produce NO, and radio contrast induced renal failure.

Preferably the medicament and/or pharmaceutical composition of the invention is used for the treatment of diabetes, preferably diabetes type 1 disease. Even more preferably, the host cell used as a medicament and/or in a pharmaceutical composition of the invention is a host cell containing a heterologous nucleic acid sequence coding for insulin as has been described in detail above.

The host cell can be administered to the patient according to methods generally known in the field, including by local or systematic administration, by surgery including minimal-invasive surgery, or by injection, preferably by subcutaneous, parenteral, intra venous or intramuscular injection.

Preferably administration also encompasses transplantation of the host cells into a location in the subject that allows for transplantation and survival of the transplant generally known to the skilled physician. In the case of diabetes-treatment, especially diabetes type I-treatment, the location of the insulin-producing host cells should allow for delivery of insulin secreted by the cells into the blood. Most preferably the cells are implanted into the pancreas but it is also feasible to introduce the host cells into the blood stream to achieve successful therapy. Suitable locations for the transplant include the pancreas, the liver, e.g. the liver portal vein, and the kidney, e.g. the kidney capsule. If desirable, the cells can be encapsulated prior to transplantation, as is described herein.

The term "treatment" is intended to refer to both therapeutic treatment and prophylactic or preventative measures, wherein the object is to prevent or slow down (lessen) the targeted pathologic condition or disorder. Those in need of treatment include those already have the disorder as well as those prone to have the disorder or those in whom the disorder is to be prevented.

Preferably, the medicament and/or pharmaceutical composition of the invention, preferably the host cell, is administered in a therapeutically effective amount. A "therapeutically effective amount" refers to an amount effective, at dosages and for periods of time necessary, to achieve the desired therapeutic result. A therapeutically effective amount and the dose regimen of the medicament may vary according to factors such as the disease state, age, sex, and weight of the individual. A therapeutically effective amount is also one in which any toxic or detrimental effects of the medicament are outweighed by the therapeutically beneficial effects. In the context of prophylactic use "therapeutically effective amount" refers to an amount effective, at dosages and for periods of time necessary, to achieve the desired prophylactic result. Typically, since a prophylactic dose is used in subjects prior to or at an earlier stage of disease, the prophylactically effective amount will be less than the therapeutically effective amount.

Desirably, the increase in survival of the transplanted cells is at least 20%, relative to control cells, and may be more than 50%, 75%, 100%, 200%, or 500%. Suitable control cells are generally known in the field and preferably include cells that do not contain the nucleic acid according to the invention, or which contain a control vector lacking the nucleic acid according to the invention. Preferably the control cells are of the same cell type as the host cells according to the invention.

Comparisons in the survival rate of host cells can be made at any time following transplantation (e.g. , one week, one month, three months, six months, one year, five years, etc.), but it is most preferred to make the comparison at one month following the transplant. It is also desirable that the transplanted cells survive in the recipient for more than a week; desirably three months; more desirably one year; and most desirably for the natural life of the individual receiving treatment.
Similarly, the host cells will desirably produce insulin in amounts sufficient to treat diabetes in the recipient host for a longer period than control cells will do. In certain embodiments, the cells will produce insulin in amounts sufficient to treat diabetes in the recipient for more than a week; desirably three months; more desirably one year; and most desirably for the natural life of the recipient.

Once transplanted into the patient suffering from diabetes, the insulin producing host cells according to the invention are protected from challenge by the immune system. Preferably, the therapy does not require further administration of immunosuppressive agents to prevent rejection of host cell grafts avoiding known problems posed by administration of these agents, i.e. such agents often impair the ability of the transplanted cells to respond to glucose and to produce insulin. By increasing the survival of and decreasing the extent of the immune challenge to the host cells, the expression of the nucleic acids according to the invention can greatly improve the outcome for the recipient.

According to a preferred embodiment the medicament and/or pharmaceutical composition of the invention and/or the host cells is/are combined with administration of a therapeutically effective amount of a second medicament. The second medicament may for example be an immunosuppressive agent, i.e. any agent that prevents, delays the occurrence of, or reduces the intensity of an immune reaction against a foreign cell in a recipient host, particularly a transplanted cell. Examples of immunosuppressive agents include, but are not limited to, cyclosporin, cyclophosphamide, prednisone, dexamethasone, methotrexate, azathioprine, mycophenolate, thalidomide, FK-506, sirolimus, tacrolimus, daclizumab, and systemic steroids. Despite the ability of the transplanted host cells to provide protection from oxidative and nitrosative stress, administration of immunosuppressive agents may in some cases be indicated to protect e.g. yet undestroyed beta-cells of the patient's pancreas, i.e. cells different from the host cells, from attack by the immune system. The second medicament can be administered together or subsequently in any order with the "first medicament".

The medicament and/or pharmaceutical composition of the invention may be administered together with suitable carrier. The term "carrier" within the meaning of the invention not only covers carriers known in the art but also encompasses additives, solvents and adjuvants, diluent and excipients that are also generally known in the art and that may be used for the medicament or pharmaceutical composition according to the invention. Intended to be covered by the term "carrier" are any of the carriers or diluents that are well-known in the art for the stabilization and/or administration of a nucleic acid based or host cell based medicaments or pharmaceutical compositions which may be administered without undue toxicity. The choice of the pharmaceutically acceptable carrier and its subsequent processing enables the medicaments and/or pharmaceutical compositions of the present invention to be provided in either liquid or solid form. When the medicament and/or pharmaceutical composition is in liquid form, the pharmaceutically acceptable carrier comprises for example a stable carrier or a diluent suitable for subcutaneous, intramuscular, intraarterial, intravenous or intracoronary injection or infusion. Suitable carriers or diluents for injectable or infusible solutions are preferably nontoxic to a human recipient at the dosages and concentrations employed, and include sterile water, sugar solutions, saline solutions, protein solutions or combinations thereof. Typically, the pharmaceutically acceptable carrier includes a buffer and one or more stabilizers, reducing agents, anti-oxidants and/or anti-oxidant chelating agents. The use of buffers, stabilizers, reducing agents, anti-oxidants and chelating agents in the preparation of nucleic acid based or host cell based compositions, particularly in the medicaments and/or pharmaceutical compositions of the invention, is well-known in the art. The choice of the carrier will depend on the type of medicament and/or pharmaceutical composition used, the treatment and route of administration envisioned and is also generally known in the art.

Another aspect of the invention relates to a method of treating a patient in need of administration of nucleic acids according to the invention, comprising the steps of:
a) administering to the patient a nucleic acid according to the invention as has been described in detail above and in the following pharmaceutical composition for diseases treatable according to the invention and set forth in detail below.

A further aspect of the present invention relates to a pharmaceutical composition comprising a nucleic acid comprising a nucleic acid selected from
a1) a nucleic acid coding for a bacterial hemoglobin or a bacterial flavohemoglobin having oxidative and/or nitrosative stress protective activity, or a complementary strand thereof;
a2) a nucleic acid coding for a polypeptide selected from the group consisting of an amino acid sequence according to SEQ ID NO: 1, 3, 11, 13, 15, 17, 19, 21, 23, 25, 27, 29, 31, 33, 35, 37, 39, 41, and 43; or a complementary strand thereof, and which nucleic acid encodes a polypeptide having oxidative and/or nitrosative stress protective activity;
a3) a nucleic acid coding for a polypeptide having at least 60% sequence identity to one of the polypeptide sequences defined in a1) and a2) and which nucleic acid encodes a polypeptide having oxidative and/or nitrosative stress protective activity;
a4) a nucleic acid selected from the group consisting of a nucleic acid sequence according to SEQ ID NO: 2, 4, 12, 14, 16, 18, 20, 22, 24, 26, 28, 30, 32, 34, 36, 38, 40, 42, and 44;
a5) a nucleic acid which on the basis of the genetic code is degenerate to one of the nucleic acid sequences defined in a1) to a4) and which nucleic acid or complementary strand thereof encodes a polypeptide having oxidative and/or nitrosative stress protective activity;
a6) a nucleic acid which hybridizes with one of the nucleic acids defined in a1) to a5) and which nucleic acid or complementary strand thereof encodes a polypeptide having oxidative and/or nitrosative stress protective activity;
a7) a nucleic acid having at least 60% sequence identity to one of the nucleic acid sequences defined in a1) to a6) and which nucleic acid or complementary strand thereof encodes a polypeptide having oxidative and/or nitrosative stress protective activity;
a8) a variant of one of the nucleic acids defined in a1) to a7), wherein compared to one of the nucleic acids defined in a1) to a7) the variant contains at least one addition, deletion, insertion and/or inversion and wherein the variant or the complementary strand thereof encodes a polypeptide having oxidative and/or nitrosative stress protective activity; and
a9) a fragment of one of the nucleic acids defined in a1) to a8), wherein the fragment or the complementary strand thereof encodes a polypeptide having oxidative and/or nitrosative stress protective activity;
and a pharmaceutical acceptable carrier.

Preferably, the nucleic acid sequences coding for bacterial hemoglobin and flavohemoglobin, respectively, are derived from the genus *Ralstonia, Vitreoscilla, Escherichia, Pseudomonas, Streptococcus, Deinococcus, Klebsiella, Saccharomycetes, Salmonella, Campylobacter, Mycobacterium;* even more preferred from *Vitreoscilla sp.* or *Ralstonia eutropha,* even more preferably the nucleic acid of the invention is a bacterial hemoglobin or flavohemoglobin listed in Figure 6, most preferably a nucleic acid coding for a bacterial hemoglobin or flavohemoglobin listed in Figure 6.

Preferably, the nucleic acid is contained in an expression vector, which can be a viral or a non-viral expression vector as defined above and in particular an expression vector selected from plasmids, cosmids, bacteriophages, retroviral vectors, vaccinia virus vectors, lentiviral vectors, herpes virus vectors, baculoviral vectors, cytomegalovirus (CMV) vectors, papillomavirus vectors, simian virus (SV40) vectors, sindbis vectors, semliki forest virus vectors, adenoviral vectors, and adeno-associated viral (AAV) vectors. Suitable vectors and expression systems have already been introduced above.

More preferably, the pharmaceutical composition further comprises a heterologous nucleic acid coding for a therapeutically active polypeptide, wherein the heterologous nucleic acid is either contained in the same expression vector located on the same nucleic acid molecule as the nucleic acid selected from a1) to a9), or the heterologous nucleic acid is contained in a separate expression vector located on a separate nucleic acid molecule than the nucleic acid selected from a1) to a9) as defined above. It is particularly preferred if the heterologous nucleic acid codes for insulin.

According to yet another aspect of the invention a method of using a nucleic acid is provided, comprising a nucleic acid selected from
a1) a nucleic acid coding for a bacterial hemoglobin or a bacterial flavohemoglobin having oxidative and/or nitrosative stress protective activity, or a complementary strand thereof;
a2) a nucleic acid coding for a polypeptide selected from the group consisting of an amino acid sequence according to SEQ ID NO: 1, 3, 11, 13, 15, 17, 19, 21, 23, 25, 27, 29, 31, 33, 35, 37, 39, 41, and 43; or a complementary strand thereof, and which nucleic acid encodes a polypeptide having oxidative and/or nitrosative stress protective activity;
a3) a nucleic acid coding for a polypeptide having at least 60% sequence identity to one of the polypeptide sequences defined in a1) and a2) and which nucleic acid encodes a polypeptide having oxidative and/or nitrosative stress protective activity;
a4) a nucleic acid selected from the group consisting of a nucleic acid sequence according to SEQ ID NO: 2, 4, 12, 14, 16, 18, 20, 22, 24, 26, 28, 30, 32, 34, 36, 38, 40, 42, and 44;
a5) a nucleic acid which on the basis of the genetic code is degenerate to one of the nucleic acid sequences defined in a1) to a4) and which nucleic acid or complementary strand thereof encodes a polypeptide having oxidative and/or nitrosative stress protective activity;
a6) a nucleic acid which hybridizes with one of the nucleic acids defined in a1) to a5) and which nucleic acid or complementary strand thereof encodes a polypeptide having oxidative and/or nitrosative stress protective activity;
a7) a nucleic acid having at least 60% sequence identity to one of the nucleic acid sequences defined in a1) to a6) and which nucleic acid or complementary strand thereof encodes a polypeptide having oxidative and/or nitrosative stress protective activity;
a8) a variant of one of the nucleic acids defined in a1) to a7), wherein compared to one of the nucleic acids defined in a1) to a7) the variant contains at least one addition, deletion, insertion and/or inversion and wherein the variant or the complementary strand thereof encodes a polypeptide having oxidative and/or nitrosative stress protective activity; and
a9) a fragment of one of the nucleic acids defined in a1) to a8), wherein the fragment or the complementary strand thereof encodes a polypeptide having oxidative and/or nitrosative stress protective activity;
for the production of a medicament for the treatment of a patient in need of protection against oxidative and/or nitrosative stress and/or for the treatment of a tissue or organ for implantation and/or transplantation into a patient.

Preferably the patient suffers from a disease selected from a disease mediated by oxidative and nitrosative stress, a neurodegenerative disorder, a disorder of gastrointestinal motility, hypotension, hypertension, septic shock, toxic shock syndrome, hemodialysis, cancer, cachexia, a disease requiring immunosuppression, a disease requiring transplant therapy, an autoimmune disease, an inflammatory disease, including sunburn, eczema or psoriasis and, a respiratory condition such as bronchitis, asthma, oxidant-induced lung injury and acute respiratory distress (ARDS), chronic obstructive pulmonary disease, glomerulonephritis, viral infections, myocarditis, viral and alcoholic hepatitis, heart failure, atherosclerosis, arthritis, rheumatoid arthritis, chronic bowel disease, inflammatory bowel disease, ulcerative colitis, Crohn's disease, systemic lupus erythematosis (SLE), ocular conditions such as ocular hypertension, retinitis and uveitis, insulin-dependent type 1 diabetes mellitus, diabetes type 2, cystic fibrosis, hypoxia, hyperbaric oxygen convulsions, a toxicity indication, dementia, Sydenham's chorea, Parkinson's disease, Huntington's disease, amyotrophic lateral sclerosis, multiple sclerosis, Korsakoff's disease, imbecility related to cerebral vessel disorder, NO mediated cerebral trauma, ischemic brain edema, stroke, sleeping disorders, schizophrenia, depression, pre-menstrual syndrome (PMS), anxiety, drug addiction, pain, migraine, immune complex disease, allograft rejection, infections caused by invasive microorganisms which produce NO, and radio contrast induced renal failure.

Preferably, the nucleic acid sequences coding for bacterial hemoglobin or flavohemoglobin contained in the pharmaceutical composition, are derived from the genus *Ralstonia, Vitreoscilla, Escherichia, Pseudomonas, Streptococcus, Deinococcus, Klebsiella, Saccharomycetes, Salmonella, Campylobacter, Mycobacterium;* even more preferred from *Vitreoscilla sp.* or *Ralstonia eutropha,* even more preferably the nucleic acid is a bacterial hemoglobin or flavohemoglobin listed in Table 1 or Figure 6, most preferably a nucleic acid coding for a bacterial hemoglobin or flavohemoglobin listed in Table 1 or Figure 6.

Preferably, the nucleic acid is contained in an expression vector. Suitable expression vectors are not particularly limited and include viral and non-viral expression vectors, such as plasmids, cosmids, bacteriophages, retroviral vectors, vaccinia virus vectors, lentiviral vectors, herpes virus vectors, such as HSV, baculoviral vectors, cytomegalovirus (CMV) vectors, papillomavirus vectors, simian virus (SV40) vectors, sindbis vectors, semliki forest virus vectors, adenoviral vectors, and adeno-associated viral (AAV) vectors. Selection, construction of such vectors has been described above and is also generally known in the art.

More preferably, the medicament further comprises a heterologous nucleic acid coding for a therapeutically active polypeptide, wherein the heterologous nucleic acid is either contained in the same expression vector located on the same nucleic acid molecule as the nucleic acid selected from a1) to a9), or the heterologous nucleic acid is contained in a separate expression vector located on a separate nucleic acid molecule than the nucleic acid selected from a1) to a9). It is preferred if the heterologous nucleic acid codes for insulin, preferably human insulin. The construction of such gene therapeutical vectors is known in the art and has for example been described by Miller and Calos, 1987 and Sambrook and Russell, 2001. Ideally the gene therapeutical vector would be equipped with a means for target cell specific delivery of the medicament which is known in the field. Preferably the expression vector is a viral vector such as those mentioned above.

Such medicaments and/or pharmaceutical compositions would be particularly useful for delivering the nucleic acids according to the invention to target cells, preferably pancreatic cells which upon uptake and expression of the nucleic acid would protect the cells from nitrosative and/or oxidative stress and optionally express a heterologous nucleic acid coding for a therapeutically active polypeptide such as insulin.

It is intended to illustrate the invention with the following figures and examples, but in no way to limit it. On the basis of the description and the examples, other embodiments are accessible to the skilled person, which are also included.

**Table 1**

| **Table of Sequences** (for further details cf. Figure 6) | |
|---|---|
| SEQ ID NO: | Sequence |
| 1 | Amino acid sequence of *Vitreoscilla sp.* hemoglobin |
| 2 | Nucleic acid coding sequence of *Vitreoscilla sp.* hemoglobin |
| 3 | *Amino acid sequence of Ralstonia eutropha* flavohemoglobin |
| 4 | Nucleic acid coding sequence *of Ralstonia eutropha* flavohemoglobin |
| 5 | VHb forward primer for *Vitreoscilla sp.* hemoglobin amplification |
| 6 | VHb reverse primer for *Vitreoscilla sp.* hemoglobin amplification |
| 7 | FHP forward primer for *Ralstonia eutropha* flavohemoglobin amplification |
| 8 | FHP reverse primer for *Ralstonia eutropha* flavohemoglobin amplification |
| 9 | pEF6-FHP expression vector for expression of *Ralstonia eutropha* flavohemoglobin |
| 10 | pEF6-VHb expression vector for expression of *Vitreoscilla sp.* hemoglobin |
| 11 | Amino acid sequence of *Clostridium perfringens* hemoglobin (Cp-Hb) |
| 12 | Nucleic acid coding sequence for *Clostridium perfringens* hemoglobin (*Cp-hb*) |
| 13 | Amino acid sequence of *Bacillus halodurans* flavohemoglobin (HmpBh) |
| 14 | Nucleic acid coding sequence for *Bacillus halodurans* flavohemoglobin (*hmpBh*) |
| 15 | Amino acid sequence of *Bacillus subtilis* flavohemoglobin (HmpBs) |
| 16 | Nucleic acid coding sequence for *Bacillus subtilis* flavohemoglobin (*hmpBs*) |
| 17 | Amino acid sequence of *Erwinia chrysanthemi* flavohemoglobin (HmpX) |
| 18 | Nucleic acid coding sequence for *Erwinia chrysanthemi* flavohemoglobin *(hmpX)* |
| 19 | Amino acid sequence of *Escherichia coli* flavohemoglobin (Hmp) |
| 20 | Nucleic acid coding sequence for *Escherichia coli* flavohemoglobin (*hmp*) |
| 21 | Amino acid sequence of *Pseudomonas aeruginosa* flavohemoglobin (HmpPa) |
| 22 | Nucleic acid coding sequence for *Pseudomonas aeruginosa* flavohemoglobin *(hmpPa)* |
| 23 | Amino acid sequence of *Deinococcus radiodurans* flavohemoglobin (HmpDr) |
| 24 | Nucleic acid coding sequence for *Deinococcus radiodurans* flavohemoglobin *(hmpDr)* |
| 25 | Amino acid sequence of *Salmonella enterica* serovar *Typhi* CT18 flavohemoglobin (HmpSt) |
| 26 | Nucleic acid coding sequence for *Salmonella enterica* serovar *Typhi* CT18 flavohemoglobin *(hmpSt)* |
| 27 | Amino acid sequence of *Salmonella typhimurium* flavohemoglobin (HmpStm) |
| 28 | Nucleic acid coding sequence for *Salmonella typhimurium* flavohemoglobin *(hmpStm)* |
| 29 | Amino acid sequence of *Saccharomyces cerevisiae* flavohemoglobin (Yhb) |
| 30 | Nucleic acid coding sequence for *Saccharomyces cerevisiae* flavohemoglobin *(yhb1)* |
| 31 | Amino acid sequence of *Homo sapiens* neuroglobin (Ngb) |
| 32 | mRNA sequence for *Homo sapiens* neuroglobin (NGB) |
| 33 | Amino acid sequence of *Homo sapiens* cytoglobin (Cgb) |
| 34 | mRNA sequence for *Homo sapiens* cytoglobin (CYGB) |
| 35 | Amino acid sequence of *Homo sapiens* myoglobin (Mgb) |
| 36 | Nucleic acid coding sequence for *Homo sapiens* myoglobin |
| 37 | Amino acid sequence of FHPg-FAD: FHPg-FAD is lacking the NADPH domain of FHP (*Ralstonia eutropha* flavohemoglobin) |
| 38 | Nucleic acid coding sequence for FHPg-FAD: FHPg-FAD is lacking the NADPH domain of FHP (*Ralstonia eutropha* flavohemoglobin) |
| 39 | Amino acid sequence of VHb-Red: a fusion of VHb (*Vitreoscilla sp.* hemoglobin) with the reductase domain of FHP (*Ralstonia eutropha* flavohemoglobin) |
| 40 | Nucleic acid coding sequence for VHb-Red: a fusion of VHb (*Vitreoscilla sp.* hemoglobin) with the reductase domain of FHP (*Ralstonia eutropha* flavohemoglobin) |
| 41 | Amino acid sequence of VHB-FAD: a fusion of VHb (*Vitreoscilla sp.* hemoglobin) with the FAD-binding domain of FHP (*Ralstonia eutropha* flavohemoglobin) |
| 42 | Nucleic acid coding sequence for VHB-FAD: a fusion of VHb (*Vitreoscilla sp.* hemoglobin) with the FAD-binding domain of FHP *(Ralstonia eutropha* flavohemoglobin) |
| 43 | Amino acid sequence of FHPg: a truncated version of FHP *(Ralstonia eutropha* flavohemoglobin) lacking the complete reductase domain |
| 44 | Nucleic acid coding sequence for FHPg: a truncated version of FHP *(Ralstonia eutropha* flavohemoglobin) lacking the complete reductase domain |

- FIG. 1: Concentration dependent decrease in viability of MIN6 cell lines in presence of SNP. Cells were cultured for 24 hours in presence of various concentrations of SNP and viability was determined with the MTT reagent as described under Experimental procedures. Data are means ± standarddeviation of 3 to 5 independent experiments performed in triplicates.
- FIG. 2: A) shows a table depicting the effect of globin proteins on survival on insulinoma cells under hypoxic conditions. B) displays caspase activity after 24 hours of growth under nitrosative stress conditions.

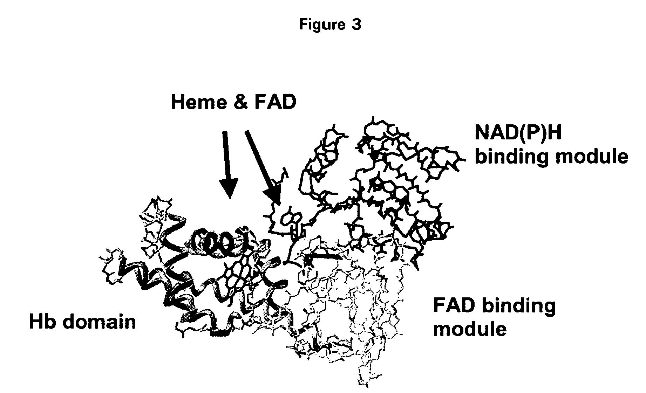

- FIG. 3: shows the structure of flavohemoglobin from Ralstonia eutropha, with N-terminal hemoglobin domain, and C-terminal FAD- und NAD(P)H-binding domains.

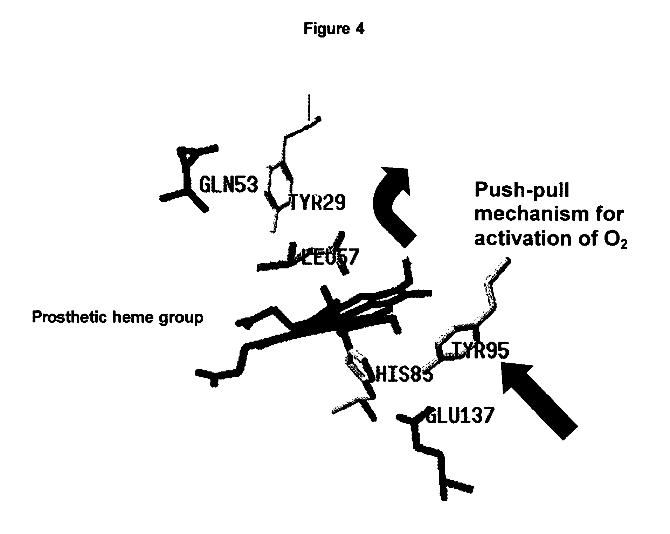

- FIG. 4: Key residues for Nitric oxide dioxygenase (NOD) activity in bacterial globin proteins as found in the structure of *Ralstonia eutropha* flavohemoglobin (FHP). The key residues have been suggested to lead to the activation of heme-bound oxygen. Tyr29 (B10), Gln53 (E7), Leu57 (E11), His85 (F8), Tyr95 (G5) and Glu137 (H23). The number in brackets indicates the position of the amino acid relative to the topological position in myoglobin. His-F8 is additionally required for coordination of heme moiety and is present in every known globin molecule.

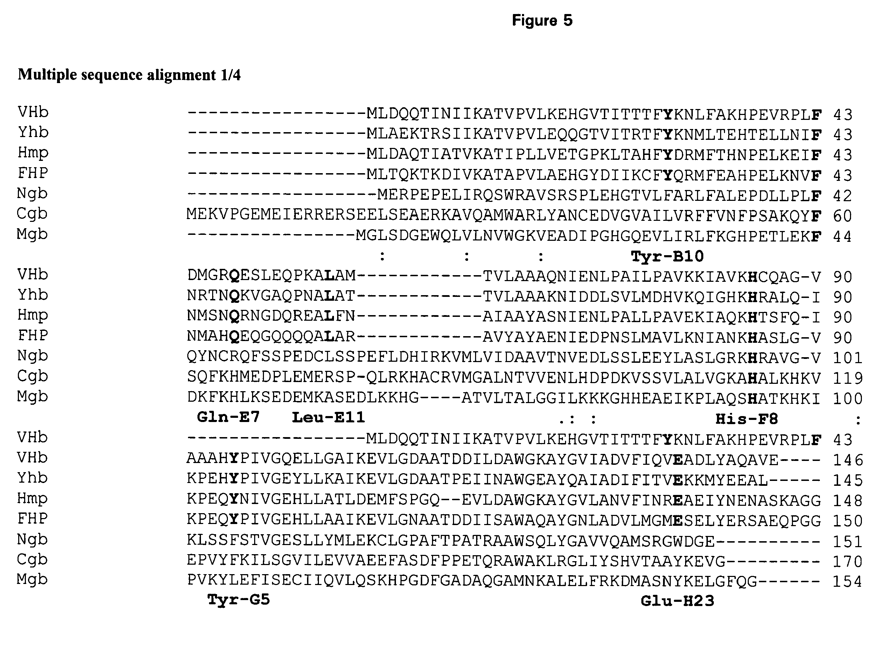

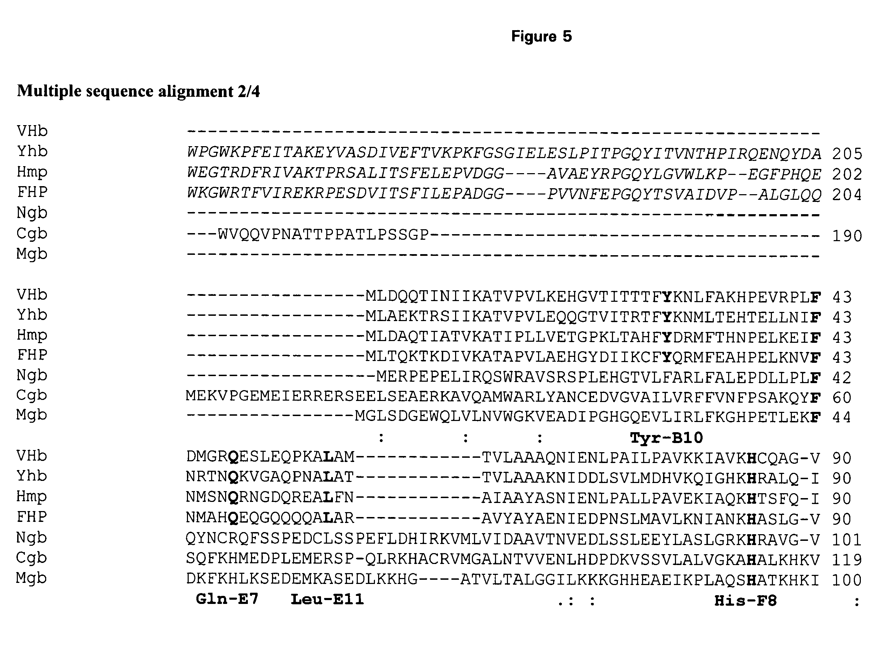

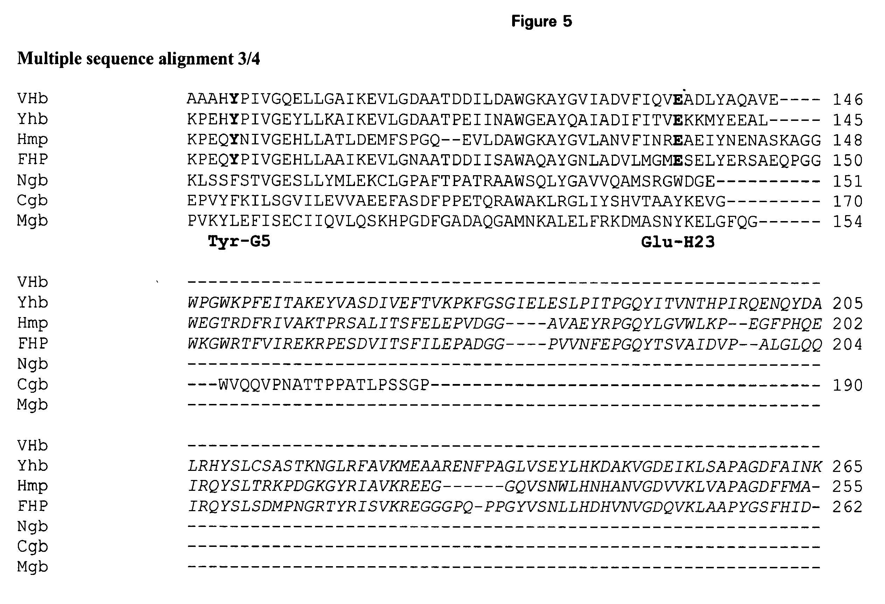

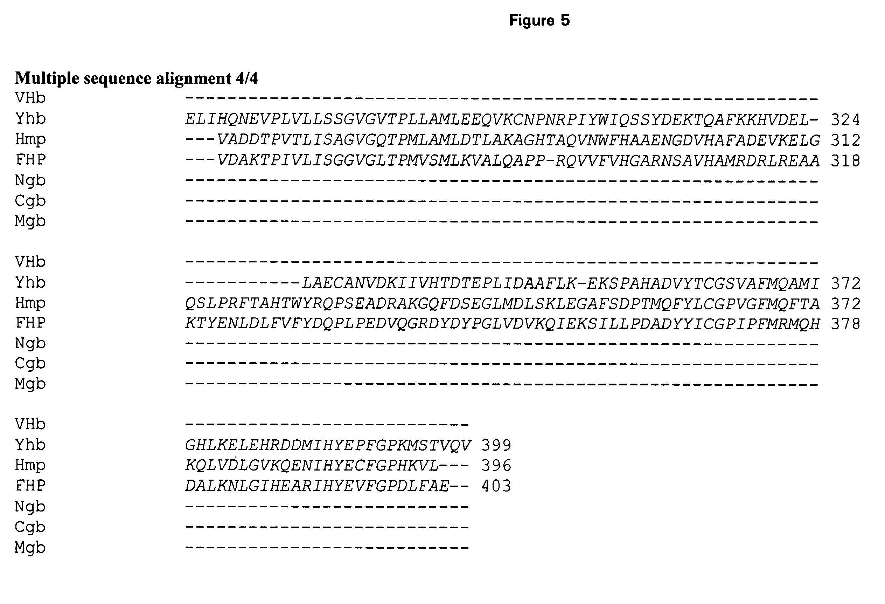

- FIG. 5: Multiple sequence alignment of bacterial globin (*Vitreoscilla* VHb), bacterial flavohemoglobins (*Ralstonia eutropha:* FHP and *Escherichia coli:* HMP), yeast flavohemoglobin and three human globin proteins as examples for mammalian globin proteins (Ngb: neuroglobin; Cgb: cytoglobin; Mgb: myoglobin). The key residues depicted in Figure 2, which are required for NO detoxification are given in bold. The reductase tail of flavohemoglobins is given in italics. Tyr29 (B10), Gln53 (E7), Leu57 (E11), Tyr95 (G5) and Glu137 (H23) are absent in all mammalian globin proteins.
- FIG. 6: Compilation of bacterial hemoglobin and flavohemoglobin with the potential to exert NOD activity.

### Examples

### Materials and Methods

*Escherichia coli* XL1-Blue (F⁻::Tn*10 proA⁺B⁺ lac*I^{q} Δ (*lacZ*)M15/*recA endA1 gyr*A96 (Nal^{r}) *thi hsdR17* (rₖ kₘ⁺) *supE44 relA1 lac*) cells were used for subcloning of the various hemoglobin genes into the plasmid pEF6/V5-His-TOPO (invitrogen). Plasmid pGE276 (Cramm et al., 1994) and pRED2 (Khosla and Bailey, 1988) were used as templates for the amplification of *fhp* and *vhb* gene by polymerase chain reaction (PCR), respectively. Oligonucleotides used for PCR amplification of the sequences encoding VHb (forward 5'-CGG GAT CCA TGT TAG ACC AGC AAA (SEQ ID NO. 5) and reverse 5'-ATA GTT TAG CGG CCG CTT ATT CAA CCG CTT GAG CG (SEQ ID NO. 6)), FHP (forward 5'-CGG GAT CCA TGC TGA CCC AGA AAA CAA A (SEQ ID NO.7) and reverse 5'-ATA GTT TAG CGG CCG CTT ACT ACT CAG CAA ACA GG (SEQ ID NO. 8)), and sequencing primers (fluorescence labeled IRD-41) were obtained from MWG Biotech (Germany). The sequence of the *fhp* gene isolated is provided in SEQ ID NO. 4 and sequence of the *vhb* gene isolated is provided in SEQ ID NO. 2.

### DNA sequencing

Cycle sequencing was done according to the supplier's manual with a Perkin Elmer GeneAmp PCR system 9600. DNA fragments were analyzed using a LI-COR 4000L automated DNA sequencer (LI-COR, Lincoln). Detection of the labeled fragments was performed with a scanning laser microscope unit and the Base Imag^{IR} software, both obtained from LI-COR (Frey et al., 2000).

### Cell culture

All cell culture media and additives were purchased form Sigma. MIN6 cells (Miyazaki et al., 1990) were cultured in Dulbecco's modified Eagle's medium (DMEM) supplemented with 10% heat inactivated FCS, 2 mM glutamine, 28 mM glucose, 100 IU/ml penicillin, 100 µg/ml streptomycin. For selection and maintenance of stably transfected cells, 1 µg/ml blasticidin (Invitrogen) was added to media. All cultures were incubated in a humidified atmosphere at 37°C and 5% CO₂.

### Transfection

The transfection of MIN6 cells was performed using the Qiagen Effectene Transfection Reagent (Qiagen, Basel, CH). 10⁵ cells were seeded in 2 ml medium per well of a 6-well plate one day before transfection. 80% confluent cells were transfected with 1 µg of plasmid DNA according to the supplier's protocol. The cells were incubated 48 hours in the presence of the transfection complexes. For stable transfection, the medium was completely aspirated , and fresh medium was added containing the selective antibiotic blasticidin (1 µg/ml). This procedure was repeated every 24 hours to ensure complete selection for stable transfected cells carrying the integrated plasmid DNA.

### Nitrosative stress and hypoxia experiments

Cells were collected by trypsinisation and quantified with a cell counter (CASY, Schärfe System). 2 x 10⁴ cells were seeded into individual wells of a 96-well tissue culture plate. Cells were incubated for 24 hours under conditions as detailed in the section "Cell culture" above, after which they were subjected to stressors or hypoxia.

Sodium nitroprusside (SNP; Sigma) was added to final concentrations ranging from 0 to 500 µM. Potassium hexacyanoferrat (K₄[Fe(CN₆)]; Sigma) at a final concentration of 200 µM was used to assess toxicity of liberated cyanide. After addition of SNP to final concentrations ranging from 0 to 500 µM, cultures were incubated in a humidified atmosphere at 37°Cand 5% CO₂ for 24 hours.

For hypoxia experiments two replicate culture plates were incubated under either standard growth conditions, i.e. 5% carbon dioxide, 21 % oxygen and 74% nitrogen (control condition) or in an atmosphere of 1 % oxygen, 5% carbon dioxide and 94% nitrogen. Control and hypoxia treatment were performed for 24 and 48 hours at 37°C h a humified athmosphere.

Cellular viability was determined using the Cell Proliferation Kit I (MTT) (Roche), which is based on a non-radioactive, colorimetric system. This assay has been shown to reliably reflect beta-cell metabolism (Janjic and Wollheim 1992). Briefly, cells were collected by trypsinisation and quantified with a cell counter (CASY, Schärfe System). Cell density was adjusted to 2 x 10⁵ cells per ml, and 100 µl of cell suspension were used to seed individual wells of a 96-well tissue culture plate. Cells were incubated for 24, after which they were subjected to stressors or hypoxia. After the incubation period, 10 µl of the MTT labeling reagent were added to each well. The microtiter plate was incubated for additional 4 hours, after which cells were solubilized in 10% SDS in 0.01 M HCI for 12 hours. For quantification of the cleavage product formazan, the difference in absorbance A₅₅₀-A₆₉₀ was measured spectrophotometrically, using a scanning multiwell spectrophotometer (Spectramax Plus, Molecular Devices). Viability was calculated in percent, using the absorbance of the non-stressed control as 100%. Experiments were performed in quadruplicates and repeated 3 to 5 times.

### Caspase activity assay

A fluorometric caspase assay was purchased from Promega (Madison, WI) and used according to the manufacturer's instructions. Cells subjected to non-lethal nitrosative stress were analyzed for the degree of caspase activity. Caspase 3-like proteases show specificity for cleavage at the C-terminal side of the aspartate residue of the sequence (Asp-Glu-Val-Asp) both caspase-3 and caspase-7. The caspase 3/7 assay is based on the use of the fluorescent compound rhodamine 110 (Leytus et al., 1983).
The caspase-3/7 substrate rhodamine 110 (bis-(N-benzyloxycarbonyl-L-aspartyl-L-glutamyl-L-valyl-L-aspartic acid amide-rhodamine 110), exists as a profluorescent substrate prior to the assay. Upon sequential cleavage and removal of the L-aspartyl-L-glutamyl-L-valyl-L-aspartic acid peptides by caspase-3/7 activity and excitation at 499nm, the rhodamine 110 leaving group becomes intensely fluorescent. The emission maximum is 521 nm. The amount of fluorescent product generated is proportional to the amount of caspase-3/7 cleavage activity present in the sample. Caspase activity is reported as the ratio of stressed versus non-stressed caspase activity. Data represents average and standard deviation of two independent experiments performed in duplicates.

### Statistical analysis

Statistical significance was determined with one-way ANOVA and Dunnett's multiple comparison test using Graphpad Statistics software package. P < 0.05 was considered significant.

### Western blot

Cells were washed twice with ice-cold PBS and scraped off in ice-cold lysis buffer (50 mM HEPES, pH 7.4, 150 mM NaCl, 1 % NP-40, 5 mM ethylene glycol bis(2-aminoethyl ether)-N,N,N'N'-tetraacetic acid (EGTA), 1 mM ethylenediaminetetraacetic acid (EDTA), 10 mg/ml leupeptin, 10 mg/ml aprotinin, 100 mM phenylmethylsulphonylfluoride, (PMSF). Suspension was incubated 5 min on ice before centrifugation at 14000 rpm, 4°Cfor 15 min. Protein concentrations of the supernatants were determined using a BCA kit (Pierce) based on the method of Bradford using bovine serum albumin as the standard (Bradford, 1976). Equal amounts of proteins were subjected to sodiumdodecylsulfate polyacrylamide gel electrophoresis (SDS-PAGE) at a constant current of 25 mA.

Proteins were transferred to polyvinylidene difluoride (PVDF) membranes (Millipore) in Towbin buffer. The transfer was performed at 100 mA at room temperature over night. The membrane was blocked in 5% non-fat dry milk in PBS for 1 hour on an orbital shaker. Antibodies were raised against purified His-tagged FHP and native VHb protein. The rabbit anti-VHb antibody has been described elsewhere (Farrés et al. 2002) and was used at a dilution of 1:3000. Anti-FHP antibody was raised in mice by immunizing with a purified, His-tagged-FHP protein. The mouse anti-FHP IgG was used at a dilution of 1:5000. Primary antibodies were diluted to the indicated concentraions in 2% blocking buffer before use. After carefully washing in PBS, proteins were visualized using a 1:5000 diluted anti-mouse IgG or 1:5000 diluted anti-rabbit IgG both coupled to horseradish-peroxidase (Amersham Pharmacia Biotech). An ECL chemiluminescent reagent was used for detection according to the manufacturer's instructions (Amersham Pharmacia Biotech).

### Results

### Construction of mammalian expression vectors

Plasmid pEF6/V5-His-TOPO was digested with *Bst*XI and self-ligated generating pEF6/V5-His. The coding sequences of *fhp* (SEQ ID NO: 4), and *vhb* (SEQ ID NO: 2), were amplified using PCR, which inserted a *Bam*HI and *Not*I restriction sites at the 5'- and the 3'-end of the amplified fragments, respectively. The designed restriction sites were used to ligate the fragments into the *Bam*HI/ *Not*I linearized plasmid pEF6/V5-His downstream of the strong EF-1 alpha promoter. The plasmids were named pEF6-FHP (SEQ ID NO: 9) and pEF6-VHb (SEQ ID NO: 101, carrying *R. eutropha fhp* (SEQ ID NO: 4) and *Vitreoscilla vhb* gene (SEQ ID NO: 2), respectively. The PCR amplified sequence of the gene was verified by DNA sequencing.

For each expression construct several independent transfections were performed and resistant cell pools were expanded. Independent blasticidin-resistant cell pools were assayed for expression of either FHP or VHb using Western blot, which verified that cell lines express VHb and FHP albeit to different extents (data not shown). An FHP- (1F2) and VHb-expressing (1V1) cell line, together with the parental MIN6 cell line, were used throughout the experimental work.

### Expression of globin proteins protects beta-cells from nitrosative stress

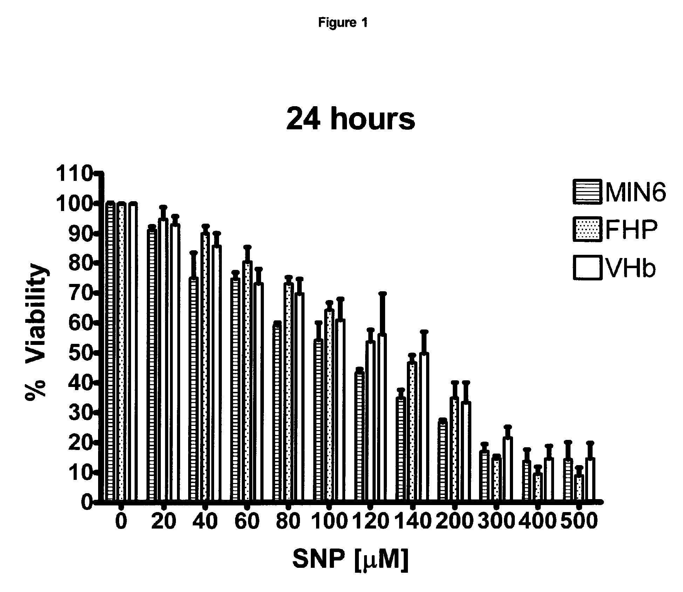

In this study MIN6 cells were challenged with various concentrations of SNP, which should mimic the NO exposure under natural conditions. SNP was added to various concentrations ranging from 0 to 500 µM. Cells were exposed to stressor for up to 48 hours, after which viability was determined using MTT reagent. Our results clearly reveal that the presence of FHP and VHb fosters the endogenous capacity of beta-cells to sustain nitrosative stress (Figure 1). The maximal protective effect was observed at an SNP concentration of 140 µM. After 24 hours of incubation, globin proteins improved viability maximally 42.8% and 33.8%, respectively relative to controls. No protective effects were observed at SNP concentrations below 80 µM or above 300 µM. Microscopic inspection revealed an increasing amount of necrotic cells at SNP concentrations above 300 µM. As a control, cells were also exposed to 200 µM K₄[Fe(CN₆)], which is structurally similar to SNP but the NO group is replaced by a sixth cyanide moiety. These experiments did not reveal any differences in cytotoxicity of this compound on the different cell lines, indicating that the adverse effect of SNP is uniquely due to the release of noxious NO and not due to cyanide. Viability after growth in 200 µM K₄[Fe(CN₆)] was 98.6% and 100% for VHb and FHP-expressing MIN6 cells, respectively.

No differences in viability were seen after an incubation period of six hours, between wildtype, VHb- and FHP-expressing MIN6 cells at different SNP concentrations (data not shown). In contrast, longer exposure did not accentuate the observed effects on viability, which could be due to the exhaustion of NO released from SNP.

### Effect of globin expression under hypoxic conditions

Besides their sensitivity towards nitrosative stress, beta-cells are also known to suffer from hypoxia, which is encountered after transplantation of islets before the revascularization of the transplanted tissue (Carlsson et al., 2000; Carlsson et al., 2001). Therefore, we subjected insulinoma cells to a treatment of low oxygen and compared their viabilities to control cultures grown under normoxic conditions. An oxygen concentrations of 1 % was chosen, which closely reflects the oxygen tension of transplanted islets at various potential transplantation sites, i.e. kidney capsule or liver (Carlsson et al., 2001). Generally, only a low reduction of viabilities was observed after 24 and 48 hours of incubation. No differences in cell viabilities between control cells and cells expressing globin proteins were observed (Figure 2A).

### Effects of NO removal on caspase activity

Whereas high NO* concentrations are known to cause necrosis, low NO concentrations will cause beta-cells death via an apoptotic pathway (Eizirik and Mandrup-Poulsen, 2001; Hui et al., 2004). Therefore, MIN6 cell lines were also challenged with 100 µM SNP for 24 hours. This concentration of SNP did not produce any significant effects on viability and was therefore considered not to destroy cells via necrosis, but rather via apoptosis. Induction of apoptosis was determined using a fluorimetric assay, which reports activity of caspase 3 and 7. Our data indicates that in the beta-cells expressing either of the two globin proteins no induction of apoptosis occurred as no increase in caspase activity was measured upon SNP administration (Figure 2B). This is in contrast to MIN6 cells, which showed a 55% higher caspase activity in cells challenged with SNP versus non-stressed cells. These data indicate that the level of NO can be controlled below a threshold level, above which apoptotic cell death through the action of caspases occurs.

Besides the generation of NO, cytokines such as TNF-alpha, IFN-gamma and II-1 beta induce also the generation of oxygen radicals (Rabinovitch et al., 1996). These findings demonstrate the necessity to protect islet cells against reactive oxygen and nitrogen species and therefore various gene transfer based therapeutic modalities have been evaluated (Bottino et al., 2003).

The data presented shows that the cell lines expressing either a hemoglobin or flavohemoglobin possess an increased tolerance against nitrosative stress induced by administration of an exogenous NO donor. Surprisingly, the cell line expressing *Vitreoscilla* hemoglobin provided an even superior protection relative to the cell lines expressing flavohemoglobin.

Based on Western blot analysis, the protective effects of VHb and FHP correlate with the expression levels of the proteins. This finding implies that, by modulating the protein expression levels, the protective effects can be optimized and adjusted to the particular needs. Furthermore, we did not select cells for high expression of transgene, but rather used mixed populations of cells. This strategy reflects more the procedure when islets will be isolated, transfected and transplanted, which occurs without selection.

The presented experimental data demonstrate that bacteria globin proteins improve the survival of host cells and in particular of pancreatic islets.

The results further demonstrate that by degrading NO, the intracellular signaling cascade can be disrupted preventing the onset of caspase mediated host cell apoptosis.

The present invention provides a new approach for protecting host cells and in particular beta-cells against nitrosative and/or oxidative stress which is particularly useful in protecting host cells against attacks of the immune system of a host organism the host cells are transplanted to.

### References

Andersson A., and Sandler S. Transplantation Rev, 6: 20, (1992).
Altschul S.F. and Gish W. Methods Enzymol, 266: 460-480 (1996)
Baba T, Takeuchi F, Kuroda M, Yuzawa H, Aoki K, Oguchi A, Nagai Y, Iwama N, Asano K, Naimi T, Kuroda H, Cui L, Yamamoto K, Hiramatsu K. Lancet, 359: 1819-1827 (2002).
Bertera, S., Crawford, M.L., Alexander, A.M., Papworth, G.D., Watkins, S.C., Robbins, P.D. and Trucco, M., Diabetes, 52: 387-393 (2003).
Bollinger C.J.T., Bailey J.E., Kallio P.T. Biotechnol. Prog., 17: 798-808 (2001).
Bottino, R., Lemarchand, P., Trucco, M. and Giannoukakis, N. Gene Ther, 10: 875-889 (2003).
Bradford, M.M., Analytical Biochemistry, 72: 248-254 (1976).
Carlsson, P.O., Palm, F., Andersson, A. and Liss, P., Transplantation, 69: 761-766. (2000).
Carlsson, P.O., Palm, F., Andersson, A. and Liss, P., Diabetes, 50: 489-495 (2001).
Contreras, J.L., Bilbao, G., Smyth, C.A., Jiang, X.L., Eckhoff, D.E., Jenkins, S.M., Thomas, F.T., Curiel, D.T. and Thomas, J.M., Transplantation, 71,: 1015-1023 (2001).
Cramm, R., Siddiqui, R.A. and Friedrich, B., J Biol Chem, 269: 7349-7354. (1994).
Crawford M.J., Goldberg D.E. J Biol Chem, 273: 12543-12547 (1998.)
Dikshit KL, Webster DA.. Gene, 70: 377-386 (1988).
Eizirik, D.L. and Mandrup-Poulsen, T., Diabetologia, 44: 2115-2133 (2001).
Farrés, J. and Kallio, P. T., Biotechnol Prog, 18: 229-233 (2002).
Farrés, J., Rechsteiner, M. P., Herold, S., Frey, A. D. and Kallio, P. T, Biochem J, 44: 4125-4134, 2005.
Frey, A.D., Bailey, J.E. and Kallio, P.T., Appl Environ Microbiol, 66: 98-104 (2000).
Frey, A.D., Farrés, J., Bollinger, C.J.T. and Kallio, P.T. Appl Environ Microbiol, 68: 4835-4840 (2002).
Frey, A.D. and Kallio, P.T., FEMS Microbiol Rev, 27: 525-545 (2003).
Gossen et al. Curr. Opin. Biotechnol. 5: 516-520 (1994).
Gossen M, Bonin AL, Bujard H. Control of Gene Activity in Higher Eukaryotic Cells by Prokaryotic Regulatory Elements. Trends Biotechnol, 12: 58-62 (1994).
Hankeln, T., Ebner, B., Fuchs, C., Gerlach, F., Haberkamp, M., Laufs, T.L., Roesner, A., Schmidt, M., Weich, B., Wystub, S., Saaler-Reinhardt, S., Reuss, S., Bolognesi, M., De Sanctis, D., Marden, M.C., Kiger, L., Moens, L., Dewilde, S., Nevo, E., Avivi, A., Weber, R.E., Fago, A. and Burmester, T., J Inorg Biochem, 99: 110-119 (2005).
Hohmeier, H.E., Thigpen, A., Tran, V.V., Davis, R. and Newgard, C.B., J Clin Invest, 101: 1811-1820 (1998).
Hui, H., Dotta, F., Di Mario, U. and Perfetti, R., J Cell Physiol, 200, 177-200 (2004).
Janjic D., and Wollheim C.B. Diabetologia, 35: 482-485 (1992).
Jin Q, Yuan ZH, Xu JG, Wang Y, Shen Y, Lu WC, Wang JH, Liu H, Yang J, Yang F, Zhang XB, Zhang JY, Yang GW, Wu HT, Qu D, Dong J, Sun LL, Xue Y, Zhao AL, Gao YS, Zhu JP, Kan B, Ding KY, Chen SX, Cheng HS, Yao ZJ, He BK, Chen RS, Ma DL, Qiang BQ, Wen YM, Hou YD, and Yu J. Nucl Acids Res, 30: 4432-4441 (2002).
Khosla, C. and Bailey, J.E., Mol Gen Genet, 214: 158-161 (1988).
Kochanek S., Hum. Gene Ther. 10: 2459-2461 (1999).
LaCelle M., Kumano M., Kurita K., Yamane K., Zuber P., Nakano M.M. J Bacteriol, 178: 3803-3808 (1996).
Lecomte, J.T., Vuletich, D.A. and Lesk, A.M., Curr Opin Struct Biol, 15: 1-12 (2005).
Leytus, S. P., Melhado, L. L. and Mangel, W. F. Biochem J, 209: 299-307, 1983.
Lortz, S., Tiedge, M., Nachtwey, T., Karlsen, A.E., Nerup, J. and Lenzen, S., Diabetes, 49: 1123-1130. (2000).
Mathis, D., Vence, L. and Benoist, C., Nature, 414: 792-798 (2001).
Mendoza V., Klein D., Ichii H., Ribeiro M.M., Ricordi C., Hankeln T., Burmester T., Pastori R.L. Transplant Proc, 37: 237-240 (2005).
Miller, J.H. and Calos M. P., eds., Cold Spring Harbor Laboratory Press (1987)
Miyazaki, J.I., Araki, K., Yamato, E., Ikegami, H., Asano, T., Shibasaki, Y., Oka, Y. and Yamamura, K.I., Endocrinology, 127: 126-132 (1990).
Moore H.P.H., Walker M.D., Lee F., Kelly R.B. Cell 35: 531-538 (1983).
Needleman S.B. and Wunsch C. D., J. Mol. Biol. 48: 443-453 (1970).
Oyadomari, S., Takeda, K., Takiguchi, M., Gotoh, T., Matsumoto, M., Wada, I., Akira, S., Araki, E. and Mori, M., Proc Natl Acad Sci U S A, 98: 10845-10850 (2001).
Qin X.Y., Shen K.T., Zhang X., Cheng Z.H., Xu X.R., Han Z.G. World J Gastroenterol, 8:367-70 (2002).
Rabinovitch, A., Suarez-Pinzon, W., Strynadka, K., Ju, Q., Edelstein, D., Brownlee, M., Korbutt, G.S. and Rajotte, R.V., Diabetes, 48: 1223-1229 (1999).
Rabinovitch, A., Suarez-Pinzon, W.L., Strynadka, K., Lakey, J.R. and Rajotte, R.V., J Clin Endocrinol Metab, 81: 3197-3202 (1996).
Salanoubat M., Genin S., Artiguenave F., Gouzy J., Mangenot S., Arlat M., Billault A., Brottier P., Camus J.C., Cattolico L., Chandler M., Choisne N., Claudel-Renard C., Cunnac S., Demange N., Gaspin C., Lavie M., Moisan A., Robert C., Saurin W., Schiex T., Siguier P., Thebault P., Whalen M., Wincker P., Levy M., Weissenbach J., Boucher C.A. Nature, 415: 497-502 (2002)
Sambrook, J. and Russell, D. Molecular Cloning: A Laboratory Manual; Cold Spring Laboratory Press (2001).
Sun, Y., Jin, K., Mao, X.O., Zhu, Y. and Greenberg, D.A., Proc Natl Acad Sci U S A, 98: 15306-1531 (2001).
Suzuki K, Ichimura A, Ogawa N, Hasebe A, Miyashita K. J Bacteriology, 184: 5714-5722 (2002).
Tiedge, M., Lortz, S., Munday, R. and Lenzen, S., Diabetologia, 42: 849-855, (1999).
Vasudevan S.G., Armarego W.L.F., Shaw D.C., Lilley P.E., Dixon N.E., Poole R.K. Mol Gen Genet, 226: 49-58 (1991).

## Claims

1. Mammalian host cell containing a nucleic acid comprising a nucleic acid selected from
a1) a nucleic acid coding for a bacterial hemoglobin or a bacterial flavohemoglobin having oxidative and/or nitrosative stress protective activity, or a complementary strand thereof;
a2) a nucleic acid coding for a polypeptide selected from the group consisting of an amino acid sequence according to SEQ ID NO: 1, 3, 1 1, 13, 15, 17, 19, 21, 23, 25, 27, 29, 37, 39, 41, and 43; or a complementary strand thereof, and which nucleic acid encodes a polypeptide having oxidative and/or nitrosative stress protective activity;
a3) a nucleic acid coding for a polypeptide having at least 60% sequence identity to one of the polypeptide sequences defined in a1) and a2) and which nucleic acid encodes a polypeptide having oxidative and/or nitrosative stress protective activity;
a4) a nucleic acid selected from the group consisting of a nucleic acid sequence according to SEQ ID NO: 2, 4, 12, 14, 16, 18, 20, 22, 24, 26, 28, 30, 38, 40, 42, and 44;
a5) a nucleic acid which on the basis of the genetic code is degenerate to one of the nucleic acid sequences defined in a1) to a4) and which nucleic acid or complementary strand thereof encodes a polypeptide having oxidative and/or nitrosative stress protective activity;
a6) a nucleic acid which hybridizes with one of the nucleic acids defined in a1) to a5) and which nucleic acid or complementary strand thereof encodes a polypeptide having oxidative and/or nitrosative stress protective activity;
a7) a nucleic acid having at least 60% sequence identity to one of the nucleic acid sequences defined in a1) to a6) and which nucleic acid or complementary strand thereof encodes a polypeptide having oxidative and/or nitrosative stress protective activity;
a8) a variant of one of the nucleic acids defined in a1) to a7), wherein compared to one of the nucleic acids defined in a1) to a7) the variant contains at least one addition, deletion, insertion and/or inversion and wherein the variant or the complementary strand thereof encodes a polypeptide having oxidative and/or nitrosative stress protective activity; and
a9) a fragment of one of the nucleic acids defined in a1) to a8), wherein the fragment or the complementary strand thereof encodes a polypeptide having oxidative and/or nitrosative stress protective activity.

2. Host cell of claim 1, wherein the nucleic acid further comprises a promoter suitable for the expression of the nucleic acid and wherein the expression of the nucleic acid encoding a polypeptide having oxidative and/or nitrosative stress protective activity is controlled by the promoter.

3. Host cell of claim 2, wherein the nucleic acid is contained in an expression vector.

4. Host cell of claim 3, wherein the expression vector is a viral or non-viral expression vector.

5. Host cell of claim 4, wherein the expression vector is selected from plasmids, cosmids, bacteriophages, retroviral vectors, vaccinia virus vectors, lentiviral vectors, herpes virus vectors, baculoviral vectors, cytomegalovirus (CMV) vectors, papillomavirus vectors, simian virus (SV40) vectors, sindbis vectors, semliki forest virus vectors, adenoviral vectors, and adeno-associated viral (AAV) vectors.

6. Host cell of any of claims 1 to 5, wherein the polypeptide encoded by the nucleic acid has at least about 70% of the oxidative and/or less than about 140% of the nitrosative stress protective activity of the *Vitreoscilla sp.* hemoglobin according to the amino acid sequence of SEQ ID NO: 1.

7. Host cell of any of claims 1 to 6, wherein the polypeptide encoded by the nucleic acid has at least about 70% of the oxidative and/or less than about 140% of the nitrosative stress protective activity of the *Ralstonia eutropha* flavohemoglobin according to the amino acid sequence of SEQ ID NO: 3.

8. Host cell of any of claims 1 to 7, wherein the cell is selected from a skin cell, a vascular cell, a neuron, a glia cell, a pancreatic cell, a hepatic cell, a chondrocyte, and a stem cell.

9. Host cell of any of claims 1 to 7, wherein the cell is selected from an HK cell, a CHO cell, a COS cell, a Vero cell, a 293 cell, a HaCaT cell, a PC12 cell, a HepG2 cell, a primary pancreatic beta-cell, a MIN6 cell, a RIN cell, a Ins-1 cell, a NIT-a cell, a HIT-T15 cell, a betaTc-3 cell, an intestinal endocrine K cell, and an intestinal endocrine L cell.

10. Host cell of any of claims 1 to 9, wherein the host cell is an autologous or a heterologous cell.

11. Host cell of any of claims 1 to 10, wherein the host cell comprises a heterologous nucleic acid suitable for expression of the polypeptide encoded by the heterologous nucleic acid and optionally for secretion of the polypeptide.

12. Host cell of claim 11, wherein the heterologous nucleic acid contains a nucleic acid sequence coding for insulin protein.

13. Culture of cells containing at least one host cell of any of claims 1 to 12.

14. Tissue containing at least one host cell of any of claims 1 to 12 or a culture of cells of claim 13.

15. Organ containing at least one host cell of any of claims 1 to 12, a culture of cells of claim 13, or a tissue of claim 14.

16. Method for producing a host cell of anyone of claims 1 to 12 comprising the steps of:
(a) providing a mammalian cell, and
(b) introducing into the cell a nucleic acid comprising a nucleic acid selected from
b1) a nucleic acid coding for a bacterial hemoglobin or a bacterial flavohemoglobin having oxidative and/or nitrosative stress protective activity, or a complementary strand thereof;
b2) a nucleic acid coding for a polypeptide selected from the group consisting of an amino acid sequence according to SEQ ID NO: 1, 3, 11, 13, 15, 17, 19, 21, 23, 25, 27, 29, 37, 39, 41, and 43; or a complementary strand thereof, and which nucleic acid encodes a polypeptide having oxidative and/or nitrosative stress protective activity;
b3) a nucleic acid coding for a polypeptide having at least 60% sequence identity to one of the polypeptide sequences defined in b1) and b2) and which nucleic acid encodes a polypeptide having oxidative and/or nitrosative stress protective activity;
b4) a nucleic acid selected from the group consisting of a nucleic acid sequence according to SEQ ID NO: 2, 4, 12, 14, 16, 18, 20, 22, 24, 26, 28, 30, 38, 40, 42, and 44;
b5) a nucleic acid which on the basis of the genetic code is degenerate to one of the nucleic acid sequences defined in b1) to b4) and which nucleic acid or complementary strand thereof encodes a polypeptide having oxidative and/or nitrosative stress protective activity;
b6) a nucleic acid which hybridizes with one of the nucleic acids defined in b1) to b5) and which nucleic acid or complementary strand thereof encodes a polypeptide having oxidative and/or nitrosative stress protective activity;
b7) a nucleic acid having at least 60% sequence identity to one of the nucleic acid sequences defined in b1) to b6) and which nucleic acid or complementary strand thereof encodes a polypeptide having oxidative and/or nitrosative stress protective activity;
b8) a variant of one of the nucleic acids defined in b1) to b7), wherein compared to one of the nucleic acids defined in b1) to b7) the variant contains at least one addition, deletion, insertion and/or inversion and wherein the variant or the complementary strand thereof encodes a polypeptide having oxidative and/or nitrosative stress protective activity; and
b9) a fragment of one of the nucleic acids defined in b1) to b8), wherein the fragment or the complementary strand thereof encodes a polypeptide having oxidative and/or nitrosative stress protective activity.

17. Method of producing a host cell of claim 16, further comprising the step
c) selecting among the cells containing the nucleic acid those cells, which express the nucleic acid defined in b).

18. Method for producing a cell of claim 16 or 17, wherein the nucleic acid further comprises a promoter suitable for the expression of the nucleic acid and wherein the expression of the nucleic acid encoding a polypeptide having oxidative and/or nitrosative stress protective activity is controlled by the promoter.

19. Method for producing a host cell of any of claims 16 to 18, wherein the nucleic acid is contained in an expression vector.

20. Method for producing a host cell of claim 19, wherein the expression vector is a viral or a non-viral expression vector.

21. Method for producing a host cell of claim 20, wherein the expression vector is selected from plasmids, cosmids, bacteriophages, retroviral vectors, vaccinia virus vectors, lentiviral vectors, herpes virus vectors, baculoviral vectors, cytomegalovirus (CMV) vectors, papillomavirus vectors, simian virus (SV40) vectors, sindbis vectors, semliki forest virus vectors, adenoviral vectors, and adeno-associated viral (AAV) vectors.

22. Method for producing a host cell of any of claims 16 to 21, further comprising the step
d) introducing into the cell a heterologous nucleic acid coding for insulin protein, wherein the heterologous nucleic acid is either contained in an expression cassette which is located on the same nucleic acid molecule as the nucleic acid defined in b) or located on a separate nucleic acid molecule and wherein in the former case the heterologous nucleic acid is introduced into the cell in step b) and in the latter case the heterologous nucleic acid and the nucleic acid defined in b) are introduced simultaneously or sequentially in either order into the cell.

23. Method for producing a host cell of any of claims 15 to 22, wherein the mammalian cell is selected from a skin cell, a vascular cell, a neuron, a glia cell, a pancreatic cell, a hepatic cell, a chondrocyte, and a stem cell.

24. Pharmaceutical composition comprising a host cell of any of claims 1 to 12 and a pharmaceutical acceptable carrier.

25. Pharmaceutical composition comprising a nucleic acid comprising a nucleic acid selected from
a1) a nucleic acid coding for a bacterial hemoglobin or a bacterial flavohemoglobin having oxidative and/or nitrosative stress protective activity, or a complementary strand thereof;
a2) a nucleic acid coding for a polypeptide selected from the group consisting of an amino acid sequence according to SEQ ID NO: 1, 3, 11, 13, 15, 17, 19, 21, 23, 25, 27, 29, 37, 39, 41, and 43; or a complementary strand thereof, and which nucleic acid encodes a polypeptide having oxidative and/or nitrosative stress protective activity;
a3) a nucleic acid coding for a polypeptide having at least 60% sequence identity to one of the polypeptide sequences defined in a1) and a2) and which nucleic acid encodes a polypeptide having oxidative and/or nitrosative stress protective activity;
a4) a nucleic acid selected from the group consisting of a nucleic acid sequence according to SEQ ID NO: 2, 4, 12, 14, 16, 18, 20, 22, 24, 26, 28, 30, 38, 40, 42, and 44;
a5) a nucleic acid which on the basis of the genetic code is degenerate to one of the nucleic acid sequences defined in a1) to a4) and which nucleic acid or complementary strand thereof encodes a polypeptide having oxidative and/or nitrosative stress protective activity;
a6) a nucleic acid which hybridizes with one of the nucleic acids defined in a1) to a5) and which nucleic acid or complementary strand thereof encodes a polypeptide having oxidative and/or nitrosative stress protective activity;
a7) a nucleic acid having at least 60% sequence identity to one of the nucleic acid sequences defined in a1) to a6) and which nucleic acid or complementary strand thereof encodes a polypeptide having oxidative and/or nitrosative stress protective activity;
a8) a variant of one of the nucleic acids defined in a1) to a7), wherein compared to one of the nucleic acids defined in a1) to a7) the variant contains at least one addition, deletion, insertion and/or inversion and wherein the variant or the complementary strand thereof encodes a polypeptide having oxidative and/or nitrosative stress protective activity; and
a9) a fragment of one of the nucleic acids defined in a1) to a8), wherein the fragment or the complementary strand thereof encodes a polypeptide having oxidative and/or nitrosative stress protective activity; and
a pharmaceutical acceptable carrier.

26. Pharmaceutical composition of claim 25, wherein the nucleic acid is contained in an expression vector.

27. Pharmaceutical composition of claim 26, wherein the expression vector is a viral or a non-viral expression vector.

28. Pharmaceutical composition of claim 27, wherein the expression vector is selected from plasmids, cosmids, bacteriophages, retroviral vectors, vaccinia virus vectors, lentiviral vectors, herpes virus vectors, baculoviral vectors, cytomegalovirus (CMV) vectors, papillomavirus vectors, simian virus (SV40) vectors, sindbis vectors, semliki forest virus vectors, adenoviral vectors, and adeno-associated viral (AAV) vectors.

29. Pharmaceutical composition of any of claims 25 to 28, wherein the composition further comprises a heterologous nucleic acid coding for a therapeutically active polypeptide, wherein the heterologous nucleic acid is either contained in the same expression vector located on the same nucleic acid molecule as the nucleic acid selected from a1) to a9), or the heterologous nucleic acid is contained in a separate expression vector located on a separate nucleic acid molecule than the nucleic acid selected from a1) to a9).

30. Pharmaceutical composition of claim 29, wherein the heterologous nucleic acid codes for insulin.

31. Use of a host cell of any of claims 1 to 12 for the production of a medicament for the treatment of a patient in need of protection against oxidative and/or nitrosative stress, and/or for the treatment of a tissue or organ for implantation and/or transplantation into a patient.

32. Use of claim 31, wherein the patient suffers from a disease selected from a disease mediated by oxidative and nitrosative stress, a neurodegenerative disorder, a disorder of gastrointestinal motility, hypotension, hypertension septic shock, toxic shock syndrome, hemodialysis, cancer, cachexia, a disease requiring immunosuppression, a disease requiring transplant therapy, an autoimmune disease, an inflammatory disease, a respiratory conditions, a chronic obstructive pulmonary disease, glomerulonephritis, viral infections, myocarditis, a viral hepatitis, an alcoholic hepatitis, heart failure, atherosclerosis, arthritis, rheumatoid arthritis, chronic bowel disease, inflammatory bowel disease, ulcerative colitis, Crohn's disease, systemic lupus erythematosis (SLE), ocular conditions, insulin-dependent type 1 diabetes mellitus, diabetes type 2, cystic fibrosis, hypoxia, hyperbaric oxygen convulsions, a toxicity indication, dementia, Sydenham's chorea, Parkinson's disease, Huntington's disease, amyotrophic lateral sclerosis, multiple sclerosis, Korsakoff's disease, imbecility related to cerebral vessel disorder, NO mediated cerebral trauma, ischemic brain edema, stroke, sleeping disorders, schizophrenia, depression, pre-menstrual syndrome (PMS), anxiety, drug addiction, pain, migraine, immune complex disease, allograft rejection, infections caused by invasive microorganisms which produce NO, and radio contrast induced renal failure.

33. Use of a nucleic acid comprising a nucleic acid selected from
a1) a nucleic acid coding for a bacterial hemoglobin or a bacterial flavohemoglobin having oxidative and/or nitrosative stress protective activity, or a complementary strand thereof;
a2) a nucleic acid coding for a polypeptide selected from the group consisting of an amino acid sequence according to SEQ ID NO: 1, 3, 11, 13, 15, 17, 19, 21, 23, 25, 27, 29, 37, 39, 41, and 43; or a complementary strand thereof, and which nucleic acid encodes a polypeptide having oxidative and/or nitrosative stress protective activity;
a3) a nucleic acid coding for a polypeptide having at least 60% sequence identity to one of the polypeptide sequences defined in a1) and a2) and which nucleic acid encodes a polypeptide having oxidative and/or nitrosative stress protective activity;
a4) a nucleic acid selected from the group consisting of a nucleic acid sequence according to SEQ ID NO: 2, 4, 12, 14, 16, 18, 20, 22, 24, 26, 28, 30, 38, 40, 42, and 44;
a5) a nucleic acid which on the basis of the genetic code is degenerate to one of the nucleic acid sequences defined in a1) to a4) and which nucleic acid or complementary strand thereof encodes a polypeptide having oxidative and/or nitrosative stress protective activity;
a6) a nucleic acid which hybridizes with one of the nucleic acids defined in a1) to a5) and which nucleic acid or complementary strand thereof encodes a polypeptide having oxidative and/or nitrosative stress protective activity;
a7) a nucleic acid having at least 60% sequence identity to one of the nucleic acid sequences defined in a1) to a6) and which nucleic acid or complementary strand thereof encodes a polypeptide having oxidative and/or nitrosative stress protective activity;
a8) a variant of one of the nucleic acids defined in a1) to a7), wherein compared to one of the nucleic acids defined in a1) to a7) the variant contains at least one addition, deletion, insertion and/or inversion and wherein the variant or the complementary strand thereof encodes a polypeptide having oxidative and/or nitrosative stress protective activity; and
a9) a fragment of one of the nucleic acids defined in a1) to a8), wherein the fragment or the complementary strand thereof encodes a polypeptide having oxidative and/or nitrosative stress protective activity;
for the production of a medicament for the treatment of a patient in need of protection against oxidative and/or nitrosative stress and/or for the treatment of a tissue or organ for implantation or transplantation into a patient.

34. Use of claim 33, wherein the patient suffers from a disease selected from a disease mediated by oxidative and nitrosative stress, a neurodegenerative disorder, a disorder of gastrointestinal motility, hypotension, hypertension septic shock, toxic shock syndrome, hemodialysis, cancer, cachexia, a disease requiring immunosuppression, a disease requiring transplant therapy, an autoimmune disease, an inflammatory disease, a respiratory conditions, a chronic obstructive pulmonary disease, glomerulonephritis, viral infections, myocarditis, a viral hepatitis, an alcoholic hepatitis, heart failure, atherosclerosis, arthritis, rheumatoid arthritis, chronic bowel disease, inflammatory bowel disease, ulcerative colitis, Crohn's disease, systemic lupus erythematosis (SLE), ocular conditions, insulin-dependent type 1 diabetes mellitus, diabetes type 2, cystic fibrosis, hypoxia, hyperbaric oxygen convulsions, a toxicity indication, dementia, Sydenham's chorea, Parkinson's disease, Huntington's disease, amyotrophic lateral sclerosis, multiple sclerosis, Korsakoff's disease, imbecility related to cerebral vessel disorder, NO mediated cerebral trauma, ischemic brain edema, stroke, sleeping disorders, schizophrenia, depression, pre-menstrual syndrome (PMS), anxiety, drug addiction, pain, migraine, immune complex disease, allograft rejection, infections caused by invasive microorganisms which produce NO, and radio contrast induced renal failure.

35. Use of claim 33 or 34, wherein the nucleic acid is contained in an expression vector.

36. Use of claim 35, wherein the expression vector is a viral or a non-viral expression vector.

37. Use of claim 36, wherein the expression vector is selected from plasmids, cosmids, bacteriophages, retroviral vectors, vaccinia virus vectors, lentiviral vectors, herpes virus vectors, baculoviral vectors, cytomegalovirus (CMV) vectors, papillomavirus vectors, simian virus (SV40) vectors, sindbis vectors, semliki forest virus vectors, adenoviral vectors, and adeno-associated viral (AAV) vectors.

38. Use of any of claims 35 to 37, wherein the medicament further comprises a heterologous nucleic acid coding for a therapeutically active polypeptide, wherein the heterologous nucleic acid is either contained in the same expression vector located on the same nucleic acid molecule as the nucleic acid selected from a1) to a9), or the heterologous nucleic acid is contained in a separate expression vector located on a separate nucleic acid molecule than the nucleic acid selected from a1) to a9).

39. Use of claim 38, wherein the heterologous nucleic acid codes for insulin.
